(19) Europäisches Patentamt
European Patent Office
Office européen des brevets

(11) Publication number: **0 095 587 B1**

(12) **EUROPEAN PATENT SPECIFICATION**

(45) Date of publication of patent specification: **20.12.89**

(51) Int. Cl.[4]: **A 61 K 6/08**, C 08 L 33/00

(21) Application number: **83104316.1**

(22) Date of filing: **02.05.83**

ERRATUM

(SEITE, SPALTE, ZEILE)
(PAGE, COLUMN, LINE)
(PAGE, COLONNE, LIGNE)

| DIE TEXTSTELLE: / TEXT PUBLISHED: / LE PASSAGE SUIVANT: | | | LAUTET BERICHTIGT: / SHOULD READ: / DEVRAIT ETRE LU: |
|---|---|---|---|
| result in depth of the pulp | 2 | 38/39 | result in death of the pulp |
| 0.15-0.75 wt% 20:1-1:3 | 3 | 24 | 0.15-0.76 wt% 20:1-1:3 |
| Associaition Specification | 7 | 31 | Association Specification |
| Time in (min) after light | 14 | 29 | Time (in min) after light |
| Lithium aluminum silicates | 21 | 25 | Lithium aluminum silicate |
| acclerator-free peroxide | 28 | 31 | accelerator-free peroxide |
| a resin-bonding silane | 31 | 48 | a resin-binding silane |
| Lithiumaluminsilikat | 34 | 59 | Lithiumaluminiumsilikat |
| Borosiliaktglas | 35 | 45 | Borosilikatglas |
| synthetische Keiselsäure | 39 | 25 | synthetische Kieselsaure |
| 1,2-hydroxypropan | 40 | 57 | 1,2-dihydroxypropan |
| 1,2-hydroxypropan | 41 | 41 | 1,2-dihydroxypropan |
| 2-hydroxypropane-1] | 49 | 38/39 | 1,2-dihydroxypropane-1] |
| (par ex., oxydes der fer) | 60 | 47 | (par ex., oxydes de fer) |
| Verre de strontium 79,878% | 60 | 57 | Verre de strontium 79,8784% |
| délai de quelconques minutes. | 61 | 25 | délai de quelques minutes. |
| 2-hydroxypropane-1 | 21 | 3 | 1,2-dihydroxypropane-1 |
| 1,2-hydroxypropan-1 | 34 | 36/37 | 1,2-dihydroxypropan-1 |

Tag der Entscheidung über die Berichtigung / Date of decision on rectification: / Date de décision portant ... : 21.3.90

Ausgabe- und Veröffentlichungstag: / Issue and publication date: / Date d'edition et de publication: 13.6.90

Patbl.Nr / EPB no: / Bull. no: 90/24

(19) Europäisches Patentamt
European Patent Office
Office européen des brevets

(11) Publication number: 0 095 587
B1

# (12) EUROPEAN PATENT SPECIFICATION

(45) Date of publication of patent specification: 20.12.89

(51) Int. Cl.[4]: A 61 K 6/08, C 08 L 33/00

(21) Application number: 83104316.1

(22) Date of filing: 02.05.83

## (54) Dental composite and porcelain repair.

(30) Priority: 03.05.82 US 374315

(43) Date of publication of application:
07.12.83 Bulletin 83/49

(45) Publication of the grant of the patent:
20.12.89 Bulletin 89/51

(84) Designated Contracting States:
AT BE CH DE FR GB IT LI LU NL SE

(56) References cited:
EP-A-0 059 649
GB-A- 569 974

(73) Proprietor: DEN-MAT CORPORATION
3130 Skyway Drive Unit 501
Santa Maria California 93454 (US)

(72) Inventor: Ibsen, Robert Louis
1571 East Main Street
Santa Maria California 93454 (US)
Inventor: Glace, William Richard
225 Soares
Santa Maria California 93455 (US)
Inventor: Jensen, Patricia A.
521 Dahlia Place
Santa Maria California 93455 (US)

(74) Representative: Kremer, Robert A.M. et al
BUREAU D.A. CASALONGA - JOSSE
Morassistrasse 8
D-8000 München 5 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 095 587 B1

Courier Press, Leamington Spa, England.

**Description**

This invention relates to dental composites and porcelain repair material and to methods for making them.

Background of the invention

Over the years many dental composites have been introduced, each composite possessing certain physical properties. However, substantially all of these dental composites can be categorized into two main groups, self-cured materials and light-cured materials.

The self-cured composites have involved free radical polymerization initiated by benzoyl peroxide (or another suitable peroxide) and accelerated, typically by a tertiary amine, typically N,N, dimethyl-p-toluidine. The curing agents must be stored separately from the resin they are to cure, with mixture just before use.

The light-cured composites have involved free radical polymerization initiated by the photoexcitation of light-sensitive compounds by ultraviolet or visible light. They are single-component systems, typically pastes, stored in opaque containers until the time of cure. Some of the photoinitiators that have been employed are the benzoin ethers, benzil ketals, dialkoxyacetophenones, benzophenone-amines, thioxanthone-amines, and hydroxyalkylphenones.

If desired, more information can be found in GB—A—569 974 and EP—A—0 059 649, the latter having been published after the priority date of the present invention. Both patent publications relate to light-curing compositions, which compositions may contain an organic peroxide for additional self-curing.

In the practice of dentistry, some tooth repairs have been better achieved by self-cured composites and some have been better achieved by light-cured composites. While many factors have helped to determine whether a dentist would or should choose a self-cured material or a light-cured material, the prime factors have been working time, set times and the architecture of the cavity preparation.

The light-cured composites, combined with special high-lumen lighting units employing fiber optics, have offered variable working time and fast "snap" sets. Setting can take between ten and forty seconds, in many instances. However, the use of light-cured composites has been limited by the depth of the repair and the ease of light penetration. Relatively unobstructed, clean, shallow repair surfaces are required. Visible-light-cured materials have helped in solving some of the limitations caused by repair depth, by roughly doubling the depths at which cure is done as compared to ultra-violet-light cured material. Also, many dentists have felt more comfortable using a visible, as opposed to an ultra-violet, activating light source.

The variable amount of cure is a function of exposure to lumens of visible light. This presents a potentially very dangerous situation in a dental restoration, because a dentist may be totally unaware of this incomplete polymerization. Consequently, the incompletely polymerized restoration may wash out, leak, or fail in adhesion. Yet the surface or bulk of the restoration may appear clinically adequate, even though new secondary decay may be beginning, and, because of its concealment, result in depth of the pulp or loss of the tooth.

This situation is extremely dangerous because most dentists do not realize this deficiency, since light-cured systems are advertized to be able to be cured through tooth structure. In reality, the situation is that at best a gradient level of cure is obtained in relation to the amount of lumens of light energy available to the restorative resin.

When a light-cured resin liner is used with a light-cured paste composite at a depth of around 3 mm or greater, the resin liner may not cure because insufficient light reaches the resin. Uncured resin liner can cause leeching, pulpal irritation, and loss of adhesion. Heretofore, the resin liner had therefore to be polymerized prior to placement of the composite. With this invention, such double cure is unnecessary.

Moreover, photoinitiators need to be used in amounts that are stoichiometric or close to it, because their effectiveness in curing diminishes rapidly when they are used either in excess or in less than stoichiometric amounts.

Self-cured systems have offered assurance of relatively complete polymerization with any repair surface architecture. However, their use has been limited by manufacturer-determined work times and set times. The peroxide and the accelerator could be adjusted to give widely varying setting times; the quicker the set time, the quicker the placement had to be made. Thus, in order to give enough time for accurate placement, the set times had to be longer than was desirable. Generally, set times have been at least two or three minutes after mix, and placement has had to be completed within forty-five seconds after mix. This had made dentists work somewhat faster than was desirable for many placements, and even then the patient had to be immobilized longer than was desirable before the composition set.

This invention overcomes all of the deficiencies of light-cured systems, while preserving all of the functional benefits.

It also overcomes the deficiencies of the self-cured systems by enabling quicker set times coupled with longer placement times, if desired.

The system of this invention thus offers the best properties of both types of curing systems without suffering from the limitations of either, and therefore significantly advances the practice of dentistry and the science of dental materials.

Similar problems occur with porcelain repair systems in general, not only in dental uses, but for repairing such porcelain articles as bathtubs, and the invention solves these problems also.

The system of the present invention can be in a powder-liquid, paste-paste, paste-powder, or gel-powder form, so that there is no loss in versatility of possible embodiments.

Consequently, this invention relates to a two-part system for making a filled methacrylate-functional resin composition by mixture of the two-parts, comprising:

first part: dental-type methacrylate-functional resin with photoinitiator therefor;

second part: dental-type filler powder with an accelerator-free peroxide curing agent for said resin;

said photoinitiator containing both (1) 2-isopropylthioxanthone or a benzil dimethyl acetal and (2) either ethyl 4-dimethylaminobenzoate or ethyl 2-dimethylaminobenzoate;

said two-part system being either

(a) a paste-paste system,

(b) a paste-powder system,

(c) a gel-powder system,

(d) a liquid-powder system,

said photoinitiator, peroxide curing agent and first and second parts in said systems being present in an amount of:

| System | Photoinitiator | Peroxide curing agent | Part 1:Part 2 |
|---|---|---|---|
| (a) | 0.06—12.8 wt% | 0.30—0.75 wt% | 1:1 |
| (b) | 0.06—12.8 wt% | 0.30—10 wt% | 20:1—10:1 |
| (c) | 0.09—14.40 wt% | 0.15—0.75 wt% | 20:1—1:3 |
| (d) | 0.18—16 wt% | 0.15—0.76 wt% | 1:2—1:3.5 |

whereby the percentages are based on the components of that part of the system containing the respective compound.

A two-component system is required, with mixture just before use. Conveniently, the resin and its photoinitiators can be stored in opaque (preferably black) containers as one component, with or without some of the filler. The peroxide is stored in a separate component, including much or all of the filler, in a container which need not be opaque. When mixed just prior to application, there is a wide latitude of mixing time, because no accelerator is used, so that the curing effect of the peroxide in the resin is quite slow, while the exposure of the resin and photoinitiator in ordinary light—whether daylight or artificial light—will not result in substantial curing. No ultraviolet inhibitor is needed or used. The time for placement is not critical, because neither the photoinitiator nor the peroxide is causing curing quickly at this stage. Then a high-lumen light source is used with a fiber-optics bundle to effect rapid cure (ten to forty seconds, typically) down to a substantial depth. If the cavity being filled is deeper than that depth, or if some of the placed material has been shaded from the light or not adequately illuminated, the light does not effect sufficient cure, but the cured deposit covering the uncured material holds the uncured material in place, and the peroxide has been found to effect cure of that material in about half an hour.

As in photoinitiator systems generally, absorption of light by the ketone group of some photoinitiators results in promotion of the photoinitiator to a chemically reactive excited state. In the presence of a hydrogen donor such as a tertiary amine, the excited-state ketone group abstracts hydrogen from the amine, and this results in the formation of free radicals.

The incorporation of a suitable peroxide such as benzoyl peroxide with these types of photoinitiated composite systems has a synergistic effect on the free radical polymerization, resulting in uniform cure without limitation as to depth of cure, due to continued polymerization after exposure to the activating light source. Other suitable peroxides, such as lauryl peroxide, isopropyl benzoyl peroxide, dicumyl peroxide, and cumene hydroperoxide may be used. The peroxide content is preferably about 0.15% to about 0.3% of the total composition.

Suitable photoinitiators include the following:

2-Isopropyl thioxanthone with ethyl 4-dimethyl amino benzoate

2-Isopropyl thioxanthone with ethyl-2-dimethylamino benzoate

Benzil dimethyl acetal with ethyl-4-dimethyl amino benzoate

Benzil dimethyl acetal with ethyl-2-dimethyl amino benzoate.

The present invention provides a much deeper cure than do light-cured composites and obtains a much more uniform degree of cure than do such systems. It obtains a certainty of cure under overhangs, as opposed to prior-art light-cured composites, and a more reliable cure through tooth structure than the prior-art light-cured composites. It provides more uniformity of cure, regardless of exposure time or intensity. It provides better adhesion to the teeth or to substrates such as porcelain as used in dentistry and in plumbing fixtures, apparently due to more complete polymerization. It has a longer shelf-life than self-cured composites. It has better physical properties than most prior-art light-cured composites and lower water sorption. It does not develop color bodies in composites using chemically active glasses, such as strontium glass, where some curing systems do develop objectionable color. Moreover, the composite,

when installed and cured in a dental environment, looks like the tooth, not only in ordinary light but also in ultraviolet light, whence it fluoresces to substantially the same degree as the tooth itself.

As stated above, the product is made up of two separate formulations, one containing all or much of the material to be cured along with the photoinitiators, and this one is kept from light, as in an opaque container. The other formulation contains the peroxide curing agent, preferably along with any ingredient not itself cured thereby, such as the fillers. In many instances (as in paste-paste systems) it is preferred that the composition be so formulated so that equal amounts of the two formulations are mixed together when they are to be used. In powder-liquid systems, there is usually two to three and one half as much powder as liquid in each mixture.

Description of some preferred embodiments

The binders or resins to be cured comprise susbtantially all of those currently used in dental composites. These are all methacrylate-functional resins. Typical is ethoxylated bisphenol-A-dimethacrylate. Others include Bis-GMA, the adducts disclosed in Waller's U.S. Patent No. 3,629,187, and the methacrylate monomer composition listed in Temin et al, U.S. Patent No. 3,991,008. Mixtures of resins may be used. Waller's adducts are of 2-2'-propane bis [3-(4-phenoxy)-1,2-dihydroxy propane-1-methacrylate] and a mono- or di- isocyanate. Temins' monomers are methacrylate monomers having 2 to 4 polymerizable double bonds and 0.3 to 2.0% by weight based on the methacrylate monomer of a substituted thiourea reducing agent having the formula

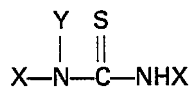

where X is hydrogen or Y and Y is $C_1$ to $C_8$ alkyl; $C_5$ or $C_6$ cycloalkyl; -chloro, hydroxy- or mercapto-substituted $C_1$ to $C_8$ allyl; $C_3$ to $C_4$ alkenyl, $C_6$ to $C_8$ aryl; chloro-, hydroxy-, methoxy-, or sulfonyl substituted phenyl; $C_2$ to $C_8$ acyl; chloro-, or methoxy- substituted $C_2$ to $C_8$ acyl; $C_7$ to $C_8$ aralkyl; or chloro- or methoxy-substituted $C_7$ to $C_8$ aralkyl.

The curing agents, as said, are of two types: peroxides and photoinitiators. Some suitable peroxides are:

Benzoyl peroxide
Isopropyl benzoyl peroxide
Lauryl peroxide
Dicumyl peroxide
Cumene hydroperoxide.

As noted above, the photoinitiators include tertiary amines. The photoinitiators are really two chemicals used together. They are solids and they are preferably dissolved in the resin or dispersed on the solid filler particles. The photoinitiators are ordinarily in the same component as the resin, the resin-photoinitiator mixture being kept in opaque containers until use. The peroxide is ordinarily kept separate from the resin, usually with or upon the filler or most of the filler. In paste-paste systems, some of the resin may be in the same component as the peroxide.

An example of a liquid curing agent is

2-Hydroxy-2-methyl-1-phenyl-propan-1-one with ethyl-2-dimethyl amino benzoate,

which curing agent is of the triamine type.

The suitable solid curing agents include:

2-Isopropyl thioxanthone with ethyl-4-dimethyl amino benzoate
2-Isopropyl thioxanthone with ethyl-2-dimethyl amino benzoate
Benzil dimethyl acetal with ethyl-4-dimethyl amino benzoate
Benzil dimethyl acetal with ethyl-2-dimethyl amino benzoate.

To the best of our knowledge, no one has previously suggested that the combination of any of these light-curing activators with any peroxide could cause curing of these dental resins to occur. Likewise, it is not ordinary to utilize peroxide in such small amounts as those utilized in this invention to initiate polymerization of these resins. Furthermore, peroxide curing agents are conventionally employed with accelerators, and no accelerator is used here. Consequently, the results obtained by this invention were totally unexpected.

Practically all inert fillers used or usable in dental composites are usable. Preferably, they are neither too coarse nor too fine. The compositions employed in this invention may contain at least about 10% by weight and up to about 90% by weight, and preferably about 70—80% by weight, of a finely divided, inert inorganic filler. The filler, which may be in the form of spheres, platelets, fibers, whiskers, or particles of any regular or irregular shape and which preferably is transparent or translucent, may comprise for example, apatite, soda glass, barium glass, strontium glass, borosilicate glass, silica, fumed silica, flint silica, alumina, quartz, lithium aluminum silicate or the like. Mixtures of more than one filler may be used. The particle size of the filler may range from about 0.005 to about 0.5 μm in the case of microfine silica, to not greater than about 500 μm in the case of irregularly shaped particles. Further, a range of particles sizes may be used. Where the filler is in the form of fibers, the maximum dimension of the fibers preferably is not

greater than about 110 μm. On the other hand, where the filler is in the form of spheres, platelets or is irregularly shaped, the maximum dimension of the particles preferably is not greater than about 350 μm.

The identity of the filler is not critical, but barium-containing glass (hereinafter called "barium glass"), strontium-containing glass (hereinafter called "strontium glass"), lithium aluminum silicate, flint silica, and fumed silica are excellent fillers, and mixtures of these are usually preferable to the use of just one of them. For example, lithium aluminum silicate has a negative heat coefficient of expansion, giving lower overall thermal dimensional changes to the composite. Barium and strontium glass impart opacity to X-rays. Flint silica imparts tooth-like color, and fumed silica adjusts viscosity and improves polishability.

One example of barium glass is Ray-Sorb T-2000, a product of Kimble Division of Owens-Illinois Glass Company. The same company makes Ray-Sorb T-4000, an example of strontium glass.

Preferably, the peroxide is dispersed on the filler powder, or by being dissolved in a suitable solvent, sprayed on the filler powder, and the solvent evaporated. Preferably, the peroxide is deposited in combination with a silane, such as gamma-methacryloxypropyltrimethoxysilane (sold by Union Carbide as A-174 silane), which is used to improve bonding between the filler and the resin. The benzoyl peroxide and the silane may be dissolved in methylene chloride or chloroform or ether or acetone, for example. Then a slurry is made with the powdered filler; the solvent is stripped off, leaving the silane and the peroxide deposited on the dry powdered filler.

Glacial acetic acid is often used, in very small amounts, because the hydrolysis reaction which attaches the silane molecule to the filler particle is carried out most effectively at a pH slightly less than neutral.

Butylated hydroxytoluene is sometimes used in order to scavenge small amounts of free radicals which can form during extended shelf storage.

The invention can assume several forms: a powder-liquid form, a paste-paste form, a paste-powder form, and a gel-powder form. These forms will be considered in order.

Powder-liquid systems

In general, the powder in this form, comprises a suitable filler material, a suitable silane, such as gamma-methacryloxypropyltrimethoxysilane, and a suitable peroxide curing agent such as benzoyl peroxide. Preferably, these ingredients are in the following approximate range of proportions by weight:

Powder:

| Ingredients | Percentage of powder |
|---|---|
| Filler | 99.75 to 97.75% |
| Silane | 0.10 to 1.50% |
| Peroxide curing agent | 0.15 to 0.75% |

As stated above, the filler may be a mixture of some or several of the fillers listed above, or it may be just one type of filler.

Liquid:

| | Percentage by weight |
|---|---|
| Resin | 99.82 to 84% |
| Photoinitiator | 0.18 to 16% |

The formulae may be further generalized in a somewhat more specific manner as follows; it being understood that the various types of ingredients (e.g., filler, resin, etc.) will be used in quantities that will total to the amounts just spelled out above:

Powder:

| Ingredients | Percent by weight |
|---|---|
| Barium glass | 0 to 30 |
| Lithium aluminum silicate | 0 to 99.75 |
| Flint silica | 0 to 10 |
| Borosilicate glass | 0 to 99.75 |
| Fumed synthetic silica | 0 to 99.75 |
| Quartz | 0 to 99.75 |
| Titanium dioxide | 0 to 0.15 |
| Tinting agents (e.g., iron oxides) | 0 to 5 |
| A-174 Silane | 0 to 1.50 |
| Peroxide curing agent | 0.15 to 0.76 |

Liquid:

| Ingredients | Percent by weight |
|---|---|
| Bis-GMA | 0 to 80 |
| Ethoxylated bisphenol-A-dimethacrylate | 0 to 99 |
| Ethylene glycol dimethacrylate | 0 to 60 |
| Diethylene glycol dimethacrylate | 0 to 60 |
| Triethylene glycol dimethacrylate | 0 to 60 |
| Polyethylene glycol dimethacrylate | 0 to 60 |
| Photoinitiator (one or more from list above) | 0.18 to 16 |

The tinting agents are used to impart a more tooth-like color. Red and yellow iron oxides are usually employed.

Storage life for each mixture is very long, no deterioration being noticed so far. The powder and the liquid are mixed together in a ratio of weight from about 2:1 to about 3.5:1, powder to liquid, just before they are needed. Mixing may be accomplished on a paper mixing pad, with a plastic instrument. Mixing may be done under normal room lighting conditions, as found in the dental operatory, illumination typically varying in intensity from 861,112 to 1076,390 lux (=about 80 to about 100 foot candles). Under these conditions, the paste begins to gel after about 10 to 30 minutes, depending on the particular formulations and illuminations.

When it is desired to initiate curing, the mixed material is exposed to the output from a dental visible light curing unit. For all examples cited herein, a Visar curing light, marketed by Den-Mat, Inc., Santa Maria, California, was used. This unit utilizes a type EKE quartz-halogen light bulb, operating at 21 VAC. The light is transmitted to the work site by a flexible fiber-optic bundle, 6.35 mm (1/4 inch) in diameter by 121.92 cm (four feet) in length. Other units are available from other manufacturers. All are similar in principle and results. Results of these examples would be expected to differ only in degree if other units were used. The output of the Visar unit used is $180 \times 10^4$ Candella per square meter, giving illumination of about 215 278 lux (20,000 foot candles) on the material being cured.

Under these conditions, the material cures in from 10 seconds to 30 seconds, with a cured depth of from 0.60 to 4.80 mm. Upon further standing, that is, from 5 minutes to 30 minutes, the material exhibits a cure depth of greater than 12 mm. It shows a tensile strength, measured by the diametral method on samples 6 mm diameter by 3 mm high, of between 331.008 $10^5$ Pa and 448.240 $10^5$ Pa (4800 psi and 6500 psi). A popular prior-art, light-cured dental composite, when tested in identical fashion, showed a cure depth of 3.05 mm immediately after exposure to the curing light, but this cure depth did not increase over a period of 16 hours after cure, and its diametral tensile strength was only 2030 psi.

## Example 1

Powder-liquid system

Powder:

| | |
|---|---|
| Barium glass | 29.82% |
| Lithium aluminum silicate | 67.30% |
| Flint silica | 1.22% |
| $TiO_2$ | 0.02% |
| Benzoyl peroxide | 0.15% |
| A-174 Silane | 1.47% |
| Glacial acetic acid | 0.02% |

A-174 Silane is a Union Carbide product, chemically gamma-methacryloxypropyltrimethoxysilane. The barium glass may be Ray-Sorb T-2000, a product of Kimble Division of Owens-Illinois Glass Company, or may be barium aluminum silicate.

Liquid:

| | |
|---|---|
| Ethoxylated bisphenol-A-dimethacrylate | 91.50% |
| 2-Isopropylthioxanthone | 0.17% |
| Ethyl 4-dimethylaminobenzoate | 8.33% |

The powder and the liquid are mixed together preferably in a ratio by weight of from about 2:1 to about 3½:1, powder to liquid, just before they are needed. Mixing takes about twenty seconds. The mixtures should preferably be applied within about five minutes. The setting time is about twenty minutes when not exposed to a dental curing light, but is less than thirty seconds on exposure to a dental curing light. The result is a hard filling.

Example 2

Hardness comparisons with some prior-art systems:

A disk of a commercially available light-curable dental composite was prepared, 20 mm diameter by 2 mm thick. One-half of this disk was shaded with aluminum foil, and the disk then was placed under a photoflood lamp for 15 minutes. The sample was removed from under the lamp, the foil removed from the sample, and Barcol hardness measured on both the shaded and unshaded halves. The unshaded half showed a Barcol hardness of 82, while the shaded side had a Barcol reading of less than 1. This same experiment was performed on a dish made of the mixed but uncured material of Example 1, and *both* sides showed a Barcol hardness of 92.

Example 3

Comparison of degree of cure:

A series of tests was performed on several commercially-available light-curing composites to determine the degree of cure of the resin matrix. Testing was accomplished by preparing duplicate samples, 1 mm thick by 40 mm in diameter. These samples were cured by exposing them to a photoflood lamp for 30 minutes, and then they were placed in 37°C. water for 24 hours. The samples were then dried, and placed in a dessicator and weighed daily until constant weight (±0.5 mg.) was achieved. The samples were then granulated, placed in a glass thimble and extracted for 12 hours in a Soxhlett extraction apparatus with methylene chloride. After extraction, the samples were weighted and the weight loss converted to per cent uncured monomer removed.

When two popular commercially-available light-cured dental composites were tested in this manner, the cured product contained 3.08% and 5.26% extractables, representative of the amount of uncured monomer.

The material of Example 1 of this invention, when tested in the same manner, contained only 2.04% extractables, showing a much higher degree of cure.

Example 4

Comparison of degree of water sorption:

A series of three light-cured dental restoratives were tested in order to determine the degree of water sorption. Samples were in duplicate, and were configured and tested in accordance with American Dental Assocaition Specification No. 27.

Two popular commercially-available light-cured dental restoratives were found to have water sorption values of 1.05 and 0.95 mg/cm$^2$, respectively. The material of Example 1 gave a value of only 0.45 mg/cm$^2$.

Example 5

Powder-liquid system of porcelain repair:

The powder-liquid system of Example 1 was tested as a porcelain repair material, as described in U.S. Patent No. 4,117,595.

A commercially available material marketed to practice the above-mentioned patent had test results of 138.9544 · 10$^5$ Pa (2015 psi) bond strength (average of 5 samples) when tested 7 days after preparation, being stored meantime in water at 37°C.

The material of Example 1, when tested in the same fashion, with the same test equipment, gave no numerical results for bond strength because the test fixture deformed under the high stresses, preventing the same from rupturing, so that though no values were obtained, the results were far better.

Example 6

Comparison with powder-liquid system using larger amounts of benzoyl peroxide, per conventional beliefs as to proper amount:

In an effort to prepare a dental composite for use in occlusal restorations, the following material was mixed:

| | | |
|---|---|---|
| Powder: | | |
| | Strontium glass (Raysorb 4000) | 100 parts |
| | A-174 Silane | 1.50 parts |
| | Benzoyl peroxide | 3.00 parts |
| Liquid: | | |
| | Ethoxylated bisphenol-A-dimethacrylate | 80 parts |
| | Triethylene glycol dimethacrylate | 20 parts |
| | 2-Hydroxy-4-methoxy benzophenone | 2 parts |
| | Butylated hydroxy toluene | 0.05 parts |
| | N,N-2-hydroxyethyl-p-toluidine | 1.50 parts |

The powder and liquid were mixed in a weight ratio of 3:1 of powder to liquid and cured properly. However, a bright green color was formed on curing.

The mixture was repeated after acid-washing the Strontium glass, but the green color persisted.

The mixture was again repeated using N,N-3,5-tetramethylanaline as a substitute for the N,N-2-hydroxyethyl-p-toluidine. Again, the green color developed.

The mixture was repeated once again using N,N-dimethyl-p-toluidine as activator instead of the N,N-2-hydroxyethyl-p-toluidine. This time the green color did not develop, but the curing characteristics of the composite were degraded.

Then the experiment was repeated, using the liquid of Example 1 with the following powder:

| | |
|---|---|
| Strontium glass (Raysorb T-4000) | 100 parts |
| A-174 Silane | 1.50 parts |
| Benzoyl peroxide | 0.30 parts |

No color developed, the material cured satisfactorily under the output from a Visar dental curing light, and exhibited a depth of cure of 3.85 mm immediately after exposure to the light, and 12 mm depth of cure after 30 minutes. Diametral tensile strength was 379.28 $10^5$ Pa (5500 psi).

Example 7

Powder-liquid system

| | | |
|---|---|---|
| Powder: | | |
| | Barium glass | 26.66% |
| | Lithium aluminum silicate | 62.24% |
| | Flint silica | 9.01% |
| | Benzoyl peroxide | 0.60% |
| | A-174 Silane | 1.47% |
| | Glacial acetic acid | 0.02% |
| Liquid: | | |
| | Ethoxylated bisphenol-A-dimethacrylate | 91.50% |
| | 2-Isopropylthioxanthone | 0.17% |
| | Ethyl 4-dimethylaminobenzoate | 8.33% |

Again, the powder and liquid are mixed together in a weight ratio of about 2:1 to about 3½:1, powder to liquid. Mixing, working, and curing times are about the same as in Example 1.

Example 8

Powder-liquid system

| | | |
|---|---|---|
| Powder: | | |
| | Lithium aluminum silicate | 69.79% |
| | Barium glass | 29.91% |
| | Benzoyl peroxide | 0.30% |
| Liquid: | | |
| | Bis-GMA, i.e 2,2′-propane bis [3-(4-phenoxy)-1,2-dihydroxy propane-1-methacrylate] | 54.6% |
| | Triethylene glycol dimethacrylate | 36.4% |
| | 2-Isopropyl thioxanthone | 0.02% |
| | Ethyl 4-dimethyl amino benzoate | 8.98% |

Three parts of the powder and one part of the liquid are mixed together to form a paste. This paste, when exposed to a visible light dental curing unit for 10 seconds, cures to a depth of 4.79 mm when measured at 10 minutes. Under normal room fluorescent lighting, the paste had a workable time of approximately 25 minutes.

Example 9

Powder-liquid system

| | | |
|---|---|---|
| Powder: | | |
| | Lithium aluminum silicate | 69.79% |
| | Barium glass | 29.91% |
| | Benzoyl peroxide | 0.30% |
| Liquid: | | |
| | Ethoxylated bisphenol-A-dimethacrylate | 72.8% |
| | Triethylene glycol dimethacrylate | 18.2% |
| | 2-Isopropyl thioxanthone | 0.02% |
| | Ethyl-4-dimethyl amino benzoate | 8.98% |

Three parts of the powder are mixed with one part of the liquid and mixed together to form a paste. This paste, when exposed to the output from a Visar dental visible light curing unit for 10 seconds, cures to a depth of 4.97 mm when measured after 10 minutes. When left exposed to room fluorescent lighting, the paste had a working time of about 25 minutes.

Example 10

A series of five resin blends, resins A to E, were made up according to Table I:

TABLE I
Resin blends

| | |
|---|---|
| Resin A: | |
| Ethoxylated bisphenol-A-dimethacrylate | 100.00% |
| Resin B: | |
| Ethoxylated bisphenol-A-dimethacrylate | 91.50% |
| 2-Isopropyl thioxanthone | 0.17% |
| Ethyl 4-dimethylaminobenzoate | 8.33% |
| Resin C: | |
| Ethoxylated bisphenol-A-dimethacrylate | 91.50% |
| 2-Isopropyl thioxanthone | 0.17% |
| Ethyl 2-dimethylaminobenzoate | 8.33% |
| Resin D: | |
| Ethoxylated bisphenol-A-dimethacrylate | 85.92% |
| Benzil dimethyl acetal | 6.26% |
| Ethyl 4-dimethylaminobenzoate | 7.82% |
| Resin E: | |
| Ethoxylated bisphenol-A-dimethacrylate | 85.92% |
| Benzil dimethyl acetal | 6.26% |
| Ethyl 2-dimethylaminobenzoate | 7.82% |

A standard blend of powder was prepared as follows:

| | |
|---|---|
| Barium glass | 30 parts |
| Lithium aluminum silicate | 70 parts |
| A-174 Silane | 1.50 parts |

To this powder were added peroxide and titanium dioxide as shown in Table II, below. The resultant powders were mixed with the resin blends as also shown in Table II. The resultant pastes were exposed to the output from a Visar dental curing light for 30 seconds, and the depth of cure measurement taken. The results, in mm., are shown in Table II.

TABLE II
Depth (in mm) of cure
immediately after 30 second light exposure

| Resin blend | Ratio liquid to powder | Powder | | | | | | | | | | |
|---|---|---|---|---|---|---|---|---|---|---|---|---|
| | | No BPO** | No BPO | .15% BPO | .2% BPO | .22% BPO | .24% BPO | .26% BPO | .30% BPO | .40% BPO | .61% BPO | .76% BPO |
| | | No $TiO_2$ | .09—.12% $TiO_2$ | .025% $TiO_2$ | .033% $TiO_2$ | .036% $TiO_2$ | .04% $TiO_2$ | .043% $TiO_2$ | .05% $TiO_2$ | .066% $TiO_2$ | .10% $TiO_2$ | .05% $TiO_2$ |
| A | *(1:2) | | | | | | | | | | no cure | |
| B | (1:2) | 2.55 | | 3.14 | | 3.18 | 3.30 | 3.20 | 3.10 | | 3.68 | 4.90 |
| B | (1:3) | | 1.20 | 3.05 | 3.10 | 3.15 | | | 3.80 | 3.20 | 4.80 | |
| C | (1:2) | 2.15 | | | | | | | | | 3.05 | |
| D | (1:2) | 2.50 | | | | | | | | | 3.35 | |
| E | (1:2) | | 1.45 | | | | | | | | 2.60 | |

*See key to resin blends
**BPO—Benzoyl peroxide

On some of these samples, glass tubes 12 mm long were filled with the test material, and wrapped with black vinyl tape to exclude light. The top of the sample was exposed to the output from the Visar light for 30 seconds, then the time was measured until the material at the opposite end of the tube was cured. Results are shown in Table III.

As a control, a popular commercially available light-cured dental composite was tested in the same manner. It initially cured in 30 seconds to a depth of 3.05 mm, and there was no increase in cure depth when measured after 16 hours.

TABLE III
Powder/liquid

Time (in min.) afer light exposure to obtain "infinite" depth of cure

| Resin blend/powder (liquid/powder ratio) | | .15% BPO** | .22% BPO | .24% BPO | .30% BPO | .40% BPO | .61% BPO | .76% BPO |
|---|---|---|---|---|---|---|---|---|
| B* | (1:3) | 20 | 15 | | 15 | 10 | 5 | |
| B | (1:2) | 30 | 17 | 17 | 15 | 15 | | 5 |
| C | (1:2) | | | | | | 15 | |
| D | (1:2) | | | | | | 10 | |
| E | (1:2) | | | | | | 29 | |

*See key to resin blends
**BPO—Benzoyl peroxide

Paste-paste systems

Some dentists (and perhaps other users) prefer to work with pastes; most of these users also prefer to use equal amounts of the two pastes, so that paste-paste systems are usually formulated to enable use of equal amounts.

In general, the paste-paste system of the present invention may be formulated as follows:

| | | Percent by weight |
|---|---|---|
| Paste A | | |
| | Resin | 18 to 40 |
| | Filler | 82 to 60 |
| | Peroxide curing agent | 0.75 to 0.30 |
| | A-174 Silane | 1.23 to 0.06 |
| | Butylated hydroxy toluene | 0.01 to 0.10 |
| Paste B | | |
| | Resin | 18 to 40 |
| | Filler | 80 to 60 |
| | Photoinitiators | 0.06 to 12.8 |
| | A-174 Silane | 1.23 to 0.06 |

The two pastes are preferably mixed in equal amounts. Mixing time, working time, and setting time are approximately the same as for the powder-liquid systems, described above, and the results in cure and hardness are approximately the same, too. Again, ordinary room lighting has little curing effect. The resin may be the same in both pastes, as may the filler. The silane is preferably deposited on the filler of both pastes prior to making the pastes. The presence of both resin and peroxide in Paste A seems not to effect a cure nor to affect substantially the storage life.

More specifically considered, the paste-paste system employs the following formulations:

| | | Percent by weight |
|---|---|---|
| Paste A | | |
| | Bis-GMA | 0 to 32 |
| | Ethoxylated bisphenol-A-dimethacrylate | 0 to 40 |
| | Ethylene glycol dimethacrylate | 0 to 24 |
| | Diethylene glycol dimethacrylate | 0 to 24 |
| | Triethylene glycol dimethacrylate | 0 to 24 |
| | Polyethylene glycol dimethacrylate | 0 to 24 |
| | Barium glass | 0 to 25 |
| | Lithium aluminum silicate | 0 to 82 |
| | Flint silica | 0 to 8.5 |
| | Borosilicate glass | 0 to 82 |
| | Fumed synthetic silica | 0 to 52 |
| | Quartz | 0 to 82 |
| | Strontium glass | 0 to 82 |
| | Titanium dioxide | 0 to 0.13 |
| | Tinting agents (e.g., iron oxides) | 0 to 4 |
| | A-174 Silane | 0 to 1.25 |
| | Peroxide curing agent | 0.30 to 0.75 |
| Paste B | | |
| | Bis-GMA | 0 to 32 |
| | Ethoxylated bisphenol-A-dimethacrylate | 0 to 40 |
| | Ethylene glycol dimethacrylate | 0 to 24 |
| | Diethylene glycol dimethacrylate | 0 to 24 |
| | Triethylene glycol dimethacrylate | 0 to 24 |
| | Polyethylene glycol dimethacrylate | 0 to 24 |
| | Barium glass | 0 to 25 |
| | Lithium aluminum silicate | 0 to 82 |
| | Flint silica | 0 to 8.5 |
| | Borosilicate glass | 0 to 82 |
| | Fumed synthetic silica | 0 to 52 |
| | Quartz | 0 to 82 |
| | Strontium glass | 0 to 82 |
| | Titanium dioxide | 0 to 0.13 |
| | Tinting agents (e.g., iron oxides) | 0 to 4 |
| | A-174 Silane | 0 to 1.25 |
| | Photoinitiators | 0.10 to 12.8 |

This formulation should be read with the more general one preceding it, to supply the percentages of fillers, resins, and silane needed, along with appropriate amounts of the curing agents.

Mixing is somewhat easier than with the powder-liquid system and emplacement is substantially the same.

Example 11

Paste-paste system

| | | |
|---|---|---|
| Paste A: | | |
| | Ethoxylated bisphenol-A-dimethacrylate | 17.36% |
| | Benzoyl peroxide | 0.60% |
| | Butylated hydroxytoluene | 0.04% |
| | Strontium glass | 79.8784% |
| | Fumed silica | 0.92% |
| | A-174 Silane | 1.20% |
| | Glacial acetic acid | 0.0016% |

The Strontium glass may be Ray Sorb T-4000 of Kimble Division of Owens-Illinois.

| | | |
|---|---|---|
| Paste B: | | |
| | Ethoxylated bisphenol-A-dimethacrylate | 14.94% |
| | 2-Isopropyl thioxanthone | 0.06% |
| | Ethyl 4-dimethylaminobenzoate | 3.00% |
| | Strontium glass | 79.8784% |
| | Fumed silica | 0.92% |
| | A-174 Silane | 1.20% |
| | Glacial acetic acid | 0.0016% |

Equal or substantially equal amounts of pastes A and B are mixed together just before use.

Example 12

A series of five pastes was made up as shown in Table IV. Pastes O, P, and Q contained light-activated curatives, and no peroxide. Pastes T and U contained peroxide and no light-activated curatives. These materials were mixed in equal parts, according to the plan shown in Table V. The resultant mixed pastes were exposed to the output from a Visar dental curing unit for 30 seconds, and the depth of cure was measured. The results are shown in Table V. The samples were further tested (as described previously) to determine the time required to achieve a 12 mm (or "inifinte") depth of cure. These results are shown in Table VI.

TABLE IV

Paste embodiment key for paste-paste charts

| | |
|---|---|
| **Paste O:** | |
| Ethoxylated bisphenol-A-dimethacrylate | 16.07% |
| 2-Isopropyl thioxanthone | .03% |
| Ethyl 4-dimethylaminobenzoate | 1.49% |
| Barium glass | 24.17% |
| Lithium aluminum silicate | 56.49% |
| A-174 Silane | .81% |
| Glacial acetic acid | .04% |
| Aerosil 972 | .90% |
| **Paste P:** | |
| Ethoxylated bisphenol-A-dimethacrylate | 16.44% |
| 2-Isopropyl thioxanthone | .015% |
| Ethyl 4-dimethylaminobenzoate | 1.52% |
| Titanium dioxide | .05% |
| Aerosil 972 | .92% |
| Barium glass | 24.025% |
| Lithium aluminum silicate | 56.14% |
| A-174 Silane | .80% |
| Glacial acetic acid | .04% |
| **Paste Q:** | |
| Ethoxylated bisphenol-A-dimethacrylate | 14.83% |
| 2-Isopropyl thioxanthone | .054% |
| Ethyl 4-dimethylaminobenzoate | 2.706% |
| Barium glass | 24.17% |
| Lithium aluminum silicate | 56.49% |
| A-174 silane | .81% |
| Glacial acetic acid | .04% |
| Aerosil 972 (fumed silica) | .90% |
| **Paste T:** | |
| Ethoxylated bisphenol-A-dimethacrylate | 18.00% |
| Aerosil (fumed silica) | .92% |
| Benzoyl peroxide | .74% |
| Barium glass | 24.72% |
| Lithium aluminum silicate | 54.413% |
| A-174 Silane | 1.19% |
| Glacial acetic acid | .017% |
| **Paste U:** | |
| Ethoxylated bisphenol-A-dimethacrylate | 16.42% |
| Aerosil (fumed silica) | .92% |
| Benzoyl peroxide | .72% |
| Barium glass | 24.10% |
| Lithium aluminum silicate | 57.364% |
| A-174 Silane | .41% |
| Butylated hydroxytoluene | .066% |

In the above paste-paste examples, it must be borne in mind that the peroxide content of the total mixed system is only about half that shown in the formulation, as the two pastes are mixed together at a 1:1 ratio and one paste has no peroxide at all.

TABLE V
Paste-paste systems

Depth (in mm) of cure
immediately after 30 second light exposure

| Paste containing light curatives | Paste containing BPO** in resin | Paste containing BPO** on powder | Depth |
|---|---|---|---|
| O* | | | 4.50 |
| P | | | 2.85 |
| O | | T | 5.04 |
| Q | | T | 5.15 |
| O | U | | 2.65 |
| Q | U | | 3.75 |

*See key to paste embodiments
**BPO—Benzoyl peroxide

TABLE VI
Paste-paste systems

Time in (min) after light exposure to
obtain "inifinte" depth of cure

| Paste containing light curatives | Paste containing BPO** in resin | Paste containing BPO** on powder | Time |
|---|---|---|---|
| O* | | | N/A |
| P | | | N/A |
| O | | T | 4 |
| Q | | T | 4 |
| O | U | | 18 |
| Q | U | | 7 |

*See key to paste embodiments
**BPO—Benzoyl peroxide

Paste-powder systems

A third type of system is somewhat of a blend between the paste-liquid system and the paste-paste system. Here, the powder remains substantially the same as in the powder-liquid system except that it has a larger percentage of peroxide. However, the paste contains both the light-active curatives dissolved in a liquid resin, and the filler, suspended therein. This paste material would be stored in a light-free container. Typically, a black polyethylene or polypropylene syringe may be used as the container, as in the paste-paste systems.

The powder is coated with a suitable silane, such as gamma-methacryloxypropyltrimethoxysilane, and has benzoyl peroxide dispersed over its surface. This component may be stored in a container suited to dispersing small amounts. A small cylindrical plastic vial with a small-orificed dropper tip would be preferred.

In practice, an amount of the paste is expressed onto a mixing pad, then a very small amount—1/10 to 1/20—of the powder is mixed in; so the powder has more peroxide in it than in previous systems discussed. The result is that the paste is thickened—a condition desired by some dentists—and also that depth of cure and cure efficiency are improved.

A general formulation may be expressed as follows:

| | | Percent by weight |
|---|---|---|
| Paste: | | |
| | Resin | 18 to 40 |
| | Filler | 80 to 60 |
| | Photoinitiators | 0.06 to 12.8 |
| | A-174 Silane | 1.23 to 0.06 |
| Powder: | | |
| | Filler material | 99.75 to 97.74 |
| | A-174 Silane | 0.10 to 1.50 |
| | Peroxide curing agent | 0.30 to 10 |

The mixture is preferably done in a ratio varying between 20 to 1 of paste to powder to 10 to 1 of paste to powder. Hence the relatively large amount of peroxide curing agent in the powder. More specifically, this system may comprise the following, with resin, filler, etc., ingredients always lying within the above-given ranges:

| | | Percent by weight |
|---|---|---|
| Paste: | | |
| | Bis-GMA | 0 to 32 |
| | Ethoxylated bisphenol-A-dimethacrylate | 0 to 40 |
| | Ethylene glycol dimethacrylate | 0 to 24 |
| | Diethylene glycol dimethacrylate | 0 to 24 |
| | Triethylene glycol dimethacrylate | 0 to 24 |
| | Polyethylene glycol dimethacrylate | 0 to 24 |
| | Barium glass | 0 to 25 |
| | Lithium aluminum silicate | 0 to 82 |
| | Flint silica | 0 to 8.5 |
| | Borosilicate glass | 0 to 82 |
| | Fumed synthetic silica | 0 to 52 |
| | Strontium glass | 0 to 82 |
| | Quartz | 0 to 82 |
| | Titanium dioxide | 0 to 0.13 |
| | Tinting agent (e.g., iron oxides) | 0 to 4 |
| | A-174 Silane | 0 to 1.25 |
| | Photoinitiators | 0.06 to 12.8 |
| Powder: | | |
| | Barium glass | 0 to 30 |
| | Lithium aluminum silicate | 0 to 99.75 |
| | Flint silica | 0 to 10 |
| | Borosilicate glass | 0 to 99.75 |
| | Fumed synthetic silica | 0 to 99.75 |
| | Strontium glass | 0 to 99.75 |
| | Titanium dioxide | 0 to 0.15 |
| | Tinting agent (e.g., iron oxides) | 0 to 5 |
| | A-174 Silane | 0 to 1.50 |
| | Peroxide curing agent (e.g. benzoyl peroxide) | 0.30 to 10 |

Example 13

Paste-powder system

| | | |
|---|---|---|
| Paste: | | |
| | Ethoxylated bisphenol-A-dimethacrylate | 16.07% |
| | 2-Isopropyl thioxanthone | 0.03% |
| | Ethyl 4-dimethyl amino benzoate | 1.49% |
| | Fumed silica | 0.90% |
| | Lithium aluminum silicate | 57.08% |
| | Barium glass | 24.45% |
| Powder: | | |
| | Fumed silica (pre-coated with silane) | 95.00% |
| | Benzoyl peroxide | 5.00% |

When a small amount (10% by weight) of the powder is added to the paste, the resulting heavy paste has a working time of over 20 minutes. When exposed to the Visar curing light for 10 seconds, the depth of

cure measured at least 4.97 mm, the limit of the test fixture used, when measured ten minutes later. Diametral tensile strength was 361.3504 $10^5$ Pa (5240 psi).

Example 14

Paste-powder system

Both the paste and powder are as in Example 13, but a smaller amount (5% by weight) of the powder is added to the paste. The resulting thick paste has a working time of about 30 minutes in ordinary room light. When exposed to a Visar light for 10 seconds, the depth of cure was at least 4.97 mm, that being the limit of the test apparatus used. Diametral tensile strength is 342.0416 $10^5$ Pa (4960 psi).

Example 15

Paste-powder system

| | | Parts by weight |
|---|---|---|
| Paste: | | |
| | Bis-GMA | 60 |
| | Triethylene glycol dimethacrylate | 40 |
| | 2-Isopropyl thioxanthone | 0.18 |
| | Ethyl-4-dimethyl aminobenzoate | 9.10 |
| | Lithium aluminum silicate | 355 |
| | Barium glass | 152 |
| Powder: | | |
| | Lithium aluminum silicate | 70 |
| | Barium glass | 30 |
| | Benzoyl peroxide | 0.75 |

The paste and powder are mixed at a weight ratio of 100:5, paste:powder. When exposed to a Visar II curing unit for 10 seconds, the depth of cure is 4.97 mm. The time to a 12 mm cure is 18 minutes. The diametral tensile strength is 360.316 $10^5$ Pa (5225 psi).

Example 16

Paste-powder system

| | | Parts by weight |
|---|---|---|
| Paste: | | |
| | Ethoxylated bisphenol-A-dimethacrylate | 80 |
| | Triethylene glycol dimethacrylate | 20 |
| | 2-Isopropyl thioxanthone | 0.18 |
| | Ethyl 4-dimethyl aminobenzoate | 9.10 |
| | Lithium aluminum silicate | 355 |
| | Barium glass | 152 |
| Powder: | | |
| | Lithium aluminum silicate | 70 |
| | Barium glass | 30 |
| | Benzoyl peroxide | 0.30 |

The paste and powder are mixed together at a weight ratio of 100:5, paste:powder. When exposed to the output from a Visar II curing unit for 10 seconds, the depth of cure is 4.50 mm. The time to a 12 mm cure is 25 minutes. The diametral tensile strength is 362.04 $10^5$ Pa (5250 psi).

Gel-powder systems

Using powder like that used in the powder-liquid system but employing a gel instead of a liquid, a general formulation for these systems may be as follows:

| | | Percent by weight |
|---|---|---|
| Gel: | | |
| | Resin | 50 to 90 |
| | Filler | 49.82 to 10 |
| | A-174 Silane | 1.23 to 0.06 |
| | Photoinitiator | 0.09 to 14.40 |
| Powder: | | |
| | Filler material | 99.75 to 97.74 |
| | A-174 Silane | 0.10 to 1.50 |
| | Peroxide curing agent | 0.15 to 0.76 |

The mixture may vary from about one part of powder to twenty parts of gel to about three parts of powder to one part of gel.

More specifically, while adhering to the general formulation above, the system may use individual ingredients as follows:

| | Percentages by weight |
|---|---|
| **Gel:** | |
| Bis-GMA | 0 to 72 |
| Ethoxylated bisphenol-A-dimethacrylate | 0 to 90 |
| Ethylene glycol dimethacrylate | 0 to 54 |
| Diethylene glycol dimethacrylate | 0 to 54 |
| Triethylene glycol dimethacrylate | 0 to 54 |
| Polyethylene glycol dimethacrylate | 0 to 54 |
| Barium glass | 0 to 14.95 |
| Lithium aluminum silicate | 0 to 49.82 |
| Flint silica | 0 to 4.98 |
| Borosilicate glass | 0 to 49.82 |
| Fumed synthetic silica | 0 to 49.82 |
| Strontium glass | 0 to 49.82 |
| Quartz | 0 to 49.82 |
| Titanium dioxide | 0 to 0.15 |
| Tinting agent (e.g., iron oxides) | 0 to 5 |
| A-174 Silane | 0 to 0.75 |
| Photoinitiator | 0.09 to 14.40 |
| **Powder:** | |
| Barium glass | 0 to 30 |
| Lithium aluminum silicate | 0 to 99.75 |
| Flint silica | 0 to 10 |
| Borosilicate glass | 0 to 99.75 |
| Fumed synthetic silica | 0 to 99.75 |
| Quartz | 0 to 99.75 |
| Strontium glass | 0 to 99.75 |
| Titanium dioxide | 0 to 1.50 |
| Tinting agent (e.g., iron oxide) | 0 to 5 |
| A-174 Silane | 0 to 1.50 |
| Peroxide curing agent | 0.15 to 0.76 |

### Example 17
### Gel-powder system

| | |
|---|---|
| **Gel:** | |
| Ethoxylated bisphenol-A-dimethacrylate | 76.40% |
| 2-Isopropylthioxanthone | 0.06% |
| Ethyl 4-dimethylaminobenzoate | 6.90% |
| Fumed silica | 8.30% |
| Barium glass | 2.43% |
| Lithium aluminum silicate | 5.74% |
| A-174 Silane | 0.08% |
| Glacial acetic acid | 0.04% |

Powder:

The powder may be the powder of Example 1 or that of Example 7

Preferably, the peroxide and silane are again deposited on the powdered glass and silicates as by dissolving the peroxide and the silane in a suitable solvent, such as methylene chloride, chloroform, ether, or acetone, making a slurry with the powder, and then stripping the solvent.

The powder and gel are mixed together in weight ratios of from about 1:20 to 3:1, powder to gel. Mixing, working, and cure time are like those for Examples 1 and 7.

Example 18
Gel-powder system

Gel:

| | |
|---|---|
| Ethoxylated bisphenol-A-dimethacrylate | 76.34% |
| 2-Isopropylthioxanthone | 0.14% |
| Ethyl 4-dimethylaminobenzoate | 6.90% |
| Fumed silica | 16.62% |

Powder:
The powder may be that shown in either of Example 1 or Example 7

Example 19
Gel-powder system

The gel is as in Example 17, and the powder is as in Example 1.

Here they are mixed at a weight ratio of 2:1, powder:gel. The working time under room lights is 20 minutes. The depth of cure after 10 seconds Visar light exposure is 3.50 mm. The length of time to a 12 mm cure is 18 minutes. The diametral tensile strength is 343.076 $10^5$ Pa (4975 psi).

This restorative system may be used in the repair of fractured porcelain material, natural teeth, or in any other intra-oral situation where a resin material is desired to replace or simulate tooth structure that is capable of being polymerized by visible light and having the process continue after the light is removed.

Also, this restorative system, which is essentially a light-cured system, achieves uniform polymerization within thirty minutes to one hour after insertion in the patient's mouth, even though variable amounts of light be exposed to the polymerizing resin.

This self-curing system eliminates the color changes that tend to occur when certain resins mixed with curing resins are exposed to ultra-violet or visible light.

The present invention therefore further relates to a method for repairing porcelain or teeth with the aid of a two-part system, said method comprising the steps of:

mixing together under ordinary indoor lighting conditions:

— a methacrylate functional resin usable in dental composites and photoinitiator for said resin, said photoinitiator including both (1) 2-isopropylthioxanthone or benzil dimethyl acetal and (2) either ethyl 4-dimethylaminobenzoate or ethyl 2-dimethylaminobenzoate (part 1), and

— powdered dental filler and at least one accelerator-free peroxide curing agent for said resin (part 2),

said two-part system being either

(a) a paste-paste system,
(b) a paste-powder system,
(c) a gel-powder system or
(d) a liquid-powder system,

said photoinitiator, peroxide curing agent and first and second parts in said systems being present in an amount of:

| System | Photoinitiator | Peroxide curing agent | Part 1:Part 2 |
|---|---|---|---|
| (a) | 0.06—12.8 wt% | 0.30—0.75 wt% | 1:1 |
| (b) | 0.06—12.8 wt% | 0.30—10 wt% | 20:1—10:1 |
| (c) | 0.09—14.40 wt% | 0.15—0.76 wt% | 20:1—1:3 |
| (d) | 0.18—16 wt% | 0.15—0.76 wt% | 1:2—1:3.5 |

whereby the percentages are based on the components of that part of the system containing the respective compound;

emplacing the mixture within a few minutes of the mixing, and curing a substantial, expressed portion of the emplaced mixture *in situ* under intense illumination, any resin then uncured by light being cured within the next hour by said peroxide curing agent.

The present invention is also concerned with a method for preparing a two-part storable filled-resin composition useful for porcelain repair and as a dental composite comprising:

coating, in order to prepare the second part, a powdered dental filler with at least one accelerator-free peroxide curing agent for said resin;

storing said filler;

mixing, in order to prepare the first part, an uncured methacrylate functional resin usable in dental composites with photoinitiator for said resin;

said photoinitiator including both (1) 2-isopropylthioxanthone or benzil dimethyl acetal and (2) either ethyl 4-dimethylaminobenzoate or ethyl 2-dimethylaminobenzoate;

storing the resin-photoinitiator mixture in an opaque container;

said two-part system being either

(a) a paste-paste system,
(b) a paste-powder system,
(c) a gel-powder system or
(d) a liquid-powder system,

said photoinitiator, peroxide curing agent and first and second parts in said systems being present in an amount of:

| System | Photoinitiator | Peroxide curing agent | Part 1:Part 2 |
|---|---|---|---|
| (a) | 0.06—12.8 wt% | 0.30—0.75 wt% | 1:1 |
| (b) | 0.06—12.8 wt% | 0.30—10 wt% | 20:1—10:1 |
| (c) | 0.09—14.40 wt% | 0.15—0.76 wt% | 20:1—1:3 |
| (d) | 0.18—16 wt% | 0.15—0.76 wt% | 1:2—1:3.5 |

whereby the percentages are based on the components of that part of the system containing the respective compound.

**Claims**

1. A two-part system for making a filled methacrylate-functional resin composition by mixture of the two-parts, comprising:

first part: dental-type methacrylate-functional resin with photoinitiator therefor;

second part: dental-type filler powder with an accelerator-free peroxide curing agent for said resin;

said photoinitiator containing both (1) 2-isopropylthioxanthone or a benzil dimethyl acetal and (2) either ethyl 4-dimethylaminobenzoate or ethyl 2-dimethylaminobenzoate;

said two-part system being either

(a) a paste-paste system,
(b) a paste-powder system,
(c) a gel-powder system,
(d) a liquid-powder system,

said photoinitiator, peroxide curing agent and first and second parts in said systems being present in an amount of:

| System | Photoinitiator | Peroxide curing agent | Part 1:Part 2 |
|---|---|---|---|
| (a) | 0.06—12.8 wt% | 0.30—0.75 wt% | 1:1 |
| (b) | 0.06—12.8 wt% | 0.30—10 wt% | 20:1—10:1 |
| (c) | 0.09—14.40 wt% | 0.15—0.76 wt% | 20:1—1:3 |
| (d) | 0.18—16 wt% | 0.15—0.76 wt% | 1:2—1:3.5 |

whereby the percentages are based on the components of that part of the system containing the respective compound.

2. The system of claim 1 wherein the first part is in the form of a powder and the second part is in the form of a gel which also contains filler.

3. The system of claim 1 wherein both the first and second parts are in paste form, both containing filler.

4. The system of claim 3 wherein the first and second parts are so formulated to enable mixture of equal parts by weight at the time of use.

5. A liquid-powder system according to claim 1 consisting by weight, of

said powder component, consisting by weight, of:

| | |
|---|---|
| Barium glass | 0% to 30.00% |
| Lithium aluminium silicate | 0% to 99.75% |
| Flint silica | 0% to 10.00% |
| Borosilicate glass | 0% to 99.75% |
| Fumed synthetic silica | 0% to 99.75% |
| Quartz | 0% to 99.75% |
| Titanium dioxide | 0% to 0.15% |
| Tinting agents (e.g. iron oxides) | 0% to 5.00% |
| Gamma-methacryloxypropyltrimethoxysilane | 0% to 1.50% |
| Peroxide curing agent | 0.15% to 0.76% |

and a liquid component consisting, by weight, of:

| | |
|---|---|
| Bis-GMA | 0% to 80.00% |
| Ethoxylated bisphenol-A-dimethacrylate | 0% to 99.00% |
| Ethylene glycol dimethacrylate | 0% to 60.00% |
| Diethylene glycol dimethacrylate | 0% to 60.00% |
| Triethylene glycol dimethacrylate | 0% to 60.00% |
| Polyethylene glycol dimethacrylate | 0% to 60.00% |
| Said photoinitiator for the above resins | 0.18% to 16.00% |

said powder component and said liquid component being mixed, at time of use, in weight ratio of 1:1 to 3.5:1 powder to liquid.

6. A liquid-powder system according to claim 1, consisting of said powder component, consisting by weight, of:

| | |
|---|---|
| Dental filler material | 99.75% to 97.74% |
| Gamma-methacryloxypropyltrimethoxysilane | 0.10% to 1.50% |
| Peroxide curing agent | 0.15% to 0.76% |

and said liquid component consisting, by weight, of:

| | |
|---|---|
| said methacrylate functional resin suitable for use in dental composites | 99.82% to 84.00% |
| said photoinitiator for said resin | 0.18% to 16.00% |

said powder and resin being mixed together in a weight ratio between 1:1 and 3.5:1 of powder to liquid.

7. A liquid-powder system according to claim 1 wherein said powder component consists of:

| | |
|---|---|
| Barium glass | 29.82% |
| Lithium aluminium silicate | 67.30% |
| Flint silica | 1.22% |
| $TiO_2$ | 0.02% |
| Benzoyl peroxide | 0.15% |
| Gamma-methacryloxypropyltrimethoxysilane | 1.47% |
| Glacial acetic acid | 0.02% |

and said liquid component consists of:

| | |
|---|---|
| Ethoxylated bisphenol-A-dimethacrylate | 91.50% |
| 2-Isopropylthioxanthone | 0.17% |
| Ethyl 4-dimethylaminobenzoate | 8.33% |

said powder and liquid components being mixed together just before use in a weight ratio of from 2:1 to 3.5:1 powder to liquid.

8. A liquid-powder system according to claim 1 wherein said powder consists of:

| | |
|---|---|
| Barium glass | 26.66% |
| Lithium aluminium silicate | 62.24% |
| Flint silica | 9.01% |
| Benzoyl peroxide | 0.60% |
| Gamma-methacryloxypropyltrimethoxysilane | 1.47% |
| Glacial acetic acid | 0.02% |

and said liquid consists of:

| | |
|---|---|
| Ethoxylated bisphenol-A-dimethacrylate | 91.50% |
| 2-Isopropylthioxanthone | 0.17% |
| Ethyl 4-dimethylaminobenzoate | 8.33% |

said liquid and powder being mixed together in a weight ratio of 2 to 3.5:1 powder:liquid.

9. A liquid-powder system according to claim 1 wherein said liquid component consists of:

| | |
|---|---|
| 2,2′-propane bis [3-(4-phenoxy)-,2-hydroxypropane-1-methacrylate] | 54.6% |
| Triethylene glycol dimethacrylate | 36.4% |
| 2-Isopropylthioxanthone | 0.02% |
| Ethyl 4-dimethylaminobenzoate | 8.98% |

and said powder component consists of:

| | |
|---|---|
| Lithium aluminium silicate | 69.79% |
| Barium glass | 29.91% |
| Benzoyl peroxide | 0.30% |

the powder and liquid being mixed for use at a weight ratio of three parts powder to one part liquid.

10. A liquid-powder system according to claim 1, wherein said liquid component consists of:

| | |
|---|---|
| Ethoxylated bisphenol-A-dimethacrylate | 72.8% |
| Triethylene glycol dimethacrylate | 18.2% |
| 2-Isopropylthioxanthone | 0.02% |
| Ethyl 4-dimethylaminobenzoate | 8.98% |

a powder component consisting of:

| | |
|---|---|
| Lithium aluminum silicates | 69.79% |
| Barium glass | 29.91% |
| Benzoyl peroxide | 0.30% |

the powder and the liquid being mixed together at the time of use at a weight ratio of about three parts powder to one part of liquid.

11. A liquid-powder system according to claim 1 wherein said powder component consists of:

| | Parts by weight |
|---|---|
| Strontium glass | 100 |
| Benzoyl peroxide | 0.3 |
| Gamma-methacryloxypropyltrimethoxysilane | 1.5 |

and said liquid component consists of:

| | |
|---|---|
| Ethoxylated bisphenol-A-dimethacrylate | 91.50% |
| 2-Isopropylthioxanthone | 0.17% |
| Ethyl 4-dimethylaminobenzoate | 8.33% |

said powder and liquid components being mixed together just before use in a weight ratio of from 3:1 powder to liquid.

12. A paste-paste system according to claim 1 consisting, in percentages by weight of:

| | |
|---|---|
| Paste A: | |
| Methacrylate functional resin suitable for use in dental composites | 18 to 40 |
| Dental filler material | 82 to 60 |
| Peroxide curing agent | 0.75 to 0.30 |
| Gamma-methacryloxypropyltrimethoxysilane | 1.23 to 0.06 |
| Butylated hydroxytoluene | 0.01 to 0.10 |
| Paste B: | |
| Methacrylate functional resin suitable for use in dental composites | 18 to 40 |
| Dental filler material | 80 to 60 |
| Said photoinitiators | 0.06 to 12.8 |
| Gamma-methacryloxypropyltrimethoxysilane | 1.23 to 0.06 |

pastes A and B being mixed together at time of use in substantially equal amounts by weight.

13. The system of claim 12 wherein Paste A consists in percentages by weight of:

| | |
|---|---|
| Bis-GMA | 0 to 32 |
| Ethoxylated bisphenol-A-dimethacrylate | 0 to 40 |
| Ethylene glycol dimethacrylate | 0 to 24 |
| Diethylene glycol dimethacrylate | 0 to 24 |
| Triethylene glycol dimethacrylate | 0 to 24 |
| Polyethylene glycol dimethacrylate | 0 to 24 |
| Barium glass | 0 to 25 |
| Lithium aluminium silicate | 0 to 82 |
| Flint silica | 0 to 8.5 |
| Borosilicate glass | 0 to 82 |
| Fumed synthetic silica | 0 to 52 |
| Quartz | 0 to 82 |
| Strontium glass | 0 to 82 |
| Titanium dioxide | 0 to 0.13 |
| Tinting agents (e.g., iron oxides) | 0 to 4 |
| Gamma-methacryloxypropyltrimethoxysilane | 0 to 1.25 |
| Peroxide curing agent | 0.30 to 0.75 |

and Paste B consists in percentages by weight of:

| | |
|---|---|
| Bis-GMA | 0 to 32 |
| Ethoxylated bisphenol-A-dimethacrylate | 0 to 40 |
| Ethylene glycol dimethacrylate | 0 to 24 |
| Diethylene glycol dimethacrylate | 0 to 24 |
| Triethylene glycol dimethacrylate | 0 to 24 |
| Polyethylene glycol dimethacrylate | 0 to 24 |
| Barium glass | 0 to 25 |
| Lithium aluminium silicate | 0 to 82 |
| Flint silica | 0 to 8.5 |
| Borosilicate glass | 0 to 82 |
| Fumed synthetic silica | 0 to 52 |
| Quartz | 0 to 82 |
| Strontium glass | 0 to 82 |
| Titanium dioxide | 0 to 0.13 |
| Tinting agents (e.g., iron oxides) | 0 to 4 |
| Gamma-methacryloxypropyltrimethoxysilane | 0 to 1.25 |
| Said photoinitiators | 0.10 to 12.8 |

14. The system of claim 12 wherein said first paste consists of:

| | |
|---|---|
| Ethoxylated bisphenol-A-dimethacrylate | 17.36% |
| Benzoyl peroxide | 0.60% |
| Butylated hydroxytoluene | 0.04% |
| Strontium glass | 79.8784% |
| Fumed silica | 0.92% |
| Gamma-methacryloxypropyltrimethoxysilane | 0.20% |
| Glacial acetic acid | 0.0016% |

and said second paste consists of:

| | |
|---|---|
| Ethoxylated bisphenol-A-dimethacrylate | 14.94% |
| 2-Isopropylthioxanthone | 0.06% |
| Ethyl 4-dimethylaminobenzoate | 3.00% |
| Strontium glass | 79.8784% |
| Fumed silica | 0.92% |
| Gamma-methacryloxypropyltrimethoxysilane | 1.20% |
| Glacial acetic acid | 0.0016% |

15. A gel-powder system according to claim 1, consisting in parts by weight of:

a gel, consisting of:

| | |
|---|---|
| methacrylate functional resin suitable for use in dental composites | 50 to 90 |
| dental filler material | 49.82 to 10 |
| gamma-methacryloxypropyltrimethoxysilane | 1.23 to 0.06 |
| said photoinitiator | 0.09 to 14.40 |

and a powder, consisting of:

| | |
|---|---|
| dental filler material | 99.75 to 97.74 |
| gamma-methacryloxypropyltrimethoxysilane | 0.10 to 1.50 |
| peroxide curing agent | 0.15 to 0.76 |

the gel and the powder being mixed together at the time of use in a weight ratio of gel to powder of 20:1 to 1:3.

16. The system of claim 15 wherein the ingredients of the gel and of the powder are made up, by weight, of the following:

Gel:

| | |
|---|---|
| Bis-GMA | 0 to 72% |
| Ethoxylated bisphenol-A-dimethacrylate | 0 to 90% |
| Ethylene glycol dimethacrylate | 0 to 54% |
| Diethylene glycol dimethacrylate | 0 to 54% |
| Triethylene glycol dimethacrylate | 0 to 54% |
| Polyethylene glycol dimethacrylate | 0 to 54% |
| Barium glass | 0 to 14.95% |
| Lithium aluminium silicate | 0 to 49.82% |
| Flint silica | 0 to 4.98% |
| Borosilicate glass | 0 to 49.82% |
| Fumed synthetic silica | 0 to 49.82% |
| Strontium glass | 0 to 49.82% |
| Quartz | 0 to 49.82% |
| Titanium dioxide | 0 to 0.15% |
| Tinting agents (e.g., iron oxides) | 0 to 5% |
| Gamma-methacryloxypropyltrimethoxysilane | 0 to 0.75% |
| Said photoinitiator | 0.09 to 14.40% |

Powder:

| | |
|---|---|
| Barium glass | 0 to 30% |
| Lithium aluminium silicate | 0 to 99.75% |
| Flint silica | 0 to 10% |
| Borosilicate glass | 0 to 99.75% |
| Fumed synthetic silica | 0 to 99.75% |
| Quartz | 0 to 99.75% |
| Strontium glass | 0 to 99.75% |
| Titanium dioxide | 0 to 1.50% |
| Tinting agents (e.g., iron oxides) | 0 to 5% |
| Gamma-methacryloxypropyltrimethoxysilane | 0 to 1.50% |
| Peroxide curing agent | 0.15 to 0.76% |

17. The system of claim 15 wherein said gel consists, by weight of:

| | |
|---|---|
| Ethoxylated bisphenol-A-dimethacrylate | 76.40% |
| 2-Isopropylthioxanthone | 0.06% |
| Ethyl 4-dimethylaminobenzoate | 6.90% |
| Fumed silica | 8.30% |
| Barium glass | 2.43% |
| Lithium aluminium silicate | 5.74% |
| Gamma-methacryloxypropyltrimethoxysilane | 0.08% |
| Glacial acetic acid | 0.04% |

and said powder consists of:

| | |
|---|---|
| Barium glass | 29.82% |
| Lithium aluminium silicate | 67.30% |
| Flint silica | 1.22% |
| $TiO_2$ | 0.02% |
| Benzoyl peroxide | 0.15% |
| Gamma-methacryloxypropyltrimethoxysilane | 0.47% |
| Glacial acetic acid | 0.02% |

the gel and powder being mixed at the time of use in a weight ratio between 1:20 and 3:1 of powder to gel.

18. The system of claim 15, wherein said gel consists, by weight, of:

| | |
|---|---|
| Ethoxylated bisphenol-A-dimethacrylate | 76.40% |
| 2-Isopropylthioxanthone | 0.06% |
| Ethyl 4-dimethylaminobenzoate | 6.90% |
| Fumed silica | 8.30% |
| Barium glass | 2.43% |
| Lithium aluminium silicate | 5.74% |
| Gamma-methacryloxypropyltrimethoxysilane | 0.08% |
| Glacial acetic acid | 0.04% |

and said powder consists of:

| | |
|---|---|
| Barium glass | 26.66% |
| Lithium aluminium silicate | 62.24% |
| Flint silica | 9.01% |
| Benzoyl peroxide | 0.60% |
| Gamma-methacryloxypropyltrimethoxysilane | 1.47% |
| Glacial acetic acid | 0.02% |

the gel and powder being mixed at the time of use in a weight ratio between 1:20 and 3:1 of powder to gel.

19. The system of claim 15, wherein said gel consists, by weight, of:

| | |
|---|---|
| Ethoxylated bisphenol-A-dimethacrylate | 76.34% |
| 2-Isopropylthioxanthone | 0.14% |
| Ethyl 4-dimethylaminobenzoate | 6.90% |
| Fumed silica | 16.62% |

and said powder consists of:

| | |
|---|---|
| Barium glass | 29.82% |
| Lithium aluminium silicate | 67.30% |
| Flint silica | 1.22% |
| $TiO_2$ | 0.02% |
| Benzoyl peroxide | 0.15% |
| Gamma-methacryloxypropyltrimethoxysilane | 0.47% |
| Glacial acetic acid | 0.02% |

the gel and powder being mixed at the time of use in a weight ratio between 1:20 and 3:1 of powder to gel.

20. A paste-powder system according to claim 1, consisting, in percentages by weight, of:

a paste, consisting of:

| | |
|---|---|
| Methacrylate functional resin suitable for use in dental composites | 18 to 40 |
| Dental filler material | 80 to 60 |
| Said photoinitiators | 0.06 to 12.8 |
| Gamma-methacryloxypropyltrimethoxysilane | 1.23 to 0.06 |

and a powder consisting of:

| | |
|---|---|
| Dental filler material | 99.75 to 97.74 |
| Gamma-methacryloxypropyltrimethoxysilane | 1.10 to 1.50 |
| Peroxide curing agent | 0.30 to 10 |

said powder being added to said paste at time of use in a weight ratio of 20:1 to 10:1 of paste to powder.

21. The system of claim 20 wherein said paste and powder are those from the following ingredients, in percentages by weight:

| | | |
|---|---|---|
| Paste: | | |
| | Bis-GMA | 0 to 32 |
| | Ethoxylated bisphenol-A-dimethacrylate | 0 to 40 |
| | Ethylene glycol dimethacrylate | 0 to 24 |
| | Diethylene glycol dimethacrylate | 0 to 24 |
| | Triethylene glycol dimethacrylate | 0 to 24 |
| | Polyethylene glycol dimethacrylate | 0 to 24 |
| | Barium glass | 0 to 25 |
| | Lithium aluminium silicate | 0 to 82 |
| | Flint silica | 0 to 8.5 |
| | Borosilicate glass | 0 to 82 |
| | Fumed synthetic silica | 0 to 52 |
| | Quartz | 0 to 82 |
| | Strontium glass | 0 to 82 |
| | Titanium dioxide | 0 to 0.13 |
| | Tinting agents (e.g., iron oxides) | 0 to 4 |
| | Gamma-methacryloxypropyltrimethoxysilane | 0 to 1.25 |
| | Said photoinitiators | 0.06 to 12.8 |
| Powder: | | |
| | Barium glass | 0 to 30 |
| | Lithium aluminium silicate | 0 to 99.75 |
| | Flint silica | 0 to 10 |
| | Borosilicate glass | 0 to 99.75 |
| | Fumed synthetic silica | 0 to 99.75 |
| | Strontium glass | 0 to 99.75 |
| | Titanium dioxide | 0 to 0.15 |
| | Tinting agents (e.g., iron oxides) | 0 to 5 |
| | Gamma-methacryloxypropyltrimethoxysilane | 0 to 1.50 |
| | Peroxide curing agent | 0.30 to 10 |

22. The system of claim 20 wherein said paste component consists of:

| | |
|---|---|
| Ethoxylated bisphenol-A-dimethacrylate | 16.07% |
| 2-Isopropylthioxanthone | 0.03% |
| Ethyl 4-dimethylaminobenzoate | 1.49% |
| Fumed silica | 0.90% |
| Lithium aluminium silicate | 57.08% |
| Barium glass | 24.45% |

and said powder component consists of:

| | |
|---|---|
| Fumed silica (pre-coated with silane) | 95.00% |
| Benzoyl peroxide | 5.00% |

the powder being added to the paste at the time of use in a weight ratio of 20:1 to 10:1 of paste to powder.

23. The system of claim 20 wherein said paste component consists of:

| | Parts by weight |
|---|---|
| Bis-GMA | 60 |
| Triethylene glycol dimethacrylate | 40 |
| 2-Isopropylthioxanthone | 0.18 |
| Ethyl 4-dimethylaminobenzoate | 9.10 |
| Lithium aluminium silicate | 355 |
| Barium glass | 152 |

and said powder component consists of:

| | Parts by weight |
|---|---|
| Lithium aluminium silicate | 70 |
| Barium glass | 30 |
| Benzoyl peroxide | 0.75 |

the paste and powder being mixed at the time of use in a weight ratio of 20:1 paste:powder.

24. The system of claim 20, wherein said paste component consists of:

| | Parts by weight |
|---|---|
| Ethoxylated bisphenol-A-dimethacrylate | 80 |
| Triethylene glycol dimethacrylate | 20 |
| 2-Isopropylthioxanthone | 0.18 |
| Ethyl 4-dimethylaminobenzoate | 9.10 |
| Lithium aluminium silicate | 355 |
| Barium glass | 152 |

and said powder component consists of:

| | Parts by weight |
|---|---|
| Lithium aluminium silicate | 70 |
| Barium glass | 30 |
| Benzoyl peroxide | 0.30 |

the paste and powder being mixed together at the time of use in a weight ratio of 20:1 paste:powder.

25. A method for repairing porcelain or teeth with the aid of a two-part system, said method comprising the steps of:

mixing together under ordinary indoor lighting conditions:

— a methacrylate functional resin usable in dental composites and photoinitiator for said resin, said photoinitiator including both (1) 2-isopropylthioxanthone or benzil dimethyl acetal and (2) either ethyl 4-dimethylaminobenzoate or ethyl 2-dimethylaminobenzoate (part 1), and

— powdered dental filler and at least one accelerator-free peroxide curing agent for said resin (part 2),

said two-part system being either

(a) a paste-paste system,

(b) a paste-powder system,

(c) a gel-powder system or

(d) a liquid-powder system,

said photoinitiator, peroxide curing agent and first and second parts in said systems being present in an amount of:

| System | Photoinitiator | Peroxide curing agent | Part 1:Part 2 |
|---|---|---|---|
| (a) | 0.06—12.8 wt% | 0.30—0.75 wt% | 1:1 |
| (b) | 0.06—12.8 wt% | 0.30—10 wt% | 20:1—10:1 |
| (c) | 0.09—14.40 wt% | 0.15—0.76 wt% | 20:1—1:3 |
| (d) | 0.18—16 wt% | 0.15—0.76 wt% | 1:2—1:3.5 |

whereby the percentages are based on the components of that part of the system containing the respective compound;

emplacing the mixture within a few minutes of the mixing, and curing a substantial, expressed portion of the emplaced mixture *in situ* under intense illumination, any resin then uncured by light being cured within the next hour by said peroxide curing agent.

26. A system or method according to claims 1 and 25, respectively, wherein the resin is at least one resin chosen from the group consisting of ethoxylated bisphenol-A-dimethacrylate, bis-GMA, an adduct of 2,2'-propane bis [3-(4-phenoxy)-1,2-dihydroxy propane-1-methacrylate] and mono- or di- isocyanate, and a methacrylate monomer having 2 to 4 polymerizable double bonds with 0.3 to 2.0% by weight of that monomer of a substituted thiourea reducing agent having the formula

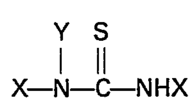

where X is hydrogen or Y and Y is $C_1$ to $C_8$ alkyl; $C_5$ or $C_6$ cycloalkyl; -chloro, hydroxy- or mercapto-substituted $C_1$ to $C_8$ allyl; $C_3$ to $C_4$ alkenyl, $C_6$ to $C_8$ aryl; chloro-, hydroxy-, methoxy-, or sulfonyl substituted phenyl; $C_2$ to $C_8$ acyl; chloro- or methoxy- substituted $C_2$ to $C_8$ acyl; $C_7$ to $C_8$ aralkyl; or chloro- or methoxy-substituted $C_7$ to $C_8$ aralkyl.

27. A method for preparing a two-part storable filled-resin composition useful for porcelain repair and as a dental composite comprising:

coating, in order to prepare the second part, a powdered dental filler with at least one accelerator-free peroxide curing agent for said resin,

storing said filler;

mixing in order to prepare the first part, an uncured methacrylate functional resin usable in dental composites with photoinitiator for said resin,

said photoinitiator including both (1) 2-isopropylthioxanthone or benzil dimethyl acetal and (2) either ethyl 4-dimethylaminobenzoate or ethyl 2-dimethylaminobenzoate,

storing the resin-photoinitiator mixture in an opaque container;

said two-part system being either

(a) a paste-paste system,

(b) a paste-powder system,

(c) a gel-powder system or

(d) a liquid-powder system,

said photoinitiator, peroxide curing agent and first and second parts in said systems being present in an amount of:

| System | Photoinitiator | Peroxide curing agent | Part 1:Part 2 |
|---|---|---|---|
| (a) | 0.06—12.8 wt% | 0.30—0.75 wt% | 1:1 |
| (b) | 0.06—12.8 wt% | 0.30—10 wt% | 20:1—10:1 |
| (c) | 0.09—14.40 wt% | 0.15—0.76 wt% | 20:1—1:3 |
| (d) | 0.18—16 wt% | 0.15—0.76 wt% | 1:2—1:3.5 |

whereby the percentages are based on the components of that part of the system containing the respective compound.

28. Method of using the composition of claim 27 characterized in that at the time of use, said resin-photoinitiator mixture is mixed with said peroxide-coated filler under ordinary indoor illumination, the resulting mixture is applied within a few minutes thereafter and then exposed to high-intensity visible light for ten to thirty seconds.

29. The method of claim 27, wherein said step of mixing said resin and photoinitiator consists of mixing, by weight,

| | |
|---|---|
| 2,2′-propane bis(3-(4-phenoxy)-1,2-dihydroxy propane-1-methacrylate) | at 54.6% and |
| Triethylene glycoldimethacrylate | at 36.4% with |
| 2-Isopropyl thioxanthone | at 0.02% and |
| Ethyl 4-dimethylaminobenzoate | at 8.98%, |

said step of coating consists essentially of coating a mixture of

| | |
|---|---|
| Lithium aluminium silicate | at 69.79% and |
| Barium glass | at 29.91% with |
| Benzoyl peroxide | at 0.30% all by weight, and |

said step of mixing the resin-photoinitiator mixture with said peroxide-coated filler comprises mixing at a weight ratio of three parts coated filler to one part resin-photoinitiator liquid.

30. The method of claim 27, wherein said step of mixing said resin and photoinitiator consists of mixing, by weight,

| | |
|---|---|
| Ethoxylated bisphenol-A-dimethacrylate | at 72.8% and |
| Triethylene glycol dimethacrylate | at 18.2% with |
| 2-Isopropylthioxanthone | at 0.02% and |
| Ethyl 4-dimethylaminobenzoate | at 8.98%, |

said step of coating consists of coating a mixture of

| | |
|---|---|
| Lithium aluminium silicate | at 69.79% and |
| Barium glass | at 29.91% with |
| Benzoyl peroxide | at 0.30%, all by weight, and |

said step of mixing said resin-photoinitiator mixture with said filler comprises mixing them together at the time of use at a weight ratio of about three parts coated filler to one part of resin-photoinitiator mixture.

31. A system or method according to claims (1, 8, 9, 15, 16, 19, 20, 24) and claims (29, 31) respectively, wherein the peroxide curing agent is benzoyl peroxide.

32. The method according to claim 25 and involving a liquid-powder system wherein the step of mixing the uncured resin comprises mixing ethoxylated bisphenol-A-dimethacrylate in an amount of 91.50% by weight with 2-isopropylthioxanthone in an amount of 0.17% by weight and with ethyl 4-dimethylaminobenzoate in an amount of 8.33% by weight, and

the step of coating comprises coating a mixture of barium glass in an amount of 29.82% by weight, lithium aluminum silicate in an amount of 67.30% by weight, flint silica in an amount of 1.22% by weight, $TiO_2$ in an amount of 0.02% by weight, with benzoyl peroxide in an amount of 0.15% by weight, and with gamma-methacryloxypropyltrimethoxysilane in an amount of 1.47% by weight, and with glacial acetic acid in an amount of 0.02%, by weight,

and said step of mixing the said powder and liquid components comprises mixing them together just before use in a weight ratio of from 2:1 to 3.5:1 powder to liquid.

33. The method according to claims 27 and 28 and involving a liquid-powder system wherein the step of mixing the resin comprises mixing, in amounts by weight, ethoxylated bisphenol-A-dimethacrylate at 91.50% with 2-isopropylthioxanthone at 0.17% and with ethyl 4-dimethylaminobenzoate at 8.33%, and

said coating step comprises coating a mixture, by weight of barium glass at 26.66%, lithium aluminium silicate at 62.24%, and flint silica at 9.01%, with benzoyl peroxide at 0.60%, gamma-methacryloxypropyltrimethoxysilane at 1.47% and glacial acetic acid at 0.02%,

said step of mixing the liquid and powder being done at a weight ratio of 2 to 3.5:1, powder:liquid.

34. A method for preparing a two-part storable filled-resin composition useful for porcelain repair and as a dental composite, comprising:

mixing as a first part an uncured methacrylate functional resin usable in dental composites with photoinitiator for said resin in an amount of 0.09 to 14.40% by wt, said photoinitiator including both (1) 2-isopropylthioxanthone or benzil dimethyl acetal and (2) either ethyl 4-dimethylaminobenzoate or ethyl 2-dimethylaminobenzoate, and with sufficient powdered dental filler to form a gel,

storing the gel in an opaque container,

coating as a second part a powdered dental filler with a resin-binding silane and at least one acclerator-free peroxide curing agent for said resin in an effective amount of 0.15—0.76% by wt, and

storing said filler as a dry powder.

35. Method of using the composition of claim 34 comprising at time of use, mixing said gel with said powder under ordinary indoor illumination at a weight ratio between 20:1 and 1:3, gel to powder to make a paste, and applying said paste within a few minutes thereafter.

36. The method of claim 34 wherein said step mixing to form a gel comprises mixing the following ingredients with following percentages by weight:

| | |
|---|---|
| Resin | 50 to 90 |
| Filler | 49.82 to 10 |
| γ-Methacryloxypropyltrimethoxysilane | 1.23 to 0.06 |
| Said photoinitiator | 0.09 to 14.40, and |

said step of coating the filler comprises coating the filler powder at 99.75 to 97.74% by weight, with gamma-methacryloxypropyltrimethoxysilane at 0.10 to 1.50% by weight, and with peroxide curing agent at 0.15 to 0.76% by weight.

37. The method of claim 36 wherein said mixing step includes the following ingredients at the following percentages by weight:

| | |
|---|---|
| Bis-GMA | 0 to 72 |
| Ethoxylated bisphenol-A-dimethacrylate | 0 to 90 |
| Ethylene glycol dimethacrylate | 0 to 54 |
| Diethylene glycol dimethacrylate | 0 to 54 |
| Triethylene glycol dimethacrylate | 0 to 54 |
| Polyethylene glycol dimethacrylate | 0 to 54 |
| Barium glass | 0 to 14.95 |
| Lithium aluminium silicate | 0 to 49.82 |
| Flint silica | 0 to 4.98 |
| Borosilicate glass | 0 to 49.82 |
| Fumed synthetic silica | 0 to 49.82 |
| Strontium glass | 0 to 49.82 |
| Quartz | 0 to 49.82 |
| Titanium dioxide | 0 to 0.15 |
| Tinting agents (e.g., iron oxides) | 0 to 5 |
| Gamma-methacryloxypropyltrimethoxysilane | 0 to 0.75 |
| Said photoinitiator | 0.09 to 14.40 and, |

said step of coating employs some of the following ingredients at the following parts by weight:

| | |
|---|---|
| Barium glass | 0 to 30 |
| Lithium aluminium silicate | 0 to 99.75 |
| Flint silica | 0 to 10 |
| Borosilicate glass | 0 to 99.75 |
| Fumed synthetic silica | 0 to 99.75 |
| Quartz | 0 to 99.75 |
| Strontium glass | 0 to 99.75 |
| Titanium dioxide | 0 to 1.50 |
| Tinting agents (e.g., iron oxides) | 0 to 5 |
| Gamma-methacryloxypropyltrimethoxysilane | 0 to 1.50 |
| Peroxide curing agent | 0.15 to 0.76. |

38. The method of claim 34, wherein the step of mixing to form the gel consists in percents, by weight, of mixing,

| | |
|---|---|
| Ethoxylated bisphenol-A-dimethacrylate | 76.40% |
| 2-Isopropylthioxanthone | 0.06% |
| Ethyl 4-dimethylaminobenzoate | 6.90% |
| Fumed silica | 8.30% |
| Barium glass | 2.43% |
| Lithium aluminium silicate | 5.74% |
| Gamma-methacryloxypropyltrimethoxysilane | 0.08% |
| Glacial acetic acid | 0.04% |

said step of coating the filler consists in percents by weight, of:

coating a mixture of:

| | |
|---|---|
| Barium glass | 29.82%, |
| Lithium aluminium silicate | 67.30%, |
| Flint silica | 1.22%, and |
| $TiO_2$ | 0.02% with |
| Benzoyl peroxide | 0.15%, |
| Gamma-methacryloxypropyltrimethoxysilane | 0.47%, and |
| Glacial acetic acid | 0.02%. |

39. The method of claim 34, wherein the step of mixing to form the gel consists, in percents by weight, of mixing together:

| | |
|---|---|
| Ethoxylated bisphenol-A-dimethacrylate | 76.40% |
| 2-Isopropylthioxanthone | 0.06% |
| Ethyl 4-dimethylaminobenzoate | 6.90% |
| Fumed silica | 8.30% |
| Barium glass | 2.43% |
| Lithium aluminium silicate | 5.74% |
| Gamma-methacryloxypropyltrimethoxysilane | 0.08% |
| Glacial acetic acid | 0.04% |

and said step of coating consists of coating, at the indicated percentages by weight, a mixture of:

| | |
|---|---|
| Barium glass | 26.66%, |
| Lithium aluminium silicate | 62.24%, and |
| Flint silica | 9.01%, with |
| Benzoyl peroxide | 0.60%, |
| Gamma-methacryloxypropyltrimethoxysilane | 0.47%, and |
| Glacial acetic acid | 0.02%. |

40. The method of claim 34 wherein the step of mixing to form the gel consists of mixing, in percents by weight:

| | |
|---|---|
| Ethoxylated bisphenol-A-dimethacrylate | 76.34% |
| 2-Isopropylthioxanthone | 0.14% |
| Ethyl 4-dimethylaminobenzoate | 6.90% |
| Fumed silica | 16.62%, and |

said step of coating the filler consists of coating, in the indicated percentages by weight, a mixture of:

| | |
|---|---|
| Barium glass | 29.82% |
| Lithium aluminium silicate | 67.30% |
| Flint silica | 1.22%, and |
| $TiO_2$ | 0.02%, with |
| Benzoyl peroxide | 0.15%, |
| Gamma-methacryloxypropyltrimethoxysilane | 1.47%, and |
| Glacial acetic acid | 0.02%. |

41. A method for preparing a storable filled-resin composition useful for porcelain repair and as a dental composite and involving a paste-paste system, said method comprising:

mixing a methacrylate functional resin usable in dental composites, with photoinitiator for said resin in an amount of 0.06—12.8% by weight, said photoinitiator including both (1) 2-isopropylthioxanthone or benzil dimethyl acetal and (2) either ethyl 4-dimethylaminobenzoate or ethyl 2-dimethylaminobenzoate, and with a sufficient amount of powdered dental filler coated with a resin-binding silane to make a first paste,

storing said first paste in an opaque container,

coating a powdered dental filler with a resin-binding silane and at least one accelerator-free peroxide curing agent for said resin in an effective amount of 0.30—0.75% by weight,

mixing said coated filler with some methacrylate functional resin to form a second paste,

storing said second paste.

42. Method of using the composition prepared according to claim 41 comprising at time of use, mixing said first and second pastes in substantially equal amounts by weight, under ordinary indoor illumination, to form a third paste, and applying said third paste within a few minutes thereafter.

43. The method of claim 41 wherein said step of mixing to form the second paste comprises mixing the following ingredients in the following percentages by weight:

| | |
|---|---|
| Resin | 18 to 40% |
| Filler | 82 to 60% |
| Peroxide curing agent | 0.75 to 0.30% |
| γ-Methacryloxypropyltrimethoxysilane | 1.23 to 0.06% |
| Butylated hydroxytoluene | 0.01 to 0.10% and |

the step of making the first paste comprises employing the following ingredients in the following amounts:

| | |
|---|---|
| Resin | 18 to 40% |
| Filler | 80 to 60% |
| Said photoinitiators | 0.06 to 12.8% |
| Gamma-methacryloxypropyltrimethoxysilane | 1.23 to 0.06%. |

44. The method of claim 43 wherein the step of forming the second paste employs the following ingredients at the following percentages by weight:

| | |
|---|---|
| Bis-GMA | 0 to 32% |
| Ethoxylated bisphenol-A-dimethacrylate | 0 to 40% |
| Ethylene glycol dimethacrylate | 0 to 24% |
| Diethylene glycol dimethacrylate | 0 to 24% |
| Triethylene glycol dimethacrylate | 0 to 24% |
| Polyethylene glycol dimethacrylate | 0 to 24% |
| Barium glass | 0 to 25% |
| Lithium aluminium silicate | 0 to 82% |
| Flint silica | 0 to 8.5% |
| Borosilicate glass | 0 to 82% |
| Fumed synthetic silica | 0 to 52% |
| Quartz | 0 to 82% |
| Strontium glass | 0 to 82% |
| Titanium dioxide | 0 to 0.13% |
| Tinting agents (e.g., iron oxides) | 0 to 4% |
| Gamma-methacryloxypropyltrimethoxysilane | 0 to 1.25% |
| Peroxide curing agent | 0.30 to 0.75% and |

the step of forming the first paste employs the following ingredients at the following percentages by weight:

| | |
|---|---|
| Bis-GMA | 0 to 32 |
| Ethoxylated bisphenol-A-dimethacrylate | 0 to 40 |
| Ethylene glycol dimethacrylate | 0 to 24 |
| Diethylene glycol dimethacrylate | 0 to 24 |
| Triethylene glycol dimethacrylate | 0 to 24 |
| Polyethylene glycol dimethacrylate | 0 to 24 |
| Barium glass | 0 to 25 |
| Lithium aluminium silicate | 0 to 82 |
| Flint silica | 0 to 8.5 |
| Borosilicate glass | 0 to 82 |
| Fumed synthetic silica | 0 to 52 |
| Quartz | 0 to 82 |
| Strontium glass | 0 to 82 |
| Titanium dioxide | 0 to 0.13 |
| Tinting agents (e.g., iron oxides) | 0 to 4 |
| Gamma-methacryloxypropyltrimethoxysilane | 0 to 1.25 |
| Said photoinitiators | 0.06 to 12.8. |

45. The method of claim 41 wherein the step of forming the second paste consists of mixing the following ingredients in the indicated percentages by weight:

| | |
|---|---|
| Ethoxylated bisphenol-A-dimethacrylate | 17.36% |
| Benzoyl peroxide | 0.60% |
| Butylated hydroxytoluene | 0.04% |
| Strontium glass | 79.8784% |
| Fumed silica | 0.92% |
| Gamma-methacryloxypropyltrimethoxysilane | 0.20% |
| Glacial acetic acid | 0.0016%, |

and the step of forming the first paste consists of mixing the following ingredients to the indicated percentages by weight:

| | |
|---|---|
| Ethoxylated bisphenol-A-dimethacrylate | 14.94% |
| 2-Isopropylthioxanthone | 0.06% |
| Ethyl 4-dimethylaminobenzoate | 3.00% |
| Strontium glass | 79.8784% |
| Fumed silica | 0.92% |
| Gamma-methacryloxypropyltrimethoxysilane | 1.20% |
| Glacial acetic acid | 0.0016%. |

46. A method for preparing a storable filled-resin composition useful for porcelain repair and as a dental composite and involving a paste-powder system, said method comprising:

mixing a methacrylate functional resin usable in dental composites, with photoinitiator for said resin in an amount of 0.06—12.8% by weight, said photoinitiator including both (1) 2-isopropylthioxanthone or a benzil dimethyl acetal and (2) either ethyl 4-dimethylaminobenzoate or ethyl 2-dimethylaminobenzoate, and with a sufficient amount of powdered dental filler coated with a resin-binding silane to make a paste,

storing said paste in an opaque container,

coating a powdered dental filler with a resin-bonding silane and at least one accelerator-free peroxide curing agent for said resin in an effective amount of 0.05—10% by weight, to make a powder,

storing said powder.

47. Method of using the composition prepared according to claim 46 comprising:

at time of use, adding said powder to said paste in an amount by weight of 5% to 10% of said paste, under ordinary indoor illumination, to make a thickened paste, and

applying said thickened paste within a few minutes thereafter.

48. The method of claim 46 wherein said step of mixing said resin with photoinitiator and filler comprises mixing the following ingredients, by weight:

| | |
|---|---|
| Resin | 18 to 40% |
| Filler | 80 to 60% |
| Said photoinitiators | 0.06 to 12.8% |
| γ-Methacryloxypropyltrimethoxysilane | 1.23 to 0.06%, and |

said step of coating the dental filler comprises coating the dental filler material at 99.75 to 97.74% by weight with gamma-methacryloxypropyltrimethoxysilane at 0.10 to 1.50% by weight, and with peroxide curing agent at 0.30 to 10% by weight.

49. The method of claim 48 wherein said step of mixing to make said first paste employs the following ingredients in the following percentages by weight:

| | |
|---|---|
| Bis-GMA | 0 to 32% |
| Ethoxylated bisphenol-A-dimethacrylate | 0 to 40% |
| Ethylene glycol dimethacrylate | 0 to 24% |
| Diethylene glycol dimethacrylate | 0 to 24% |
| Triethylene glycol dimethacrylate | 0 to 24% |
| Polyethylene glycol dimethacrylate | 0 to 24% |
| Barium glass | 0 to 25% |
| Lithium aluminium silicate | 0 to 82% |
| Flint silica | 0 to 8.5% |
| Borosilicate glass | 0 to 82% |
| Fumed synthetic silica | 0 to 52% |
| Strontium glass | 0 to 82% |
| Quartz | 0 to 82% |
| Titanium dioxide | 0 to 0.13% |
| Tinting agents (e.g., iron oxides) | 0 to 4% |
| Gamma-methacryloxypropyltrimethoxysilane | 0 to 1.25% |
| Said photoinitiators | 0.06 to 12.8%, and |

said step of making the powder employs the following ingredients in the following percentages by weight:

| | |
|---|---|
| Barium glass | 0 to 30% |
| Lithium aluminium silicate | 0 to 99.75% |
| Flint silica | 0 to 10% |
| Borosilicate glass | 0 to 99.75% |
| Fumed synthetic silica | 0 to 99.75% |
| Strontium glass | 0 to 99.75% |
| Titanium dioxide | 0 to 0.15% |
| Tinting agents (e.g., iron oxides) | 0 to 5% |
| Gamma-methacryloxypropyltrimethoxysilane | 0 to 1.50% |
| Peroxide curing agent | 0.30 to 10%. |

50. The method of claim 46, wherein the step of making said first paste consists in mixing the following ingredients in the following percentages by weight:

| | |
|---|---|
| Ethoxylated bisphenol-A-dimethacrylate | 16.07% |
| 2-Isopropylthioxanthone | 0.03% |
| Ethyl 4-dimethylaminobenzoate | 1.49% |
| Fumed silica | 0.90% |
| Lithium aluminium silicate | 57.08% |
| Barium glass | 24.45%, and |

said step of making said powder comprising mixing the following ingredients in the following percentages by weight:

| | |
|---|---|
| Fumed silica (pre-coated with resin-binding silane) | 95.00% |
| Benzoyl peroxide | 5.00%. |

**Patentansprüche**

1. Zweikomponenten-System zur Herstellung einer Zusammensetzung mit füllstoffhaltigem Harz mit Methacrylatfunktion durch Mischen dieser beiden Komponenten umfassend:

erste Komponente: ein Harz mit Methacrylatfunktion, vom Dentaltyp, mit einem dafür geeigneten Photoinitiator;

zweite Komponente: ein pulverförmiger Füllstoff vom Dentaltyp mit einem beschleunigerfreien Peroxid-Vernetzungsmittel für das Harz;

wobei der Photoinitiator sowohl (1) 2-Isopropylthioxanthon oder Benzildimethylacetal als auch (2) entweder Äthyl-4-dimethylaminobenzoat oder Äthyl-2-dimethylaminobenzoat enthält, und

wobei das Zweikomponenten-System ein

(a) Paste-Paste-System oder ein

(b) Paste-Pulver-System oder ein

(c) Gel-Pulver-System oder ein

(d) Flüssigkeit-Pulver-System ist, und

der Photoinitiator, das Peroxid-Vernetzungsmittel und die erste und die zweite Komponente dieser Systeme in folgenden Mengen vorliegen:

| System | Photoinitiator | Peroxid-Vernetzungsmittel | Komponente 1:Komponente 2 |
|---|---|---|---|
| (a) | 0,06—12,8 Gew.-% | 0,30—0,75 Gew.-% | 1:1 |
| (b) | 0,06—12,8 Gew.-% | 0,30—10 Gew.-% | 20:1—10:1 |
| (c) | 0,09—14,40 Gew.-% | 0,15—0,76 Gew.-% | 20:1—1:3 |
| (d) | 0,18—16 Gew.-% | 0,15—0,76 Gew.-% | 1:2—1:3,5 |

wobei sich die Prozentsätze auf die Bestandteile jener Komponente des Systems beziehen, die den entsprechenden Bestandteil enthält.

2. System nach Anspruch 1, wobei die erste Komponente in Form eines Pulvers und die zweite Komponente in Form eines Gels, das auch den Füllstoff enthält, vorliegen.

3. System nach Anspruch 1, wobei sowohl die erste als auch die zweite Komponente in Form von Pasten vorliegen, die beide den Füllstoff enthalten.

4. System nach Anspruch 3, wobei die erste und die zweite Komponente so zusammengesetzt sind, daß sie bei der Verwendung eine Mischung gleicher Gewichtsteile ermöglichen.

5. Flüssigkeit-Pulver-System nach Anspruch 1, bestehend aus, in Gew-%

der Pulver-Komponente, bestehend aus, in Gew.-%:

| | |
|---|---|
| Bariumglas | 0% bis 30.00% |
| Lithiumaluminiumsilikat | 0% bis 99,75% |
| Flintquartz | 0% bis 10,00% |
| Borosilikatglas | 0% bis 99,75% |
| pyrogene synthetische Kieselsäure | 0% bis 99,75% |
| Quarz | 0% bis 99,75% |
| Titandioxid | 0% bis 0,15% |
| Färbemittel (z.B. Eisenoxide) | 0% bis 5,00% |
| Gamma-Methacryloxypropyltrimethoxysilan | 0% bis 1,50% |
| Peroxid-Vernetzungsmittel | 0,15% bis 0,76% |

und einer flüssigen Komponente, bestehend aus, in Gew.-%:

| | |
|---|---|
| Bis-GMA | 0% bis 80,00% |
| äthoxyliertes Bisphenol-A-Dimethacrylat | 0% bis 99,00% |
| Äthylenglykoldimethacrylat | 0% bis 60,00% |
| Diäthylenglykoldimethacrylat | 0% bis 60,00% |
| Triäthylenglykoldimethacrylat | 0% bis 60,00% |
| Polyäthylenglykoldimethacrylat | 0% bis 60,00% |
| Photoinitiator für obige Harze | 0,18% bis 16,00% |

wobei die Pulver-Komponente und die flüssige Komponente bei der Verwendung in einem Gewichtsverhältnis von 1:1 bis 3,5:1 Pulver:Flüssigkeit vermischt werden.

6. Flüssigkeit-Pulver-System nach Anspruch 1, bestehend aus der Pulver-Komponente, bestehend aus, in Gew.-%:

| | |
|---|---|
| Dental-Füllstoffmaterial | 99,75% bis 97,74% |
| Gamma-Methacryloxypropyltrimethoxysilan | 0,10% bis 1,50% |
| Peroxid-Vernetzungsmittel | 0,15 bis 0,76% |

und der flüssigen Komponente bestehend aus, in Gew.-%:

| | |
|---|---|
| dem Harz mit Methacrylatfunktion, geeignet zur Verwendung in Dental-Verbundwerkstoffen | 99,82% bis 84,00% |
| dem Photoinitiator für das Harz | 0,18% bis 16,00% |

wobei das Pulver und das Harz in einem Gewichtsverhältnis zwischen 1:1 und 3,5:1 Pulver:Flüssigkeit vermischt werden.

7. Flüssigkeit-Pulver-System nach Anspruch 1, wobei die Pulver-Komponente besteht aus:

| | |
|---|---|
| Bariumglas | 29,82% |
| Lithiumaluminiumsilikat | 67,30% |
| Flintquarz | 1,22% |
| $TiO_2$ | 0,02% |
| Benzoylperoxid | 0,15% |
| Gamma-Methacryloxypropyltrimethoxysilan | 1,47% |
| Eisessig | 0,02% |

und die flüssige Komponente besteht aus:

| | |
|---|---|
| äthoxyliertes Bisphenol-A-Dimethacrylat | 91,50% |
| 2-Isopropylthioxanthon | 0,17% |
| Äthyl-4-dimethylaminobenzoat | 8,33% |

wobei die pulverförmige und die flüssige Komponente unmittelbar vor Gebrauch in einem Gewichtsverhältnis von 2:1 bis 3,5:1 Pulver:Flüssigkeit vermischt werden.

8. Flüssigkeit-Pulver-System nach Anspruch 1, wobei das Pulver besteht aus:

| | |
|---|---|
| Bariumglas | 26,66% |
| Lithiumaluminiumsilikat | 62,24% |
| Flintquarz | 9,01% |
| Benzoylperoxid | 0,60% |
| Gamma-Methacryloxypropyltrimethoxysilan | 1.47% |
| Eisessig | 0,02% |

und die Flüssigkeit besteht aus:

| | |
|---|---|
| äthoxyliertes Bisphenol-A-Dimethacrylat | 91,50% |
| 2-Isopropylthioxanthon | 0,17% |
| Äthyl-4-dimethylaminobenzoat | 8,33% |

und die Flüssigkeit und das Pulver in einem Gewichtsverhältnis von 2 bis 3,5:1 Pulver:Flüssigkeit vermischt werden.

9. Flüssigkeit-Pulver-System nach Anspruch 1, wobei die flüssige Komponente besteht aus:

| | |
|---|---|
| 2,2'-Propan-bis-[3-(4-phenoxy)-1,2-hydroxy-propan-1-methacrylat] | 54,6% |
| Triäthylenglykoldimethacrylat | 36,4% |
| 2-Isopropylthioxanthon | 0,02% |
| Äthyl-4-dimethylaminobenzoat | 8,98% |

und die Pulver-Komponente besteht aus:

| | |
|---|---|
| Lithiumaluminiumsilikat | 69,79% |
| Bariumglas | 29,21% |
| Benzoylperoxid | 0,30% |

und das Pulver und die Flüssigkeit zum Gebrauch in einem Gewichtsverhältnis von drei Teilen Pulver zu einen Teil Flüssigkeit vermischt werden.

10. Flüssigkeit-Pulver-System nach Anspruch 1, wobei die flüssige Komponente besteht aus:

| | |
|---|---|
| äthoxyliertes Bisphenol-A-Dimethacrylat | 72,8% |
| Triäthylenglykoldimethacrylat | 18,2% |
| 2-Isopropylthioxanthon | 0,02% |
| Äthyl-4-dimethylaminobenzoat | 8,98% |

und einer Pulver-Komponente bestehend aus:

| | |
|---|---|
| Lithiumaluminsilikat | 69,79% |
| Bariumglas | 29,91% |
| Benzoylperoxid | 0,30% |

wobei das Pulver und die Flüssigkeit bei der Verwendung in einem Gewichtsverhältnis von etwa 3 Teilen Pulver zu einem Teil Flüssigkeit vermischt werden.

11. Flüssigkeit-Pulver-System nach Anspruch 1, wobei die Pulver-Komponente besteht aus:

| | Gewichtsteile |
|---|---|
| Strontiumglas | 100 |
| Benzoylperoxid | 0,3 |
| Gamma-Methacryloxypropyltrimethoxysilan | 1,5 |

und die flüssige Komponente besteht aus:

| | |
|---|---|
| äthoxyliertes Bisphenol-A-Dimethacrylat | 91,50% |
| 2-Isopropylthioxanthon | 0,17% |
| Äthyl-4-dimethylaminobenzoat | 8,33% |

und die pulverförmige und die flüssige Komponente unmittelbar vor der Verwendung in einem Gewichtsverhältnis von 3:1 Pulver:Flüssigkeit vermischt werden.

12. Paste-Paste-System nach Anspruch 1, bestehend aus, in Gew-%:

| | |
|---|---|
| Paste A: | |
| Harz mit Methacrylatfunktion, geeignet zur Verwendung in Dental-Verbundwerkstoffen | 18 bis 40 |
| Dental-Füllstoffmaterial | 82 bis 60 |
| Peroxid-Vernetzungsmittel | 0,75 bis 0,30 |
| Gamma-methacryloxypropyltrimethoxysilan | 1,23 bis 0,06 |
| butyliertes Hydroxytoluol | 0,01 bis 0,10 |
| Paste B: | |
| Harz mit Methacrylatfunktion, geeignet zur Verwendung in Dental-Verbundwerkstoffen | 18 bis 40 |
| Dental-Füllstoffmaterial | 80 bis 60 |
| obige Photoinitiatoren | 0,06 bis 12,8 |
| Gamma-Methacryloxypropyltrimethoxysilan | 1,23 bis 0,06 |

wobei die Pasten A und B bei der Verwendung in im wesentlichen gleichen Gewichtsteilen vermischt werden.

13. System nach Anspruch 12, wobei die Paste A besteht aus, in Gew.-%:

| | |
|---|---|
| Bis-GMA | 0 bis 32 |
| äthoxyliertes Bisphenol-A-Dimethacrylat | 0 bis 40 |
| Äthylenglykoldimethacrylat | 0 bis 24 |
| Diäthylenglykoldimethacrylat | 0 bis 24 |
| Triäthylenglykoldimethacrylat | 0 bis 24 |
| Polyäthylenglykoldimethacrylat | 0 bis 24 |
| Bariumglas | 0 bis 25 |
| Lithiumaluminiumsilikat | 0 bis 82 |
| Flintquarz | 0 bis 8,5 |
| Borosiliaktglas | 0 bis 82 |
| pyrogene synthetische Kieselsäure | 0 bis 52 |
| Quarz | 0 bis 82 |
| Strontiumglas | 0 bis 82 |
| Titandioxid | 0 bis 0,13 |
| Färbemittel (z.B. Eisenoxide) | 0 bis 4 |
| Gamma-Methacryloxypropyltrimethoxysilan | 0 bis 1,25 |
| Peroxid-Vernetzungsmittel | 0,30 bis 0,75 |

und Paste B besteht aus, in Gew.-%:

| | |
|---|---|
| Bis-GMA | 0 bis 32 |
| äthoxyliertes Bisphenol-A-Dimethacrylat | 0 bis 40 |
| Äthylenglykoldimethacrylat | 0 bis 24 |
| Diäthylenglykoldimethacrylat | 0 bis 24 |
| Triäthylenglykoldimethacrylat | 0 bis 24 |
| Polyäthylenglykoldimethacrylat | 0 bis 24 |
| Bariumglas | 0 bis 25 |
| Lithiumaluminiumsilikat | 0 bis 82 |
| Flintquarz | 0 bis 8,5 |
| Borosilikatglas | 0 bis 82 |
| pyrogene synthetische Kieselsäure | 0 bis 52 |
| Quarz | 0 bis 82 |
| Strontiumglas | 0 bis 82 |
| Titandioxid | 0 bis 0,13 |
| Färbemittel (z.B. Eisenoxide) | 0 bis 4 |
| Gamma-Methacryloxypropyltrimethoxysilan | 0 bis 1,25 |
| obige Photoinitiatoren | 0,10 bis 12,8. |

14. System nach Anspruch 12, wobei die erste Paste besteht aus:

| | |
|---|---|
| äthoxyliertes Bisphenol-A-Dimethacrylat | 17,36% |
| Benzoylperoxid | 0,60% |
| butyliertes Hydroxytoluol | 0,04% |
| Strontiumglas | 79,8784% |
| pyrogene Kieselsäure | 0,92% |
| Gamma-Methacryloxypropyltrimethoxysilan | 0,20% |
| Eisessig | 0,0016% |

und die zweite Paste besteht aus:

| | |
|---|---|
| äthoxyliertes Bisphenol-A-Dimethacrylat | 14,94% |
| 2-Isopropylthioxanthon | 0,06% |
| Äthyl-4-dimethylaminobenzoat | 3,00% |
| Strontiumglas | 79,8784% |
| pyrogene Kieselsäure | 0,92% |
| Gamma-Methacryloxypropyltrimethoxysilan | 1,20% |
| Eisessig | 0,0016%. |

15. Gel-Pulver-System nach Anspruch 1, bestehend aus, in Gewichtsteilen: einem Gel bestehend aus:

| | |
|---|---|
| Harz mit Methacrylatfunktion, geeignet zur Verwendung in Dental-Verbundwerkstoffen | 50 bis 90 |
| Dental-Füllstoffmaterial | 49,82 bis 10 |
| Gamma-Methacryloxypropyltrimethoxysilan | 1,23 bis 0,06 |
| obiger Photoinitiator | 0,09 bis 14,40 |

und einem Pulver bestehend aus:

| | |
|---|---|
| Dental-Füllstoffmaterial | 99,75 bis 97,74 |
| Gamma-Methacryloxypropyltrimethoxysilan | 0,10 bis 1,50 |
| Peroxid-Vernetzungsmittel | 0,15 bis 0,76 |

wobei das Gel und das Pulver bei der Verwendung in einem Gewichtsverhältnis von Gel:Pulver von 20:1 bis 1:3 vermischt werden.

16. System nach Anspruch 15, wobei die Bestandteile des Gels und des Pulvers in Gew.-% wie folgt zusammengesetzt sind:

Gel:

| | |
|---|---|
| Bis-GMA | 0 bis 72% |
| äthoxyliertes Bisphenol-A-Dimethacrylat | 0 bis 90% |
| Äthylenglykoldimethacrylat | 0 bis 54% |
| Diäthylenglykoldimethacrylat | 0 bis 54% |
| Triäthylenglykoldimethacrylat | 0 bis 54% |
| Polyäthylenglykoldimethacrylat | 0 bis 54% |
| Bariumglas | 0 bis 14,95% |
| Lithiumaluminiumsilikat | 0 bis 49,82% |
| Flintquarz | 0 bis 4,98% |
| Borosilikatglas | 0 bis 49,82% |
| pyrogene synthetische Kieselsäure | 0 bis 49,82% |
| Strontiumglas | 0 bis 49,82% |
| Quarz | 0 bis 49,82% |
| Titandioxid | 0 bis 0,15% |
| Färbemittel (z.B. Eisenoxide) | 0 bis 5% |
| Gamma-Methacryloxypropyltrimethoxysilan | 0 bis 0,75% |
| obiger Photoinitiator | 0,09 bis 14,40% |

Pulver:

| | |
|---|---|
| Bariumglas | 0 bis 30% |
| Lithiumaluminiumsilikat | 0 bis 99,75% |
| Flintquarz | 0 bis 10% |
| Borosilikatglas | 0 bis 99,75% |
| pyrogene synthetische Kieselsäure | 0 bis 99,75% |
| Quarz | 0 bis 99,75% |
| Strontiumglas | 0 bis 99,75% |
| Titandioxid | 0 bis 1,50% |
| Färbemittel (z.B. Eisenoxide) | 0 bis 5% |
| Gamma-Methacryloxypropyltrimethoxysilan | 0 bis 1,50% |
| Peroxid-Vernetzungsmittel | 0,15 bis 0,76%. |

17. System nach Anspruch 15, wobei das Gel besteht aus, in Gew.-%:

| | |
|---|---|
| äthoxyliertes Bisphenol-A-Dimethacrylat | 76,40% |
| 2-Isopropylthioxanthon | 0,06% |
| Äthyl-4-dimethylaminobenzoat | 6,90% |
| pyrogene Kieselsäure | 8,30% |
| Bariumglas | 2,43% |
| Lithiumaluminiumsilikat | 5,74% |
| Gamma-Methacryloxypropyltrimethoxysilan | 0,08% |
| Eisessig | 0,04% |

und das Pulver besteht aus:

| | |
|---|---|
| Bariumglas | 29,82% |
| Lithiumaluminiumsilikat | 67,30% |
| Flintquarz | 1,22% |
| $TiO_2$ | 0,02% |
| Benzoylperoxid | 0,15% |
| Gamma-Methacryloxypropyltrimethoxysilan | 0,47% |
| Eisessig | 0,02% |

und das Gel und das Pulver bei der Verwendung in einem Gewichtsverhältnis zwischen 1:20 und 3:1 Pulver:Gel vermischt werden.

18. System nach Anspruch 15, wobei das Gel besteht aus, in Gew-%:

| | |
|---|---|
| äthoxyliertes Bisphenol-A-Dimethacrylat | 76,40% |
| 2-Isopropylthioxanthon | 0,06% |
| Äthyl-4-dimethylaminobenzoat | 6,90% |
| pyrogene Kieselsäure | 8,30% |
| Bariumglas | 2,43% |
| Lithiumaluminiumsilikat | 5,74% |
| Gamma-Methacryloxypropyltrimethoxysilan | 0,08% |
| Eisessig | 0,04% |

und das Pulver besteht aus:

| | |
|---|---|
| Bariumglas | 26,66% |
| Lithiumaluminiumsilikat | 62,24% |
| Flintquarz | 9,01% |
| Benzoylperoxid | 0,60% |
| Gamma-Methacryloxypropyltrimethoxysilan | 1,47% |
| Eisessig | 0,02% |

wobei das Gel und das Pulver bei der Verwendung in einem Gewichtsverhältnis zwischen 1:20 und 3:1 Pulver:Gel vermischt werden.

19. System nach Anspruch 15, wobei das Gel besteht aus, in Gew.-%:

| | |
|---|---|
| äthoxyliertes Bisphenol-A-Dimethacrylat | 76,34% |
| 2-Isopropylthioxanthon | 0,14% |
| Äthyl-4-dimethylaminobenzoat | 6,90% |
| pyrogene Kieselsäure | 16,62% |

und das Pulver besteht aus:

| | |
|---|---|
| Bariumglas | 29,82% |
| Lithiumaluminiumsilikat | 67,30% |
| Flintquarz | 1,22% |
| $TiO_2$ | 0,02% |
| Benzoylperoxid | 0,15% |
| Gamma-Methacryloxypropyltrimethoxysilan | 0,47% |
| Eisessig | 0,02% |

und das Gel und das Pulver bei der Verwendung in einem Gewichtsverhältnis zwischen 1:20 und 3:1 Pulver:Gel vermischt werden.

20. Paste-Pulver-System nach Anspruch 1, bestehend aus, in Gew.-%:
einer Paste, bestehend aus:

| | |
|---|---|
| Harz mit Methacrylatfunktion, geeignet zur Verwendung in Dental-Verbundwerkstoffen | 18 bis 40 |
| Dental-Füllstoffmaterial | 80 bis 60 |
| obige Photoinitiatoren | 0,06 bis 12,8 |
| Gamma-Methacryloxypropyltrimethoxysilan | 1,23 bis 0,06 |

und einem Pulver bestehend aus:

| | |
|---|---|
| Dental-Füllstoffmaterial | 99,75 bis 97,74 |
| Gamma-Methacryloxypropyltrimethoxysilan | 1,10 bis 1,50 |
| Peroxid-Vernetzungsmittel | 0,30 bis 10 |

wobei das Pulver zu der Paste bei der Verwendung in einem Gewichtsverhältnis von 20:1 bis 10:1 Paste:Pulver zugefügt wird.

21. System nach Anspruch 20, wobei die Paste und das Pulver aus folgenden Bestandteilen, in Gew.-%, bestehen:

Paste:

| | |
|---|---|
| Bis-GMA | 0 bis 32 |
| äthoxyliertes Bisphenol-A-Dimethacrylat | 0 bis 40 |
| Äthylenglykoldimethacrylat | 0 bis 24 |
| Diäthylenglykoldimethacrylat | 0 bis 24 |
| Triäthylenglykoldimethacrylat | 0 bis 24 |
| Polyäthylenglykoldimethacrylat | 0 bis 24 |
| Bariumglas | 0 bis 25 |
| Lithiumaluminiumsilikat | 0 bis 82 |
| Flintquarz | 0 bis 8,5 |
| Borosilikatglas | 0 bis 82 |
| pyrogene synthetische Kieselsäure | 0 bis 52 |
| Quarz | 0 bis 82 |
| Strontiumglas | 0 bis 82 |
| Titandioxid | 0 bis 0,13 |
| Färbemittel (z.B. Eisenoxide) | 0 bis 4 |
| Gamma-Methacryloxypropyltrimethoxysilan | 0 bis 1,25 |
| obige Photoinitiatoren | 0,06 bis 12,8 |

Pulver:

| | |
|---|---|
| Bariumglas | 0 bis 30 |
| Lithiumaluminiumsilikat | 0 bis 99,75 |
| Flintquarz | 0 bis 10 |
| Borosilikatglas | 0 bis 99,75 |
| pyrogene synthetische Keiselsäure | 0 bis 99,75 |
| Strontiumglas | 0 bis 99,75 |
| Titandioxid | 0 bis 0,15 |
| Färbemittel (z.B. Eisenoxide) | 0 bis 5 |
| Gamma-Methacryloxypropyltrimethoxysilan | 0 bis 1,50 |
| Peroxid-Vernetzungsmittel | 0,30 bis 10 |

22. System nach Anspruch 20, wobei die Paste-Komponente besteht aus:

| | |
|---|---|
| äthoxyliertes Bisphenol-A-Dimethacrylat | 16,07% |
| 2-Isopropylthioxanthon | 0,03% |
| Äthyl-4-dimethylaminobenzoat | 1,49% |
| pyrogene Kieselsäure | 0,90% |
| Lithiumaluminiumsilikat | 57,08% |
| Bariumglas | 24,45% |

und die Pulver-Komponente besteht aus:

| | |
|---|---|
| pyrogene Kieselsäure (vorbeschichtet mit Silan) | 95,00% |
| Benzoylperoxid | 5,00% |

wobei das Pulver der Paste bei der Verwendung in einem Gewichtsverhältnis von 20:1 bis 10:1 Paste:Pulver zugesetzt wird.

23. System nach Anspruch 20, wobei die Paste-Komponente besteht aus:

| | Gewichtsteile |
|---|---|
| Bis-GMA | 60 |
| Triäthylenglykoldimethacrylat | 40 |
| 2-Isopropylthioxanthon | 0,18 |
| Äthyl-4-dimethylaminobenzoat | 9,10 |
| Lithiumaluminiumsilikat | 355 |
| Bariumglas | 152 |

und die Pulver-Komponente besteht aus:

| | Gewichtsteile |
|---|---|
| Lithiumaluminiumsilikat | 70 |
| Bariumglas | 30 |
| Benzoylperoxid | 0,75 |

und die Paste und das Pulver bei der Verwendung in einem Gewichtsverhältnis von 20:1 Paste:Pulver vermischt werden.

24. System nach Anspruch 20, wobei die Paste-Komponente besteht aus:

| | Gewichtsteile |
|---|---|
| äthoxyliertes Bisphenol-A-Dimethacrylat | 80 |
| Triäthylenglykoldimethacrylat | 20 |
| 2-Isopropylthioxanthon | 0,18 |
| Äthyl-4-dimethylaminobenzoat | 9,10 |
| Lithiumaluminiumsilikat | 355 |
| Bariumglas | 152 |

und die Pulver-Komponente besteht aus:

| | Gewichtsteile |
|---|---|
| Lithiumaluminiumsilikat | 70 |
| Bariumglas | 30 |
| Benzoylperoxid | 0,30 |

wobei die Paste und das Pulver bei der Verwendung in einem Gewichtsverhältnis von 20:1 Paste:Pulver vermischt werden.

25. Verfahren zur Reparatur von Porzellan oder Zähnen mit Hilfe eines Zwei-Komponenten-Systems, wobei das Verfahren besteht aus:

Vermischen unter normalen Innenraumbeleuchtungsverhältnissen von:

— einem in Dental-Verbundwerkstoffen verwendbaren Harz mit Methacrylatfunktion und einem Photoinitiator für dieses Harz, wobei der Photoinitiator sowohl (1) 2-Isopropylthioxanthon oder Benzildimethylacetal als auch (2) entweder Äthyl-4-dimethylaminobenzoat oder Äthyl-2-dimethylaminobenzoat enthält (Komponente 1), und

— einem gepulverten Dental-Füllstoff und mindestens einem beschleunigerfreien Peroxid-Vernetzungsmittel für das Harz (Komponente 2),

wobei das Zwei-Komponenten-System

(a) ein Paste-Paste-System oder

(b) ein Paste-Pulver-System oder

(c) ein Gel-Pulver-System oder

(d) ein Flüssigkeit-Pulver-System ist und

der Photoinitiator, das Peroxid-Vernetzungsmittel und die erste und die zweite Komponente dieser Systeme in folgenden Mengen vorliegen:

| System | Photoinitiator | Peroxid-Vernetzungsmittel | Komponente 1:Komponente 2 |
|---|---|---|---|
| (a) | 0,06—12,8 Gew.-% | 0,30—0,75 Gew.-% | 1:1 |
| (b) | 0,06—12,8 Gew.-% | 0,30—10 Gew.-% | 20:1—10:1 |
| (c) | 0,09—14,40 Gew.-% | 0,15—0,76 Gew.-% | 20:1—1:3 |
| (d) | 0,18—16 Gew.-% | 0,15—0,76 Gew.-% | 1:2—1:3,5 |

wobei sich die Prozentsätze auf die Bestandteile jener Komponente des Systems beziehen, die den entsprechenden Bestandteil enthält;

Einbringen der Mischung innerhalb einiger Minuten nach dem Vermischen und Vernetzung eines wesentlichen, ausgedrückten Anteils der eingebrachten Mischung an Ort und Stelle unter intensiver Beleuchtung, wobei dann von dem Licht noch nicht vernetztes Harz innerhalb der nächsten Stunde durch das Peroxid-Vernetzungsmittel vernetzt wird.

26. System oder Verfahren nach Ansprüchen 1 bzw. 25, wobei das Harz mindestens ein Harz ist ausgewählt aus der Gruppe bestehend aus äthoxyliertem Bisphenol-A-Dimethacrylat, bis-GMA, einem Addukt von 2,2'-Propan-bis-[3-(4-phenoxy)-1,2-hydroxypropan-1-methacrylat] und Mono- oder Diisocyanat und einem Methacrylat-Monomer mit 2 bis 4 polymerisierbaren Doppelbindungen mit 0,3 bis 2,0 Gew.-% dieses Monomeren eines substituierten Thioharnstoff-Reduktionsmittels der Formel

```
     Y  S
     |  ||
X—N—C—NHX
```

wobei X Wasserstoff oder Y, und Y $C_1$ bis $C_8$ Alkyl; $C_5$ oder $C_6$ Cycloalkyl; Chlor-, Hydroxy- oder

Mercapto-substituiertes $C_1$ bis $C_8$ Allyl; $C_3$ bis $C_4$ Alkenyl, $C_6$ bis $C_8$ Aryl; Chlor-, Hydroxy-, Methoxy- oder Sulfonyl-substituiertes Phenyl; $C_2$ bis $C_8$ Acyl; Chlor- oder Methoxy-substituiertes $C_2$ bis $C_8$ Acyl; $C_7$ bis $C_8$ Aralkyl; oder Chlor- oder Methoxy-substituiertes $C_7$ bis $C_8$ Aralkyl bedeuten.

27. Verfahren zur Herstellung einer lagerfähigen Zwei-Komponenten-Zusammensetzung mit füllstoffhaltigem Harz geeignet zur Reparatur von Porzellan und als Dental-Verbundwerkstoff, bestehend aus:

Beschichten, zu Herstellung der ersten Komponente, eines gepulverten Dental-Füllstoffs mit mindestens einem beschleunigerfreien Peroxid-Vernetzungsmittel für das Harz,

Lagern des Füllstoffs;

Mischung, zur Herstellung der zweiten Komponente, eines unvernetzten Harzes mit Methacrylatfunktion, das in Dental-Verbundswerkstoffen verwendbar ist, mit einem Photoinitiator für das Harz,

wobei der Photoinitiator sowohl (1) 2-Isopropylthioxanthon oder Benzildimethylacetal als auch (2) entweder Äthyl-4-dimethylaminobenzoat oder Äthyl-2-dimethylaminobenzoat enthält,

Aufbewahren der Harz-Photoinitiator-Mischung in einem undurchsichtigen Behälter;

wobei das Zwei-Komponenten-System

(a) ein Paste-Paste-System, oder

(b) ein Paste-Pulver-System oder

(c) ein Gel-Pulver-System oder

(d) ein Flüssigkeit-Pulver-System ist und

der Photoinitiator, das Peroxid-Vernetzungsmittel und die erste und die zweite Komponente dieser Systeme in folgenden Mengen vorliegen:

| System | Photoinitiator | Peroxid-Vernetzungsmittel | Komponente 1:Komponente 2 |
|---|---|---|---|
| (a) | 0,06—12,8 Gew.-% | 0,30—0,75 Gew.-% | 1:1 |
| (b) | 0,06—12,8 Gew.-% | 0,30—10 Gew.-% | 20:1—10:1 |
| (c) | 0,09—14,40 Gew.-% | 0,15—0,76 Gew.-% | 20:1—1:3 |
| (d) | 0,18—16 Gew.-% | 0,15—0,76 Gew.-% | 1:2—1:3,5 |

wobei sich die Prozentsätze auf die Bestandteile jener Komponente des Systems beziehen, die den entsprechenden Bestandteil enthält.

28. Verfahren unter Verwendung der Zusammensetzung von Anspruch 27, dadurch gekennzeichnet, daß die Harz-Photoinitiator-Mischung mit dem Peroxid-beschichteten Füllstoff bei der Verwendung unter normaler Innenraumbeleuchtung vermischt wird und die erhaltene Mischung innerhalb einiger Minuten danach angewendet und hierauf zehn bis dreißig Sekunden lang einem sichtbaren Licht hoher Intensität ausgesetzt wird.

29. Verfahren nach Anspruch 27, wobei der Schritt der Vermischung des Harzes und des Photoinitiators besteht aus der Vermischung, in Gewichtsprozent:

| | |
|---|---|
| 2,2′-Propan-bis-[3-(4-phenoxy)-1,2-hydroxypropan-1-methacrylat] | zu 54,6% und |
| Triäthylenglykoldimethacrylat | zu 36,4% mit |
| 2-Isopropylthioxanthon | zu 0,02% und |
| Äthyl-4-dimethylaminobenzoat | zu 8,98% |

und der Schritt der Beschichtung im wesentlichen besteht aus der Beschichtung einer Mischung von

| | |
|---|---|
| Lithiumaluminiumsilikat | zu 69,79% und |
| Bariumglas | zu 29,91% mit |
| Benzoylperoxid | zu 0,30% |

alles in Gewichtsprozent

und der Schritt der Vermischung der Harz-Photoinitiator-Mischung mit dem Peroxid-beschichteten Füllstoff besteht aus der Vermischung in einem Gewichtsverhältnis von drei Teilen beschichteter Füllstoff zu einem Teil Harz-Photoinitiator-Flüssigkeit.

30. Verfahren nach Anspruch 27, wobei der Schritt der Vermischung des Harzes und des Photoinitiators besteht aus der Vermischung, in Gewichtsprozent, von:

| | |
|---|---|
| äthoxyliertes Bisphenol-A-Dimethacrylat | zu 72,8% und |
| Triäthylenglykoldimethacrylat | zu 18,2% mit |
| 2-Isopropylthioxanthon | zu 0,02% und |
| Äthyl-4-dimethylaminobenzoat | zu 8,98%, |

und der Schritt der Beschichtung besteht aus der Beschichtung einer Mischung von

| | |
|---|---|
| Lithiumaluminiumsilikat | zu 69,79% und |
| Bariumglas | zu 29,91% mit |
| Benzoylperoxid | zu 0,30% |

alles in Gewichtsprozent

und der Schritt der Vermischung der Harz-Photoinitiator-Mischung mit dem Füllstoff besteht aus der Vermischung bei der Verwendung in einem Gewichtsverhältnis von etwa drei Teilen beschichteter Füllstoff zu einem Teil Harz-Photoinitiator-Mischung.

31. System oder Verfahren nach den Ansprüchen (1, 8, 9, 15, 16, 19, 20, 24) bzw. Ansprüchen (29, 31), wobei das Peroxid-Vernetzungsmittel Benzoylperoxid ist.

32. Verfahren nach Anspruch 25, umfassend ein Flüssigkeit-Pulver-System, wobei der Schritt der Mischung des unvernetzten Harzes umfaßt die Mischung von äthoxyliertem Bisphenol-A-Dimethacrylat in einer Menge von 91,50 Gew.-% mit 2-Isopropylthioxanthon in einer Menge von 0,17 Gew.-% und mit Äthyl-4-dimethylaminobenzoat in einer Menge von 8,33 Gew.-%, und

der Schritt der Beschichtung umfaßt die Beschichtung einer Mischung von Bariumglas in einer Menge von 29,82 Gew.-%, Lithiumaluminiumsilikat in einer Menge von 67,30 Gew.-%, Flintquarz in einer Menge von 1,22 Gew.-%, $TiO_2$ in einer Menge von 0,02 Gew.-% mit Benzoylperoxid in einer Menge von 0,15 Gew.-% und mit Gamma-Methacryloxypropyltrimethoxysilan in einer Menge von 1,47 Gew.-% und mit Eisessig in einer Menge von 0,02 Gew.-%,

und der Schritt der Vermischung der pulverförmigen und flüssigen Komponenten darin besteht, sie unmittelbar vor der Verwendung in einem Gewichtsverhältnis von 2:1 bis 3,5:1 Pulver zu Flüssigkeit zu vermischen.

33. Verfahren nach den Ansprüchen 27 und 28 und umfassend ein Flüssigkeit-Pulver-System, wobei der Schritt der Mischung des Harzes umfaßt die Mischung von äthoxyliertem Bisphenol-A-Dimethacrylat zu 91,50 Gew.-% mit 2-Isopropylthioxanthon zu 0,17 Gew.-% und mit Äthyl-4-dimethylaminobenzoat zu 8,33 Gew.-% und

der Schritt der Beschichtung umfaßt die Beschichtung einer Mischung von Bariumglas zu 26,66 Gew.-%, Lithiumaluminiumsilikat zu 62,24 Gew.-% und Flintquarz zu 9,01 Gew.-% mit Benzoylperoxid zu 0,60 Gew.-%, Gamma-Methacryloxypropyltrimethoxysilan zu 1,47 Gew.-% und Eisessig zu 0,02 Gew.-%,

und der Schritt der Vermischung von Flüssigkeit und Pulver in einem Gewichtsverhältnis von 2 bis 3,5:1 Pulver:Flüssigkeit durchgeführt wird.

34. Verfahren zur Herstellung einer lagerfähigen Zwei-Komponenten-Zusammensetzung mit füllstoffhaltigem Harz geeignet zur Reparatur von Porzellan und als Dental-Verbundwerkstoff, umfassend:

Vermischung, als erste Komponente, eines unvernetzten Harzes mit Methacrylatfunktion, das in Dental-Verbundwerkstoffen verwendbar ist, mit einem Photoinitiator für das Harz in einer Menge von 0,09 bis 14,40 Gew.-%, wobei der Photoinitiator sowohl (1) 2-Isopropylthioxanthon oder Benzildimethylacetal als auch (2) entweder Äthyl-4-dimethylaminobenzoat oder Äthyl-2-dimethylaminobenzoat enthält, und mit einer genügenden Menge an pulverförmigen Dental-Füllstoff um ein Gel zu bilden,

Aufbewahren des Gels in einem undurchsichtigen Behälter;

Beschichten, als zweite Komponente, eines gepulverten Dental-Füllstoffs mit einem an das Harz bindenden Silan und mindestens einem beschleunigerfreien Peroxid-Vernetzungsmittel für das Harz in einer wirksamen Menge von 0,15—0,76 Gew.-%, und

Aufbewahren des Füllstoffs als trockenes Pulver.

35. Verfahren unter Verwendung der Zusammensetzung aus Anspruch 34, bestehend darin, daß zum Zeitpunkt der Verwendung das Gel mit dem Pulver unter normalen Innenraumbeleuchtungsbedingungen in einem Gewichtsverhältnis zwischen 20:1 und 1:3 Gel zu Pulver zu einer Paste vermischt wird und diese Paste innerhalb weniger Minuten danach angewendet wird.

36. Verfahren nach Anspruch 34, wobei

der Schritt der Vermischung zur Bildung eines Gels darin besteht, daß die folgenden Bestandteile in den folgenden Gewichtsprozenten vermischt werden:

| | |
|---|---|
| Harz | 50 bis 90 |
| Füllstoff | 49,82 bis 10 |
| Gamma-Methacryloxypropyltrimethoxysilan | 1,23 bis 0,06 |
| obiger Photoinitiator | 0,09 bis 14,40 und |

der Schritt der Beschichtung des Füllstoffs darin besteht, daß der pulverförmige Füllstoff zu 99,75 bis 97,74 Gew.-% mit Gamma-Methacryloxypropyltrimethoxysilan zu 0,10 bis 1,50 Gew.-% und mit dem Peroxid-Vernetzungsmittel zu 0,15 bis 0,76 Gew.-% beschichtet wird.

37. Verfahren nach Anspruch 36, wobei der Schritt der Vermischung die folgenden Bestandteile in den folgenden Gewichtsprozenten enthält:

| | |
|---|---|
| Bis-GMA | 0 bis 72 |
| äthoxyliertes Bisphenol-A-Dimethacrylat | 0 bis 90 |
| Äthylenglykoldimethacrylat | 0 bis 54 |
| Diäthylenglykoldimethacrylat | 0 bis 54 |
| Triäthylenglykoldimethacrylat | 0 bis 54 |
| Polyäthylenglykoldimethacrylat | 0 bis 54 |
| Bariumglas | 0 bis 14,95 |
| Lithiumaluminiumsilikat | 0 bis 49,82 |
| Flintquarz | 0 bis 4,98 |
| Borosilikatglas | 0 bis 49,82 |
| pyrogene synthetische Kieselsäure | 0 bis 49,82 |
| Strontiumglas | 0 bis 49,82 |
| Quarz | 0 bis 49,82 |
| Titandioxid | 0 bis 0,15 |
| Färbemittel (z.B. Eisenoxide) | 0 bis 5 |
| Gamma-Methacryloxypropyltrimethoxysilan | 0 bis 0,75 |
| obiger Photoinitiator | 0,09 bis 14,40 |

und der Schritt der Beschichtung einige der folgenden Bestandteile in den folgenden Gewichtsteilen umfaßt:

| | |
|---|---|
| Bariumglas | 0 bis 30 |
| Lithiumaluminiumsilikat | 0 bis 99,75 |
| Flintquarz | 0 bis 10 |
| Borosilikat | 0 bis 99,75 |
| pyrogene synthetische Kieselsäure | 0 bis 99,75 |
| Quarz | 0 bis 99,75 |
| Strontiumglas | 0 bis 99,75 |
| Titandioxid | 0 bis 1,50 |
| Färbemittel (z.B. Eisenoxide) | 0 bis 5 |
| Gamma-Methacryloxypropyltrimethoxysilan | 0 bis 1,50 |
| Peroxid-Vernetzungsmittel | 0,15 bis 0,76. |

38. Verfahren nach Anspruch 34, wobei der Schritt der Mischung zur Bildung des Gels besteht in der Vermischung von, in Gewichtsprozent:

| | |
|---|---|
| äthoxyliertes Bisphenol-A-Dimethacrylat | 76,40% |
| 2-Isopropylthioxanthon | 0,06% |
| Äthyl-4-dimethylaminobenzoat | 6,90% |
| pyrogene Kieselsäure | 8,30% |
| Bariumglas | 2,43% |
| Lithiumaluminiumsilikat | 5,74% |
| Gamma-Methacryloxypropyltrimethoxysilan | 0,08% |
| Eisessig | 0,04% |

und der Schritt der Beschichtung des Füllstoffs besteht, in Gewichtsprozent, in

Beschichten einer Mischung von

| | |
|---|---|
| Bariumglas | 29,82% |
| Lithiumaluminiumsilikat | 67,30% |
| Flintquarz | 1,22% und |
| $TiO_2$ | 0,02% mit |
| Benzoylperoxid | 0,15% |
| Gamma-Methacryloxypropyltrimethoxysilan | 0,47% und |
| Eisessig | 0,02% |

39. Verfahren nach Anspruch 34, wobei der Schritt der Mischung zur Bildung des Gels besteht in der Vermischung, in Gewichtsprozent, von:

| | |
|---|---|
| äthoxyliertes Bisphenol-A-Dimethacrylat | 76,40% |
| 2-Isopropylthioxanthon | 0,06% |
| Äthyl-4-dimethylaminobenzoat | 6,90% |
| pyrogene Kieselsäure | 8,30% |
| Bariumglas | 2,43% |
| Lithiumaluminiumsilikat | 5,74% |
| Gamma-Methacryloxypropyltrimethoxysilan | 0,08% |
| Eisessig | 0,04% |

und der Schritt der Beschichtung besteht im Beschichten in den angeführten Gewichtsprozenten, einer Mischung von:

| | |
|---|---|
| Bariumglas | 26,66% |
| Lithiumaluminiumsilikat | 62,24% und |
| Flintquarz | 9,01% mit |
| Benzoylperoxid | 0,60% |
| Gamma-Methacryloxypropyltrimethoxysilan | 0,47% und |
| Eisessig | 0,02% |

40. Verfahren nach Anspruch 34, wobei der Schritt der Mischung zur Bildung des Gels besteht in der Vermischung, in Gewichtsprozent, von:

| | |
|---|---|
| äthoxyliertes Bisphenol-A-Dimethacrylat | 76,34% |
| 2-Isopropylthioxanthon | 0,14% |
| Äthyl-4-dimethylaminobenzoat | 6,90% |
| pyrogene Kieselsäure | 16,62% und |

und der Schritt der Beschichtung des Füllstoffs besteht im Beschichten in den angeführten Gewichtsprozenten, einer Mischung von:

| | |
|---|---|
| Bariumglas | 29,82% |
| Lithiumaluminiumsilikat | 67,30% |
| Flintquarz | 1,22% und |
| $TiO_2$ | 0,02% mit |
| Benzoylperoxid | 0,15% |
| Gamma-Methacryloxypropyltrimethoxysilan | 1,47% und |
| Eisessig | 0,02%. |

41. Verfahren zur Herstellung einer lagerfähigen füllstoffhaltigen Harz-Zusammensetzung verwendbar zur Reparatur von Porzellan und als Dental-Verbundwerkstoff und umfassend ein Paste-Paste-System, bestehend in:

der Mischung eines in Dental-Verbundwerkstoffen verwendbaren Harzes mit Methacrylatfunktion mit einem Photoinitiator dafür in einer Menge von 0,06—12,8 Gew.-%,

wobei der Photoinitiator sowohl (1) 2-Isopropylthioxanthon oder Benzildimethylacetal als auch (2) entweder Äthyl-4-dimethylaminobenzoat oder Äthyl-2-dimethylaminobenzoat enthält, mit einer ausreichenden Menge an pulverförmigem Dental-Füllstoff, der mit einem an das Harz bindenden Silan beschichtet ist, um eine erste Paste herzustellen,

dem Aufbewahren dieser ersten Paste in einem undurchsichtigen Behälter,

dem Beschichten eines pulverförmigen Dental-Füllstoffs mit einem an das Harz bindenden Silan und mindestens einem beschleunigerfreien Peroxid-Vernetzungsmittel für das Harz in einer wirksamen Menge von 0,30—0,75 Gew.-%,

dem Vermischen des beschichteten Füllstoffs mit etwas Harz mit Methacrylatfunktion um eine zweite Paste herzustellen,

dem Aufbewahren der zweiten Paste.

42. Verfahren zur Verwendung der Zusammensetzung nach Anspruch 41, darin bestehend, daß bei der Verwendung die erste und die zweite Paste in im wesentlichen gleichen Gewichtsteilen unter normaler Innenraumbeleuchtung vermischt werden, um eine dritte Paste zu bilden, und daß diese dritte Paste wenige Minuten danach angewendet wird.

43. Verfahren nach Anspruch 41, wobei der Schritt der Mischung zur Bildung der zweiten Paste die Mischung der folgenden Bestandteile in folgenden Gewichtsprozenten umfaßt:

| | |
|---|---|
| Harz | 18 bis 40% |
| Füllstoff | 82 bis 60% |
| Peroxid-Vernetzungsmittel | 0,75 bis 0,30% |
| Gamma-Methacryloxypropyltrimethoxysilan | 1,23 bis 0,06% |
| butyliertes Hydroxytoluol | 0,01 bis 0,10% und |

der Schritt der Herstellung der ersten Paste besteht in der Verwendung der folgenden Bestandteile in den folgenden Mengen:

| | |
|---|---|
| Harz | 18 bis 40% |
| Füllstoff | 80 bis 60% |
| obige Photoinitiatoren | 0,06 bis 12,8% |
| Gamma-Methacryloxypropyltrimethoxysilan | 1,23 bis 0,06%. |

44. Verfahren nach Anspruch 43, wobei der Schritt der Herstellung der zweiten Paste die folgenden Bestandteile in den folgenden Gewichtsprozenten umfaßt:

| | |
|---|---|
| Bis-GMA | 0 bis 32% |
| äthoxyliertes Bisphenol-A-Dimethacrylat | 0 bis 40% |
| Äthylenglykoldimethacrylat | 0 bis 24% |
| Diäthylenglykoldimethacrylat | 0 bis 24% |
| Triäthylenglykoldimethacrylat | 0 bis 24% |
| Polyäthylenglykoldimethacrylat | 0 bis 24% |
| Bariumglas | 0 bis 25% |
| Lithiumaluminiumsilikat | 0 bis 82% |
| Flintquarz | 0 bis 8,5% |
| Borosilikatglas | 0 bis 82% |
| pyrogene synthetische Kieselsäure | 0 bis 52% |
| Quarz | 0 bis 82% |
| Strontiumglas | 0 nis 82% |
| Titandioxid | 0 bis 0,13% |
| Färbemittel (z.B. Eisenoxide) | 0 bis 4% |
| Gamma-Methacryloxypropyltrimethoxysilan | 0 bis 1,25% |
| Peroxid-Vernetzungsmittel | 0,30 bis 0,75% und |

der Schritt der Herstellung der ersten Paste besteht in der Verwendung der folgenden Bestandteile in den folgenden Gewichtsprozenten:

| | |
|---|---|
| Bis-GMA | 0 bis 32 |
| äthoxyliertes Bisphenol-A-Dimethacrylat | 0 bis 40 |
| Äthylenglykoldimethacrylat | 0 bis 24 |
| Diäthylenglykoldimethacrylat | 0 bis 24 |
| Triäthylenglykoldimethacrylat | 0 bis 24 |
| Polyäthylenglykoldimethacrylat | 0 bis 24 |
| Bariumglas | 0 bis 25 |
| Lithiumaluminiumsilikat | 0 bis 82 |
| Flintquarz | 0 bis 8,5 |
| Borosilikatglas | 0 bis 82 |
| pyrogene synthetische Keiselsäure | 0 bis 52 |
| Quarz | 0 bis 82 |
| Strontiumglas | 0 bis 82 |
| Titandioxid | 0 bis 0,13 |
| Färbemittel (z.B. Eisenoxide) | 0 bis 4 |
| Gamma-Methacryloxypropyltrimethoxysilan | 0 bis 1,25 |
| obige Photoinitiatoren | 0,06 bis 12,8. |

45. Verfahren nach Anspruch 41, wobei der Schritt der Herstellung der zweiten Paste besteht im Vermischen der folgenden Bestandteile in den angegebenen Gewichtsprozenten:

| | |
|---|---|
| äthoxyliertes Bisphenol-A-Dimethacrylat | 17,36% |
| Benzoylperoxid | 0,60% |
| butyliertes Hydroxytoluol | 0,04% |
| Strontiumglas | 79,8784% |
| pyrogene Kieselsäure | 0,92% |
| Gamma-Methacryloxypropyltrimethoxysilan | 0,20% |
| Eisessig | 0,0016%, und |

der Schritt der Herstellung der ersten Paste besteht in der Verwendung der folgenden Bestandteile in den folgenden Gewichtsprozenten:

| | |
|---|---|
| äthoxyliertes Bisphenol-A-Dimethacrylat | 14,94% |
| 2-Isopropylthioxanthon | 0,06% |
| Äthyl-4-dimethylaminobenzoat | 3,00% |
| Strontriumglas | 79,8784% |
| pyrogene Kieselsäure | 0,92% |
| Gamma-Methacryloxypropyltrimethoxysilan | 1,20% |
| Eisessig | 0,0016%. |

46. Verfahren zur Herstellung einer lagerfähigen füllstoffhaltigen Harz-Zusammensetzung verwendbar zur Reparatur von Porzellan und als Dental-Verbundwerkstoff und enthaltend ein Paste-Pulver-System, umfassend:

das Mischen eines in Dental-Verbundwerkstoffen verwendbaren Harzes mit Methacrylat-Funktion mit einem Photoinitiator dafür in einer Menge von 0,06—12,8 Gew.-%,

wobei der Photoinitiator sowohl (1) 2-Isopropylthioxanthon oder Benzildimethylacetal als auch (2) entweder Äthyl-4-dimethylaminobenzoat oder Äthyl-2-dimethylaminobenzoat enthält, mit einer ausreichenden Menge an pulverförmigem Dental-Füllstoff, der mit einem an das Harz bindenden Silan beschichtet ist, um eine Paste herzustellen,

das Aufbewahren dieser Paste in einem undurchsichtigen Behälter,

das Beschichten eines pulverförmigen Dental-Füllstoffs mit einem an das Harz bindenden Silan und mindestens einem beschleunigerfreien Peroxid-Vernetzungsmittel für das Harz in einer wirksamen Menge von 0,05—10 Gew.-%, um ein Pulver herzustellen,

das Aufbewahren des Pulvers.

47. Verfahren zur Verwendung der Zusammensetzung nach Anspruch 46, darin bestehend, daß bei der Verwendung das Pulver zu der Paste in eine Menge von 5 bis 10 Gew.-% der Paste unter normaler Innenraumbeleuchtung zugefügt wird, um eine verdickte Paste herzustellen, und

daß diese verdickte Paste innerhalb weniger Minuten danach angewendet wird.

48. Verfahren nach Anspruch 46, wobei

der Schritt der Mischung des Harzes mit dem Photoinitiator und Füllstoff darin besteht, die folgenden Bestandteile, in Gewichtsprozent zu vermischen:

| | |
|---|---|
| Harz | 18 bis 40% |
| Füllstoff | 80 bis 60% |
| obige Photoinitiatoren | 0,06 bis 12,8% |
| Gamma-Methacryloxypropyltrimethoxysilan | 1,23 bis 0,06% und |

der Schritt der Beschichtung des Dental-Füllstoffs besteht in der Beschichtung des Dental-Füllstoffs zu 99,75 bis 97,74 Gew.-% mit Gamma-Methacryloxypropyltrimethoxysilan zu 0,10 bis 1,50 Gew.-% und mit Peroxid-Vernetzungsmittel zu 0,30 bis 10 Gew.-%.

49. Verfahren nach Anspruch 48, wobei der Schritt der Vermischung zur Herstellung der ersten Paste besteht in der Verwendung der folgenden Bestandteile in den folgenden Gewichtsprozenten:

| | |
|---|---|
| Bis-GMA | 0 bis 32% |
| äthoxyliertes Bisphenol-A-Dimethacrylat | 0 bis 40% |
| Äthylenglykoldimethacrylat | 0 bis 24% |
| Diäthylenglykoldimethacrylat | 0 bis 24% |
| Triäthylenglykoldimethacrylat | 0 bis 24% |
| Polyäthylenglykoldimethacrylat | 0 bis 24% |
| Bariumglas | 0 bis 25% |
| Lithiumaluminiumsilikat | 0 bis 82% |
| Flintquarz | 0 bis 8,5% |
| Borosilikatglas | 0 bis 82% |
| pyrogene synthetische Kieselsäure | 0 bis 52% |
| Strontiumglas | 0 bis 82% |
| Quarz | 0 bis 82% |
| Titandioxid | 0 bis 0,13% |
| Färbemittel (z.B. Eisenoxide) | 0 bis 4% |
| Gamma-Methacryloxypropyltrimethoxysilan | 0 bis 1,25% |
| obige Photoinitiatoren | 0,06 bis 12,8% und |

der Schritt der Herstellung des Pulvers umfaßt die Verwendung der folgenden Bestandteile in den folgenden Gewichtsprozenten:

| | |
|---|---|
| Bariumglas | 0 bis 30% |
| Lithiumaluminiumsilikat | 0 bis 99,75% |
| Flintquarz | 0 bis 10% |
| Borosilikatglas | 0 bis 99,75% |
| pyrogene synthetische Kieselsäure | 0 bis 99,75% |
| Strontiumglas | 0 bis 99,75% |
| Titandioxid | 0 bis 0,15% |
| Färbemittel (z.B. Eisenoxide) | 0 bis 5% |
| Gamma-Methacryloxypropyltrimethoxysilan | 0 bis 1,50% |
| Peroxid-Vernetzungsmittel | 0,30 bis 10%. |

50. Verfahren nach Anspruch 46, wobei der Schritt zur Herstellung der ersten Paste besteht in der Vermischung der folgenden Bestandteile in den folgenden Gewichtsprozenten:

| | |
|---|---|
| äthoxyliertes Bisphenol-A-Dimethacrylat | 16,07% |
| 2-Isopropylthioxanthon | 0,03% |
| Äthyl-4-dimethylaminobenzoat | 1,49% |
| pyrogene Kieselsäure | 0,90% |
| Lithiumaluminiumsilikat | 57,08% |
| Bariumglas | 24,45% und |

der Schritt der Herstellung des Pulvers besteht in der Mischung der folgenden Bestandteile in den folgenden Gewichtsprozenten:

| | |
|---|---|
| pyrogene Kieselsäure (vorbeschichtet mit an das Harz bindendem Silan) | 95,00% |
| Benzoylperoxid | 5,00%. |

**Revendications**

1. Système en deux parties pour préparer une composition de résine à fonction méthacrylate, chargée, par mélange des deux parties, comprenant:

première partie: une résine à fonction méthacrylate du type dentaire, avec photoinitiateur prévu à cet effet;

seconde partie: une charge pulvérulente du type dentaire avec un agent durcisseur peroxyde exempt d'accélérateur pour ladite résine;

ledit photoinitiateur contenant à la fois (1) la 2-isopropylthioxanthone ou un benzyl-diméthyl-acétal et (2) soit un éthyl-4-diméthylaminobenzoate soit un éthyl-2-diméthylaminobenzoate;

ledit système en deux parties étant soit

(a) un système pâte-pâte,

(b) un système pâte-poudre,

(c) un système gel-poudre,

(d) un système liquide-poudre,

lesdits photoinitiateur, agent durcisseur peroxyde et lesdites première et seconde parties dudit système étant présents dans une proportion de:

| système | photoinitiateur | agent durcisseur peroxyde | partie 1:partie 2 |
|---|---|---|---|
| (a) | 0,06—12,8% en pds | 0,30—0,75% en pds | 1:1 |
| (b) | 0,06—12,8% en pds | 0,30—10% en pds | 20:1—10:1 |
| (c) | 0,09—14,40% en pds | 0,15—0,76% en pds | 20:1—1:3 |
| (d) | 0,18—16% en pds | 0,15—0,76% en pds | 1:2—1:3,5 |

les pourcentages étant basés sur les composants de la partie du système contenant le composé considéré.

2. Système selon la revendication 1, dans lequel la première partie est sous la forme d'une poudre et la seconde partie est sous la forme d'un gel qui contient également une charge.

3. Système selon la revendication 1, dans lequel les première et deuxième parties sont toutes deux sous forme de pâte, toutes deux contenant une charge.

4. Système selon la revendication 3, dans lequel les première et seconde parties sont formulées de façon à permettre un mélange à parties égales en poids au moment de l'utilisation.

5. Système liquide-poudre selon la revendication 1, constitué, en poids, de

ledit composant en poudre, constitué, en poids, de:

| | |
|---|---|
| Verre de baryum | 0% à 30,00% |
| Silicate de lithium et d'aluminium | 0% à 99,75% |
| Silice flint | 0% à 10,00% |
| Verre de borosilicate | 0% à 99,75% |
| Silice synthétique fumée | 0% à 99,75% |
| Quartz | 0% à 99,75% |
| Dioxyde de titane | 0% à 0,15% |
| Agents de teinture (par ex. oxydes der fer) | 0% à 5,00% |
| Gamma-méthacryloxypropyl-triméthoxy silane | 0% à 1,50% |
| Agent durcisseur peroxyde | 0,15% à 0,76% |

et un composant liquide constitué, en poids, de:

| | |
|---|---|
| Bis-GMA | 0% à 80,00% |
| Diméthacrylate de bisphénol-A éthoxylé | 0% à 99,00% |
| Diméthacrylate d'éthylène glycol | 0% à 60,00% |
| Diméthacrylate de diéthylène glycol | 0% à 60,00% |
| Diméthacrylate de triéthylène glycol | 0% à 60,00% |
| Diméthacrylate de polyéthylène glycol | 0% à 60,00% |
| Ledit photoinitiateur pour les résines ci-dessus | 0,18% à 16,00%, |

ledit composant en poudre et ledit composant liquide étant mélangés, au moment de l'utilisation, dans un rapport en poids de 1:1 à 3,5:1 de la poudre au liquide.

6. Système liquide-poudre selon la revendication 1, constitué de ledit composant en poudre, constitué, en poids, de:

| | |
|---|---|
| charge dentaire | 99,75% à 97,74% |
| Gamma-méthacryloxypropyl-triméthoxysilane | 0,10% à 1,50% |
| Agent durcisseur peroxyde | 0,15% à 0,76% |

et ledit composant liquide constitué, en poids, de

| | |
|---|---|
| ladite résine à fonction méthacrylate adaptée à l'utilisation dans les matériaux composites dentaires | 99,82% à 84,00% |
| ledit photoinitiateur pour ladite résine | 0,18% à 16,00%, |

ladite poudre et ladite résine étant mélangées entre elles dans un rapport en poids de 1:1 à 3,5:1 de la poudre au liquide.

7. Système liquide-poudre selon la revendication 1, dans lequel ledit composant en poudre est constitué de

| | |
|---|---|
| Verre de baryum | 29,82% |
| Silicate de lithium et d'aluminium | 67,30% |
| Silice flint | 1,22% |
| $TiO_2$ | 0,02% |
| Peroxyde de benzoyle | 0,15% |
| Gamma-méthacryloxypropyl-triméthoxy-silane | 1,47% |
| Acide acétique glacial | 0,02% |

et ledit composant liquide est constitué de:

| | |
|---|---|
| Diméthacrylate de bisphénol-A éthoxylé | 91,50% |
| 2-Isopropylthioxanthone | 0,17% |
| 4-Diméthylaminobenzoate d'éthyle | 8,33% |

lesdits liquide et poudre étant mélangés entre eux juste avant le moment d'utilisation dans un rapport poids de 2 à 1 à 3,5:1 poudre:liquide.

8. Système liquide-poudre selon la revendication 1, dans lequel ledit composant en poudre est constitué de:

| | |
|---|---|
| Verre de baryum | 26,66% |
| Silicate de lithium et d'aluminium | 62,24% |
| Silice flint | 9,01% |
| Peroxyde de benzoyle | 0,60% |
| Gamma-méthacryloxypropyl-triméhoxy-silane | 1,47% |
| Acide acétique glacial | 0,02% |

et ledit composant liquide est constitué de:

| | |
|---|---|
| Diméthacrylate de bisphénol-A éthoxylé | 91,50% |
| 2-Isopropylthioxanthone | 0,17% |
| 4-Diméthylaminobenzoate d'éthyle | 8,33% |

lesdits liquide et poudre étant mélangés entre eux dans un rapport en poids de 2 à 3,5:1 poudre:liquide.

9. Système liquide-poudre selon la revendication 1, dans lequel ledit composant liquide est constitué de:

| | |
|---|---|
| Méthacrylate de 2,2'-propane bis[3-(4-phénoxy)-2-hydroxypropane-1] | 54,6% |
| Diméthacrylate de triéthylène glycol | 36,4% |
| 2-Isopropylthioxanthone | 0,02% |
| 4-Diméthylaminobenzoate d'éthyle | 8,98%, |

et ledit composant en poudre étant constitué de:

| | |
|---|---|
| Silicate de lithium et d'aluminium | 69,79% |
| Verre de baryum | 29,91% |
| Peroxyde de benzoyle | 0,30% |

la poudre et le liquide étant mélangés pour l'utilisation dans un rapport en poids de trois parties de poudre pour une partie de liquide.

10. Système liquide-poudre selon la revendication 1, dans lequel ledit composant liquide est constitué de:

| | |
|---|---|
| Diméthacrylate de bisphénol-A éthoxylé | 72,8% |
| Diméthacrylate de triéthylène glycol | 18,2% |
| 2-Isopropylthioxanthone | 0,02% |
| 4-Diméthylaminobenzoate d'éthyle | 8,98% |

le composant en poudre étant constitué de:

| | |
|---|---|
| Silicate de lithium et d'aluminium | 69,79% |
| Verre de baryum | 29,91% |
| Peroxyde de benzoyle | 0,30% |

la poudre et le liquide étant mélangés pour l'utilisation dans un rapport en poids de trois parties de poudre pour une partie de liquide.

11. Système liquide-poudre selon la revendication 1, dans lequel ledit composant poudre est constitué de:

| | Parties en poids |
|---|---|
| Verre de strontium | 100 |
| Peroxyde de benzoyle | 0,3 |
| Gamma-méthacryloxypropyl-triméthoxy-silane | 1,5 |

et ledit composant liquide étant constitué de:

| | |
|---|---|
| Diméthacrylate de bisphénol-A éthoxylé | 91,50% |
| 2-Isopropylthioxanthone | 0,17% |
| 4-Diméthylaminobenzoate d'éthyle | 8,33%, |

lesdits composants poudre et liquide étant mélangés juste avant l'utilisation dans un rapport en poids de 3:1 de la poudre au liquide.

12. Système pâte-pâte selon la revendication 1, constitué, en pourcentages en poids, de:

| | |
|---|---|
| Pâte A: | |
| Résine à fonction méthacrylate, adaptée à l'utilisation dans les matériaux composites dentaires | 18 à 40 |
| charge dentaire | 82 à 60 |
| Agent durcisseur peroxyde | 0,75 à 0,30 |
| Gamma-méthacryloxypropyl-triméthoxy-silane | 1,23 à 0,06 |
| Hydroxytoluène butylé | 0,01 à 0,10 |
| Pâte B: | |
| Résine à fonction méthacrylate, adaptée à l'utilisation dans les matériaux composites dentaires | 18 à 40 |
| charge dentaire | 80 à 60 |
| Lesdits photoinitiateurs | 0,06 à 12,8 |
| Gamma-méthacryloxypropyl-triméthoxy-silane | 1,23 à 0,06 |

les pâtes A et B étant mélangées entre elles au moment de l'utilisation dans des proportions en poids pratiquement équivalentes.

13. Système selon la revendication 12, dans lequel la pâte A est constituée, en pourcentages en poids, de:

| | |
|---|---|
| Bis-GMA | 0 à 32 |
| Diméthacrylate de bisphénol-A éthoxylé | 0 à 40 |
| Diméthacrylate d'éthylène glycol | 0 à 24 |
| Diméthacrylate de diéthylène glycol | 0 à 24 |
| Diméthacrylate de triéthylène glycol | 0 à 24 |
| Diméthacrylate de polyéthylène glycol | 0 à 24 |
| Verre de baryum | 0 à 25 |
| Silicate de lithium et d'aluminium | 0 à 82 |
| Silice flint | 0 à 8,5 |
| Verre de borosilicate | 0 à 82 |
| Silice synthétique fumée | 0 à 52 |
| Quartz | 0 à 82 |
| Verre de strontium | 0 à 82 |
| Dioxyde de titane | 0 à 0,13 |
| Agents de teinture (par ex., oxydes de fer) | 0 à 4 |
| Gamma-méthacryloxypropyl-triméthoxy-silane | 0 à 1,25 |
| Agent durcisseur peroxyde | 0,30 à 0,75 |

et la pâte B est constituée, en pourcentages en poids, de:

| | |
|---|---|
| Bis-GMA | 0 à 32 |
| Diméthacrylate de bisphénol-A éthoxylé | 0 à 40 |
| Diméthacrylate d'éthylène glycol | 0 à 24 |
| Diméthacrylate de diéthylène glycol | 0 à 24 |
| Diméthacrylate de triéthylène glycol | 0 à 24 |
| Diméthacrylate de polyéthylène glycol | 0 à 24 |
| Verre de baryum | 0 à 25 |
| Silicate de lithium et d'aluminium | 0 à 82 |
| Silice flint | 0 à 8,5 |
| Verre de borosilicate | 0 à 82 |
| Silice synthétique fumée | 0 à 52 |
| Quartz | 0 à 82 |
| Verre de strontium | 0 à 82 |
| Dioxyde de titane | 0 à 0,13 |
| Agents de teinture (par ex., oxydes de fer) | 0 à 4 |
| Gamma-méthacryloxypropyl-triméthoxy-silane | 0 à 1,25 |
| Lesdits photoinitiateurs | 0,10 à 12,8. |

14. Système selon la revendication 12, dans lequel ladite première pâte est constitué de:

| | |
|---|---|
| Diméthacrylate de bisphénol-A éthoxylé | 17,36% |
| Peroxyde de benzoyle | 0,60% |
| Hydroxytoluène butylé | 0,04% |
| Verre de strontium | 79,8784% |
| Silice fumée | 0,92% |
| Gamma-méthacryloxypropyl-triméthoxy-silane | 0,20% |
| Acide acétique glacial | 0,0016% |

et ladite seconde pâte est constituée de:

| | |
|---|---|
| Diméthacrylate de bisphénol-A éthoxylé | 14,94% |
| 2-Isopropylthioxanthone | 0,06% |
| 4-Diméthylaminobenzoate d'éthyle | 3,00% |
| Verre de strontium | 79,8784% |
| Silice fumée | 0,92% |
| Gamma-méthacryloxypropyl-triméthoxy-silane | 1,20% |
| Acide acétique glacial | 0,0016%. |

15. Système gel-poudre selon la revendication 1, constitué, en parties en poids, de:

un gel, constitué de:

| | |
|---|---|
| Résine à fonction méthacrylate, adaptée à l'utilisation dans les matériaux composites dentaires | 50 à 90 |
| charge dentaire | 49,82 à 10 |
| Gamma-méthacryloxypropyl-trimethoxy-silane | 1,23 à 0,06 |
| Ledit photoinitiateur | 0,09 à 14,40 |

et une poudre, constituée de:

| | |
|---|---|
| charge dentaire | 99,75 à 97,74 |
| Gamma-méthacryloxypropyl-triméthoxy-silane | 0,10 à 1,50 |
| Agent durcisseur peroxyde | 0,15 à 0,76 |

le gel et la poudre étant mélangés entre eux au moment de l'utilisation, dans un rapport en poids du gel à la poudre de 20:1 à 1:3.

16. Système selon la revendication 15, dans lequel les composants du gel et de la poudre sont constitués, en poids, des composés suivants:

gel:

| | |
|---|---|
| Bis-GMA | 0 à 72% |
| Diméthacrylate de bisphénol-A éthoxylé | 0 à 90% |
| Diméthacrylate d'éthylène glycol | 0 à 54% |
| Diméthacrylate de diéthylène glycol | 0 à 54% |
| Diméthacrylate de triéthylène glycol | 0 à 54% |
| Diméthacrylate de polyéthylène glycol | 0 à 54% |
| Verre de baryum | 0 à 14,95% |
| Silicate de lithium et d'aluminium | 0 à 49,82% |
| Silice flint | 0 à 4,98% |
| Verre de borosilicate | 0 à 49,82% |
| Silice synthétique fumée | 0 à 49,82% |
| Quartz | 0 à 49,82% |
| Verre de strontium | 0 à 49,82% |
| Dioxyde de titane | 0 à 0,15% |
| Agents de teinture (par ex., oxydes de fer) | 0 à 5% |
| Gamma-méthacryloxypropyl-triméthoxy-silane | 0 à 0,75% |
| Ledit photoinitiateur | 0,09 à 14,40% |

poudre:

| | |
|---|---|
| Verre de baryum | 0 à 30% |
| Silicate de lithium et d'aluminium | 0 à 99,75% |
| Silice flint | 0 à 10% |
| Verre de borosilicate | 0 à 99,75% |
| Silice synthétique fumée | 0 à 99,75% |
| Quartz | 0 à 99,75% |
| Verre de strontium | 0 à 99,75% |
| Dioxyde de titane | 0 à 1,50% |
| Agents de teinture (par ex., oxydes de fer) | 0 à 5% |
| Gamma-méthacryloxypropyl-triméthoxy-silane | 0 à 1,50% |
| Agent durcisseur peroxyde | 0,15 à 0,76%. |

17. Système selon la revendication 15, dans lequel le gel est constitué, en poids, de:

| | |
|---|---|
| Diméthacrylate de bisphénol-A éthoxylé | 76,40% |
| 2-Isopropylthioxanthone | 0,06% |
| 4-Diméthylaminobenzoate d'éthyle | 6,90% |
| Silice fumée | 8,30% |
| Verre de baryum | 2,43% |
| Silicate de lithium et d'aluminium | 5,74% |
| Gamma-méthacryloxypropyl-triméthoxy-silane | 0,08% |
| Acide acétique glacial | 0,04% |

et ladite poudre est constituée de:

| | |
|---|---|
| Verre de baryum | 29,82% |
| Silicate de lithium et d'aluminium | 67,30% |
| Silice flint | 1,22% |
| $TiO_2$ | 0,02% |
| Peroxyde de benzoyle | 0,15% |
| Gamma-méthacryloxypropyl-triméthoxy-silane | 0,47% |
| Acide acétique glacial | 0,02% |

le gel et la poudre étant mélangés au moment de l'utilisation dans un rapport en poids compris entre 1:20 et 3:1 de la poudre au gel.

18. Système selon la revendication 15, dans lequel ledit gel est constitué, en poids, de:

| | |
|---|---|
| Diméthacrylate de bisphénol-A éthoxylé | 76,40% |
| 2-Isopropylthioxanthone | 0,06% |
| 4-Diméthylaminobenzoate d'éthyle | 6,90% |
| Silice fumée | 8,30% |
| Verre de baryum | 2,43% |
| Silicate de lithium et d'aluminium | 5,74% |
| Gamma-méthacryloxypropyl-triméthoxy-silane | 0,08% |
| Acide acétique glacial | 0,04% |

et ladite poudre est constituée de:

| | |
|---|---|
| Verre de baryum | 26,66% |
| Silicate de lithium et d'aluminium | 62,24% |
| Silice flint | 9,01% |
| Peroxyde de benzoyle | 0,60% |
| Gamma-méthacryloxypropyl-triméthoxy-silane | 1,47% |
| Acide acétique glacial | 0,02% |

le gel et la poudre étant mélangés au moment de l'utilisation dans un rapport en poids compris entre 1:20 et 3:1 de la poudre au gel.

19. Système selon la revendication 15, dans lequel ledit gel est constitué, en poids, de:

| | |
|---|---|
| Diméthacrylate de bisphénol-A éthoxylé | 76,34% |
| 2-Isopropylthioxanthone | 0,14% |
| 4-Diméthylaminobenzoate d'éthyle | 6,90% |
| Silice fumée | 16,62% |

et ladite poudre est constituée de:

| | |
|---|---|
| Verre be baryum | 29,82% |
| Silicate de lithium et d'aluminium | 67,30% |
| Silice flint | 1,22% |
| $TiO_2$ | 0,02% |
| Peroxyde de benzoyle | 0,15% |
| Gamma-méthacryloxypropyl-triméthoxy-silane | 0,47% |
| Acide acétique glacial | 0,02% |

le gel et la poudre étant mélangés au moment de l'utilisation dans un rapport en poids compris entre 1:20 et 3:1 de la poudre au gel.

20. Système pâte-poudre selon la revendication 1, constitué en pourcentages en poids de:

une pâte, constituée de:

| | |
|---|---|
| Résine à fonction méthacrylate, adaptée à l'utilisation dans les matériaux composites dentaires | 18 à 40 |
| charge dentaire | 80 à 60 |
| Lesdits photoinitiateurs | 0,06 à 12,8 |
| Gamma-méthacryloxypropyl-triméthoxy-silane | 1,23 à 0,06 |

et une poudre, constituée de:

| | |
|---|---|
| charge dentaire | 99,75 à 97,74 |
| Gamma-méthacryloxypropyl-triméthoxy-silane | 1,10 à 1,50 |
| Agent durcisseur peroxyde | 0,30 à 10 |

ladite poudre étant ajoutée à ladite pâte au moment de l'utilisation dans un rapport en poids de 20:1 à 10:1 de la pâte à la poudre.

21. Système selon la revendication 20, dans lequel lesdites pâte et poudre sont constituées des composants suivants, exprimé en pourcentages en poids:

Pâte:

| | |
|---|---|
| Bis-GMA | 0 à 32 |
| Diméthacrylate de bisphénol-A éthoxylé | 0 à 40 |
| Diméthacrylate d'éthylène glycol | 0 à 24 |
| Diméthacrylate de diéthylène glycol | 0 à 24 |
| Diméthacrylate de triéthylène glycol | 0 à 24 |
| Diméthacrylate de polyéthylène glycol | 0 à 24 |
| Verre de baryum | 0 à 25 |
| Silicate de lithium et d'aluminium | 0 à 82 |
| Silice flint | 0 à 8,5 |
| Verre de borosilicate | 0 à 82 |
| Silice synthétique fumée | 0 à 52 |
| Quartz | 0 à 82 |
| Verre de strontium | 0 à 82 |
| Dioxyde de titane | 0 à 0,13 |
| Agents de teinture (par ex., oxydes de fer) | 0 à 4 |
| Gamma-méthacryloxypropyl-triméthoxy-silane | 0 à 1,25 |
| Lesdits photoinitiateurs | 0,06 à 12,8 |

poudre:

| | |
|---|---|
| Verre de baryum | 0 à 30 |
| Silicate de lithium et d'aluminium | 0 à 99,75 |
| Silice flint | 0 à 10 |
| Verre de borosilicate | 0 à 99,75 |
| Silice synthétique fumée | 0 à 99,75 |
| Verre de strontium | 0 à 99,75 |
| Dioxyde de titane | 0 à 0,15 |
| Agents de teinture (par ex., oxydes de fer) | 0 à 5 |
| Gamma-méthacryloxypropyl-triméthoxy-silane | 0 à 1,50% |
| Agent durcisseur peroxyde | 0,30 à 0,10. |

22. Système selon la revendication 20, dans lequel ledit composant pâte est constitué de:

| | |
|---|---|
| Diméthacrylate de bisphénol-A éthoxylé | 16,07% |
| 2-Isopropylthioxanthone | 0,03% |
| 4-Diméthylaminobenzoate d'éthyle | 1,49% |
| Silice fumée | 0,90% |
| Silicate de lithium et d'aluminium | 57,08% |
| Verre de baryum | 24,45% |

et ladite poudre est constituée de:

| | |
|---|---|
| Silice fumée (pré-enrobée de silane) | 95,00% |
| Peroxyde de benzoyle | 5% |

la poudre étant ajoutée à la pâte au moment de l'utilisation dans un rapport en poids de 20:1 à 10:1 de la pâte à la poudre.

23. Système selon la revendication 20, dans lequel ledit composant pâte est constitué de:

| | Parties en poids |
|---|---|
| Bis-GMA | 60 |
| Diméthacrylate de triéthylène glycol | 40 |
| 2-Isopropylthioxanthone | 0,18 |
| 4-Diméthylaminobenzoate d'éthyle | 9,10 |
| Silicate de lithium et d'aluminium | 355 |
| Verre de baryum | 152 |

et ledit composant poudre étant constitué de:

| | Parties en poids |
|---|---|
| Silicate de lithium et d'aluminium | 70 |
| Verre de baryum | 30 |
| Peroxyde de benzoyle | 0,75 |

la pâte et la poudre étant mélangés au moment de l'utilisation dans un rapport en poids de 20:1 de la pâte à la poudre.

24. Système selon la revendication 20, dans lequel ledit composant pâte est constitué de:

| | Parties en poids |
|---|---|
| Diméthacrylate de bisphénol-A éthoxylé | 80 |
| Diméthacrylate de triéthylène glycol | 20 |
| 2-Isopropylthioxanthone | 0,18 |
| 4-Diméthylaminobenzoate d'éthyle | 9,10 |
| Silicate de lithium et d'aluminium | 355 |
| Verre de baryum | 152 |

et ladite poudre est constituée de:

| | Parties en poids |
|---|---|
| Silicate de lithium et d'aluminium | 70 |
| Verre de baryum | 30 |
| Peroxyde de benzoyle | 0,30 |

la poudre étant ajoutée à la pâte au moment de l'utilisation dans un rapport en poids de 20:1 de la pâte à la poudre.

25. Procédé pour la réparation de porcelaine ou de dents avec l'aide d'un système en deux parties, ledit procédé comprenant les étapes consistant à:

mélanger entre eux, dans des conditions d'éclairage intérieur habituelles:

— une résine à fonction méthacrylate utilisable dans les matériaux composites dentaires et un photoinitiateur pour ladite résine, ledit photoinitiateur comprenant à la fois (1) la 2-isopropylthioxanthone ou le benzyl diméthyl acétal et (2) soit le 4-diméthylaminobenzoate d'éthyle soit le 2-diméthylaminobenzoate d'éthyle (partie 1), et

— une charge dentaire en poudre et au moins un agent durcisseur peroxyde exempt d'accélérateur pour ladite résine (partie 2),

ledit système en deux parties étant soit

(a) un système pâte-pâte,

(b) un système pâte-poudre,

(c) un système gel-poudre,

(d) un système liquide-poudre,

lesdits photoinitiateur, agent durcisseur peroxyde et lesdites première et seconde parties desdits systèmes étant présents dans une proportion de:

| système | photoinitiateur | agent durcisseur peroxyde | partie 1:partie 2 |
|---|---|---|---|
| (a) | 0,06—12,8% en pds | 0,30—0,75% en pds | 1:1 |
| (b) | 0,06—12,8% en pds | 0,30—10% en pds | 20:1—10:1 |
| (c) | 0,09—14,40% en pds | 0,15—0,76% en pds | 20:1—1:3 |
| (d) | 0,18—16% en pds | 0,15—0,76% en pds | 1:2—1:3,5 |

les pourcentages étant basés sur les composants de la partie du système contenant le composé considéré;

mettre en place le mélange dans un délai de quelques minutes après l'opération de mélange, et soumettre au durcissement une partie importante, exprimée du mélange mis en place *in situ* sous éclairage intense, toute résine non durcie alors par la lumière se durcissant dans l'heure qui suit à l'aide dudit agent durcisseur peroxyde.

26. Système ou procédé selon les revendications 1 et 5 respectivement, dans laquelle la résine est au moins une des résines choisie dans le groupe constitué par le diméthacrylate de bisphénol-A éthoxylé, le bis-GMA, un adduct du 2,2'-propane bis[3-(4-phénoxy)-1,2-dihydroxypropane-1-méthacrylate] et de mono- ou diisocyanate, et d'un monomère méthacrylate comprenant de 2 à 4 liaisons doubles polymérisables, avec 0,3 à 2,0% en poids de ce monomère, d'un agent réducteur thiourée substitué répondant à la formule

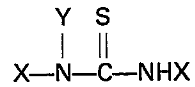

dans laquelle X représente l'hydrogène ou Y et Y représente un groupe alkyle en $C_1$ à $C_8$; un groupe

cycloalkyle en $C_3$ à $C_6$; un groupe allyle en $C_1$ à $C_8$, chloro-, hydroxy- ou mercapto-substitué; un groupe alcényle en $C_3$ à $C_4$; un groupe aryle en $C_6$ à $C_8$; un groupe phényle chloro-, hydroxy- ou méthoxy- ou sulfonyle-substitué; un groupe acyle en $C_2$ à $C_8$; un groupe acyle en $C_2$ à $C_8$ chloro- ou méthoxy-substitué; un groupe aralkyle en $C_7$ à $C_8$; ou un groupe aralkyle en $C_7$ à $C_8$ chloro- ou méthoxy-substitué.

27. Procédé pour la préparation d'une composition chargée de résine, en deux parties, apte au stockage, utilisable dans la réparation de la porcelaine et comme composition dentaire, comprenant:

l'enrobage, pour préparer la seconde partie, d'une charge dentaire en poudre avec au moins un agent durcisseur exempt d'accélérateur pour ladite résine,

le stockage de ladite charge:

le mélange, pour préparer la première partie, d'une résine à fonction méthacrylate non durcie, utilisable dans les matériaux composites dentaires, avec un photoinitiateur pour ladite résine,

ledit photoinitiateur comprenant à la fois (1) la 2-isopropylthioxanthone ou le benzyl diméthyl acétal et (2) soit le 4-diméthylaminobenzoate d'éthyle soit le 2-diméthylaminobenzoate d'éthyle,

le stockage du mélange résine-photoinitiateur dans un conteneur opaque;

ledit système en deux parties étant soit

(a) un système pâte-pâte,

(b) un système pâte-poudre,

(c) un système gel-poudre,

(d) un système liquide-poudre,

lesdits photoinitiateur, agent durcisseur peroxyde et lesdites première et seconde parties desdits systèmes étant présents dans une proportion de:

| système | photoinitiateur | agent durcisseur peroxyde | partie 1:partie 2 |
|---|---|---|---|
| (a) | 0,06—12,8% en pds | 0,30—0,75% en pds | 1:1 |
| (b) | 0,06—12,8% en pds | 0,30—10% en pds | 20:1—10:1 |
| (c) | 0,09—14,40% en pds | 0,15—0,76% en pds | 20:1—1:3 |
| (d) | 0,18—16% en pds | 0,15—0,76% en pds | 1:2—1:3,5 |

les pourcentages étant basés sur les composants de la partie du système contenant le composé considéré.

28. Procédé pour l'utilisation de la composition de la revendication 27, caracterisé en ce que, au moment de l'utilisation, ledit mélange résine-photoinitiateur est mélangé avec ladite charge enrobée de peroxyde sous éclairage intérieur ordinaire, le mélange obtenu étant appliqué quelques minutes après, et ensuite, exposé à une lumière visible intense pendant dix à trente secondes.

29. Procédé selon la revendication 27, dans lequel ladite étape de mélange de ladite résine et dudit photoinitiateur consiste à mélanger, en poids,

| | |
|---|---|
| 2,2'-Propane bis[3-(4-phénoxy)-1,2-Dihydroxy-propane-1-méthacrylate] | à 54,6% et |
| Diméthacrylate de triéthylène glycol | à 36,4% avec |
| 2-Isopropylthioxanthone | à 0,02% et |
| 4-Dimethylaminobenzoate d'éthyle | à 8,98%, |

ladite étape d'enrobage consistant essentiellement à enrober un mélange de

| | |
|---|---|
| Silicate de lithium et d'aluminium | à 69,79% et |
| Verre de baryum | à 29,91% avec |
| Peroxyde de benzoyle | à 0,30%, tout exprimé en poids, et |

ladite étape de mélange du mélange résine-photoinitiateur avec ladite charge enrobée de peroxyde comprenant le mélange dans un rapport en poids de trois parties de charge enrobée pour une partie de liquide résine-photoinitiateur.

30. Procédé selon la revendication 27, dans lequel

ladite étape de mélange de ladite résine et dudit photoinitiateur consiste à mélanger, en poids,

| | |
|---|---|
| Diméthacrylate de bisphénol-A éthoxylé | à 72,8% et |
| Diméthacrylate de triéthylène glycol | à 18,2% avec |
| 2-Isopropylthioxanthone | à 0,02% et |
| 4-Diméthylaminobenzoate d'éthyle | à 8,98%, |

ladite étape d'enrobage consistant essentiellement à enrober un mélange de

| | |
|---|---|
| Silicate de lithium et d'aluminium | à 69,79% et |
| Verre de baryum | à 29,91% avec |
| Peroxyde de benzoyle | à 0,30%, tout exprimé en poids, et |

ladite étape de mélange du mélange résine-photoinitiateur avec ladite charge comprenant leur mélange au moment de l'utilisation dans un rapport en poids d'environ trois parties de charge enrobée pour une partie de mélange résine-photoinitiateur.

31. Système ou procédé selon les revendications (1, 8, 9, 15, 16, 19, 20, 24) et les revendications (29, 31), respectivement, dans lequel l'agent durcisseur peroxyde est le peroxyde de benzoyle.

32. Procédé selon la revendication 25 et impliquant un système liquide-poudre, dans lequel l'étape de mélange de la résine non durcie comprend le mélange du diméthacrylate de bisphénol-A éthoxylé dans une proportion de 91,50% en poids à la 2-isopropylthioxanthone dans une proportion de 0,17% en poids et au 4-diméthylaminobenzoate d'éthyle dans une proportion de 8,33% en poids, et

l'étape d'enrobage comprend l'enrobage d'un mélange de verre de baryum dans une proportion de 29,82% en poids, de silicate d'aluminium et de lithium dans une proportion de 67,30% en poids, de silice flint dans une proportion de 1,22% en poids, de $TiO_2$ dans une proportion de 0,02%, en poids avec du peroxyde de benzoyle dans une proportion de 0,15% en poids, et avec le gamma-méthacryloxypropyl-triméthoxy-silane dans une proportion de 1,47% en poids, et avec de l'acide acétique glacial dans une proportion de 0,02% en poids,

et l'étape de mélange desdits composants liquide et poudre comprend leur mélange juste avant l'utilisation dans un rapport en poids de 2:1 à 3,5:1 de la poudre au liquide.

33. Procédé selon les revendications 27 et 28 et impliquant un système liquide-poudre, dans lequel l'étape de mélange de la résine comprend le mélange, en proportions en poids, du diméthacrylate de bisphénol-A éthoxylé à 91,50% à la 2-isopropylthioxanthone à 0,17% et au 4-diméthylaminobenzoate d'éthyle à 8,33%, et l'étape d'enrobage comprend l'enrobage d'un mélange, en poids, de verre de baryum à de 26,66%, de silicate d'aluminium et de lithium à 62,24% et de silice flint dans une proportion de 9,01%, avec du peroxyde de benzoyle à 0,60%, le gamma-méthacryloxypropyl-triméthoxy-silane à 1,47% et avec de l'acide acétique glacial dans une proportion de 0,02%,

l'étape de mélange du liquide et de la poudre étant effectuée dans un rapport en poids de 2 à 3,5:1 de la poudre au liquide.

34. Procédé pour la préparation d'une composition chargée de résine, en deux parties, apte au stockage, utilisable dans la réparation de la porcelaine et comme composition dentaire, comprenant:

le mélange, comme première partie, d'une résine à fonction méthacrylate, non durcie, utilisable dans les matériaux composites dentaires, avec un photoinitiateur pour ladite résine, dans une proportion de 0,09 à 14,40% en poids, ledit photoinitiateur comprenant à la fois (1) la 2-isopropylthioxanthone ou le benzyl diméthyl acétal et (2) soit le 4-diméthylaminobenzoate d'éthyle soit le 2-diméthylaminobenzoate d'éthyle, et avec suffisamment de charge dentaire en poudre pour former un gel,

le stockage du gel dans un conteneur opaque,

l'enrobage, comme seconde partie, d'une charge dentaire en poudre avec un silane liant la résine et au moins un agent durcisseur peroxyde exempt d'accélérateur pour ladite résine, dans une proportion efficace de 0,15—0,76% en poids, et

le stockage de ladite charge sous la forme d'une poudre sèche.

35. Procédé pour l'utilisation de la composition de la revendication 34, comprenant le mélange, au moment de l'utilisation, dudit gel avec ladite poudre sous éclairage intérieur ordinaire, le mélange obtenu étant appliqué dans un rapport en poids compris entre 20:1 et 1:3, du gel à la poudre, pour former une pâte, et l'application de ladite pâte dans un délai de quelques minutes.

36. Procédé selon la revendication 34, dans lequel ladite étape de mélange pour former un gel comprenant le mélange des composants suivants, dans les pourcentages en poids suivants:

| | |
|---|---|
| résine | 50 à 90 |
| charge | 49,82 à 10 |
| γ-méthacryloxypropyltriméthoxysilane | 1,23 à 0,06 |
| ledit photoinitiateur | 0,09 à 14,40, et |

ladite étape d'enrobage de la charge comprend l'enrobage de la charge en poudre dans une proportion de 99,75 à 97,74% en poids, avec 0,10 à 1,50% en poids de γ-méthacryloxypropyltriméthoxysilane et avec un agent durcisseur peroxyde dans une proportion de 0,15 à 0,76% en poids.

37. Procédé selon la revendication 36, dans lequel ladite étape de mélange comprend les constituants suivants, dans les pourcentages en poids suivants:

| | |
|---|---|
| Bis-GMA | 0 à 72 |
| Diméthacrylate de bisphénol-A éthoxylé | 0 à 90 |
| Diméthacrylate d'éthylène glycol | 0 à 54 |
| Diméthacrylate de diéthylène glycol | 0 à 54 |
| Diméthacrylate de triéthylène glycol | 0 à 54 |
| Diméthacrylate de polyéthylène glycol | 0 à 54 |
| Verre de baryum | 0 à 14,95 |
| Silicate de lithium et d'aluminium | 0 à 49,82 |
| Silice flint | 0 à 4,98 |
| Verre de borosilicate | 0 à 49,82 |
| Silice synthétique fumée | 0 à 49,82 |
| Verre de strontium | 0 à 49,82 |
| Quartz | 0 à 49,82 |
| Dioxyde de titane | 0 à 0,15 |
| Agents de teinture (par ex., oxydes de fer) | 0 à 5 |
| Gamma-méthacryloxypropyl-triméthoxy-silane | 0 à 0,75 |
| Ledit photoinitiateur | 0,09 à 14,40, et |

ladite étape d'enrobage utilise certains des constituants ci-après, dans les proportions suivantes, exprimées en parties en poids:

| | |
|---|---|
| Verre de baryum | 0 à 30 |
| Silicate de lithium et d'aluminium | 0 à 99,75 |
| Silice flint | 0 à 10 |
| Verre de borosilicate | 0 à 99,75 |
| Silice synthétique fumée | 0 à 99,75 |
| Quartz | 0 à 99,75 |
| Verre de strontium | 0 à 99,75 |
| Dioxyde de titane | 0 à 1,50 |
| Agents de teinture (par ex., oxydes de fer) | 0 à 5 |
| Gamma-méthacryloxypropyl-triméthoxy-silane | 0 à 1,50 |
| Agent durcisseur peroxyde | 0,15 à 0,76. |

38. Procédé selon la revendication 34, dans lequel ladite étape de mélange pour former un gel comprend le mélange des composants suivants, dans les pourcentages en poids suivants:

| | |
|---|---|
| Diméthacrylate de bisphénol-A éthoxylé | 76,40% |
| 2-Isopropylthioxanthone | 0,06% |
| 4-Diméthylaminobenzoate d'éthyle | 6,90% |
| Silice fumée | 8,30% |
| Verre de baryum | 2,43% |
| Silicate d'aluminium et de lithium | 5,74% |
| Gamma-méthacryloxypropyl-triméthoxy-silane | 0,08% |
| Acide acétique glacial | 0,04% |

ladite étape d'enrobage de la charge dentaire étant constituée, les proportions étant exprimées en pourcentages en poids, par:

l'application d'un mélange formé de

| | |
|---|---|
| Verre de baryum | 29,82% |
| Silicate de lithium et d'aluminium | 67,30% |
| Silice flint | 1,22% et |
| $TiO_2$ | 0,02% avec |
| Peroxyde de benzoyle | 0,15% |
| Gamma-méthacryloxypropyl-triméthoxy-silane | 0,47 % et |
| Acide acétique glacial | 0,02%. |

39. Procédé selon la revendication 34, dans lequel l'étape de mélange pour former le gel consiste à mélanger entre eux, en pourcentages en poids:

| | |
|---|---|
| Diméthacrylate de bisphénol-A éthoxylé | 76,40% |
| 2-Isopropylthioxanthone | 0,06% |
| 4-Diméthylaminobenzoate d'éthyle | 6,90% |
| Silice fumée | 8,30% |
| Verre de baryum | 2,43% |
| Silicate d'aluminium et de lithium | 5,74% |
| Gamma-méthacryloxypropyl-triméthoxy-silane | 0,08% |
| Acide acétique glacial | 0,04% |

ladite étape d'enrobage étant constituée par l'application d'un mélange formé, les proportions étant exprimées en pourcentages en poids, par:

| | |
|---|---|
| Verre de baryum | 26,66% |
| Silicate de lithium et d'aluminium | 62,24% et |
| Silice flint | 9,01% avec |
| Peroxyde de benzoyle | 0,60% |
| Gamma-méthacryloxypropyl-triméthoxy-silane | 0,47% et |
| Acide acétique glacial | 0,02% |

40. Procédé selon la revendication 34, dans lequel l'étape de mélange pour former un gel consiste à mélanger, en pourcentages en poids:

| | |
|---|---|
| Diméthacrylate de bisphénol-A éthoxylé | 76,34% |
| 2-Isopropylthioxanthone | 0,14% |
| 4-Diméthylaminobenzoate d'éthyle | 6,90% |
| Silice fumée | 16,62%, et |

ladite étape d'enrobage de la charge étant constituée par l'application d'un mélange formé, les proportions étant exprimées en pourcentages en poids, par:

| | |
|---|---|
| Verre de baryum | 29,82% |
| Silicate de lithium et d'aluminium | 67,30% |
| Silice flint | 1,22% et |
| $TiO_2$ | 0,02%, avec |
| Peroxyde de benzoyle | 0,15% |
| Gamma-méthacryloxypropyl-triméthoxy-silane | 1,47% et |
| Acide acétique glacial | 0,02%. |

41. Procédé pour la préparation d'une composition de résine chargée stockable, utilisable pour la réparation de porcelaine et comme matériau composite dentaire, et impliquant un système pâte-pâte, ledit procédé comprenant les étapes de

mélange d'une résine à fonction méthacrylate utilisable dans les matériaux composites dentaires, avec un photoinitiateur pour ladite résine dans une proportion de 0,06—12,8% en poids, ledit photoinitiateur comprenant à la fois (1) la 2-isopropylthioxanthone ou le benzyl diméthyl acétal et (2) soit le 4-diméthylaminobenzoate d'éthyle soit le 2-diméthylaminobenzoate d'éthyle, et avec une quantité suffisante de charge dentaire en poudre appliquée avec un silane liant la résine pour préparer une première pâte,

le stockage de ladite première pâte dans un récipient opaque,

l'application d'une charge dentaire en poudre avec un silane liant la résine et au moins un agent durcisseur peroxyde exempt d'accélerateur pour ladite résine, dans une proportion efficace de 0,30—0,75% en poids,

le mélange de ladite charge appliquée avec une résine à fonction méthacrylate pour former une second pâte,

le stockage de ladite seconde pâte.

42. Procédé pour l'utilisation d'une composition préparée selon la revendication 41, comprenant, au moment de l'utilisation, le mélange desdites première et seconde pâtes dans des proportions en poids pratiquement équivalentes, sous un éclairage intérieur ordinaire, pour former une troisième pâte, et l'application de ladite troisième pâte dans un délai de quelques minutes.

43. Procédé selon la revendication 41, dans lequel ladite étape de mélange pour former la seconde pâte comprend le mélange des constituants suivants, dans les pourcentages en poids suivants:

| | |
|---|---|
| résine | 18 à 40% |
| charge | 82 à 60% |
| agent durcisseur peroxyde | 0,75 à 0,30% |
| gamma-méthacryloxypropyl-triméthoxy-silane | 1,23 à 0,06% |
| hydroxytoluene butylé | 0,01 à 0,10%, et |

l'étape de préparation de la première pâte comprenant l'utilisation des constituants suivants, dans les proportions suivantes:

| | |
|---|---|
| résine | 18 à 40% |
| charge | 80 à 60% |
| lesdits photoinitiateurs | 0,06 à 12,8% |
| gamma-méthacryloxypropyl-triméthoxy-silane | 1,23 à 0,06%. |

44. Procédé selon la revendication 43, dans lequel l'étape de formation de la seconde pâte utilise les constituants suivants, dans les pourcentage en poids suivants:

| | |
|---|---|
| Bis-GMA | 0 à 32% |
| Diméthacrylate de bisphénol-A éthoxylé | 0 à 40% |
| Diméthacrylate d'éthylène glycol | 0 à 24% |
| Diméthacrylate de diéthylène glycol | 0 à 24% |
| Diméthacrylate de triéthylène glycol | 0 à 24% |
| Diméthacrylate de polyéthylène glycol | 0 à 24% |
| Verre de baryum | 0 à 25% |
| Silicate de lithium et d'aluminium | 0 à 82% |
| Silice flint | 0 à 8,5% |
| Verre de borosilicate | 0 à 82% |
| Silice synthétique fumée | 0 à 52% |
| Quartz | 0 à 82% |
| Verre de strontium | 0 à 82% |
| Dioxyde de titane | 0 à 0,13% |
| Agents de teinture (par ex., oxydes de fer) | 0 à 4% |
| Gamma-méthacryloxypropyl-triméthoxy-silane | 0 à 1,25% |
| Agent durcisseur peroxyde | 0,30 à 0,75%, et |

ladite étape de formage de la première pâte utilise les constituants ci-après, dans les proportions suivantes, exprimées en pourcentages en poids:

| | |
|---|---|
| Bis-GMA | 0 à 32 |
| Diméthacrylate de bisphénol-A éthoxylé | 0 à 40 |
| Diméthacrylate d'éthylène glycol | 0 à 24 |
| Diméthacrylate de diéthylène glycol | 0 à 24 |
| Diméthacrylate de triéthylène glycol | 0 à 24 |
| Diméthacrylate de polyéthylène glycol | 0 à 24 |
| Verre de baryum | 0 à 25 |
| Silicate de lithium et d'aluminium | 0 à 82 |
| Silice flint | 0 à 8,5 |
| Verre de borosilicate | 0 à 82 |
| Silice synthétique fumée | 0 à 52 |
| Quartz | 0 à 82 |
| Verre de strontium | 0 à 82 |
| Dioxyde de titane | 0 à 0,13 |
| Agents de teinture (par ex., oxydes der fer) | 0 à 4 |
| Gamma-méthacryloxypropyl-triméthoxy-silane | 0 à 1,25 |
| Lesdits photoinitiateurs | 0,06 à 12,8. |

45. Procédé selon la revendication 41 dans lequel l'étape de formage de la seconde pâte consiste à mélanger les consituants suivants, dans les pourcentages en poids indiqués:

| | |
|---|---|
| Diméthacrylate de bisphénol-A éthoxylé | 17,36% |
| Peroxyde de benzoyle | 0,60% |
| Hydroxytoluène butylé | 0,04% |
| Verre de strontium | 79,878% |
| Silice fumée | 0,92% |
| Gamma-méthacryloxypropyl-triméthoxy-silane | 0,20% |
| Acide acétique glacial | 0,0016%, |

et l'étape de formation de la première pâte consistant à mélanger les constituants suivants, dans les pourcentages en poids indiqués:

| | |
|---|---|
| Diméthacrylate de bisphénol-A éthoxylé | 14,94% |
| 2-Isopropylthioxanthone | 0,06% |
| 4-Dimethylaminobenzoate d'éthyle | 3,00% |
| Verre de strontium | 79,8784% |
| Silice fumée | 0,92% |
| Gamma-méthacryloxypropyl-triméthoxy-silane | 1,20% |
| Acide acétique glacial | 0,0016%. |

46. Procédé pour la préparation d'une composition chargée de résine, apte au stockage, utilisable dans la réparation de la porcelaine et comme composition dentaire, et impliquant un système pâte-poudre, ledit procédé comprenant:

le melange d'une résine à fonction méthacrylate, utilisable dans les matériaux composites dentaires, avec un photoinitiateur pour ladite résine, dans une proportion de 0,06 à 12,80% en poids, ledit photoinitiateur comprenant à la fois (1) la 2-isopropylthioxanthone ou le benzyl diméthyl acétal et (2) soit le 4-diméthylaminobenzoate d'éthyle soit le 2-diméthylaminobenzoate d'éthyle, et avec suffisamment de charge dentaire en poudre, enrobée avec un silane liant la résine, pour former une pâte,

le stockage de ladite pâte dans un conteneur opaque,

l'enrobage d'une charge dentaire en poudre avec un silane liant la résine et au moins un agent durcisseur peroxyde exempt d'accélérateur pour ladite résine, dans une proportion efficace de 0,05—10% en poids pour former une pâte,

le stockage de ladite poudre.

47. Procédé d'utilisation de la composition selon la revendication 46, comprenant:

au moment de l'utilisation, l'addition de ladite poudre à ladite pâte dans une proportion de 5,0 à 10% en poids de ladite pâte, sous éclairage intérieur ordinaire, pour former une pâte épaissie, et

l'application de ladite pâte épaissie dans un délai de quelconques minutes.

48. Procédé selon la revendication 46 dans lequel ladite étape de mélange de ladite résine avec un photoinitiateur et une charge comprend le mélange des ingrédients suivants, en poids:

| | |
|---|---|
| Résine | 18 à 40% |
| Charge | 80 à 60% |
| γ-méthacryloxypropyltriméthoxylsilane | 1,23 à 0,06% |
| ledit photoinitiateur | 0,06 à 12,8%, et |

ladite étape d'enrobage de la charge dentaire comprend l'enrobage de la charge dentaire dans une proportion de 99,75 à 97,74% en poids, avec 0,10 à 1,50% en poids de γ-méthacryloxypropyltriméthoxysilane et avec un agent durcisseur peroxyde dans une proportion de 0,30 à 10% en poids.

49. Procédé selon la revendication 48, dans lequel ladite étape de mélange pour former ladite première pâte utilise les constituants suivants, dans les pourcentages en poids suivants:

| | |
|---|---|
| Bis-GMA | 0 à 32% |
| Diméthacrylate de bisphénol-A éthoxylé | 0 à 40% |
| Diméthacrylate d'éthylène glycol | 0 à 24% |
| Diméthacrylate de diéthylène glycol | 0 à 24% |
| Diméthacrylate de triéthylène glycol | 0 à 24% |
| Diméthacrylate de polyéthylène glycol | 0 à 24% |
| Verre de baryum | 0 à 25% |
| Silicate de lithium et d'aluminium | 0 à 82% |
| Silice flint | 0 à 8,5% |
| Verre de borosilicate | 0 à 82% |
| Silice synthétique fumée | 0 à 52% |
| Verre de strontium | 0 à 82% |
| Quartz | 0 à 82% |
| Dioxyde de titane | 0 à 0,13% |
| Agents de teinture (par ex., oxydes de fer) | 0 à 4% |
| Gamma-méthacryloxypropyl-triméthoxy-silane | 0 à 1,25% |
| Lesdits photoinitiateurs | 0,06 à 12,8%, et |

ladite étape de préparation de la poudre utilise les constituants ci-après, dans les pourcentages en poids suivants:

| | |
|---|---|
| Verre de baryum | 0 à 30% |
| Silicate de lithium et d'aluminium | 0 à 99,75% |
| Silice flint | 0 à 10% |
| Verre de borosilicate | 0 à 99,75% |
| Silice synthétique fumée | 0 à 99,75% |
| Verre de strontium | 0 à 99,75% |
| Dioxyde de titane | 0 à 0,15% |
| Agents de teinture (par ex., oxydes de fer) | 0 à 5% |
| Gamma-méthacryloxypropyl-triméthoxy-silane | 0 à 1,50% |
| Agent durcisseur peroxyde | 0,30 à 10%. |

50. Procédé selon la revendication 46, dans lequel l'étape de préparation de ladite première pâte consiste à mélanger les constituants suivants, dans les pourcentages en poids suivants:

| | |
|---|---|
| Diméthacrylate de bisphénol-A éthoxylé | 16,07% |
| 2-Isopropylthioxanthone | 0,03% |
| 4-Diméthylaminobenzoate d'éthyle | 1,49% |
| Silice fumée | 0,90% |
| Silicate de lithium et d'aluminium | 57,08% |
| Verre de baryum | 24,45% |

et ladite étape de préparation de la dite poudre comprend le mélange des constituants suivants, dans les pourcentages en poids suivants:

| | |
|---|---|
| Silice fumée (pré-enrobée avec un silane liant la résine) | 95,00% |
| Peroxyde de benzoyle | 5,00%. |